# EUROPEAN PATENT SPECIFICATION

(11) **EP 2 742 155 B1**
(45) Date of publication and mention of the grant of the patent: **24.01.2018**
(21) Application number: 12821977.1
(22) Date of filing: 08.08.2012
(51) Int. Cl.: C12Q 1/68, G01N 33/53

(54) **BIOMARKER FOR ALZHEIMER'S DISEASE AND/OR MILD COGNITIVELY IMPAIRMENT**
BIOMARKER FÜR MORBUS ALZHEIMER UND/ODER LEICHTE KOGNITIVE BEEINTRÄCHTIGUNG
BIOMARQUEUR POUR LA MALADIE D'ALZHEIMER ET/OU LE TROUBLE LÉGER COGNITIF

(30) Priority: 08.08.2011 US 201161521241 P; 13.01.2012 US 201261586291 P; 15.05.2012 US 201261647214 P
(43) Date of publication of application: 18.06.2014
(73) Proprietor: Advanced Genomic Technology LLC, Louisville, KY 40241 (US)
(72) Inventor: WANG, Eugenia, Louisville, KY 40241 (US)
(74) Representative: Gallois, Valérie
(86) International application number: PCT/US2012/049971
(87) International publication number: WO 2013/022953

(56) References cited:
- WO-A1-2009/009457
- WO-A1-2009/009457
- WO-A1-2011/015829
- WO-A2-2009/025852
- US-A1- 2010 292 281
- HODGES A K ET AL: "Plasma microRNA: A new source of biomarker for Alzheimer's disease", ALZHEIMER'S & DEMENTIA: THE JOURNAL OF THE ALZHEIMER'SASSOCIATION, ELSEVIER, NEW YORK, NY, US, vol. 6, no. 4, 1 July 2010 (2010-07-01), page S510, XP027440172, ISSN: 1552-5260, DOI: 10.1016/J.JALZ.2010.05.1701 [retrieved on 2010-07-01]
- HYMAN SCHIPPER ET AL: "MicroRNA expression in Alzheimer blood mononuclear cells.", GENE REGULATION AND SYSTEMS BIOLOGY, vol. 1, 1 January 2007 (2007-01-01), pages 263-274, XP55047309, ISSN: 1177-6250
- OLIVIER C MAES ET AL: "MicroRNA: Implications for Alzheimer Disease and other Human CNS Disorders", CURRENT GENOMICS, BENTHAM SCIENCE PUBLISHERS LTD, NL, vol. 10, no. 3, 1 May 2009 (2009-05-01), pages 154-168, XP002617830, ISSN: 1389-2029, DOI: 10.2174/138920209788185252
- WANG X ET AL: "miR-34a, a microRNA up-regulated in a double transgenic mouse model of Alzheimer's disease, inhibits bcl2 translation", BRAIN RESEARCH BULLETIN, ELSEVIER SCIENCE LTD, OXFORD, GB, vol. 80, no. 4-5, 28 October 2009 (2009-10-28), pages 268-273, XP026662852, ISSN: 0361-9230, DOI: 10.1016/J.BRAINRESBULL.2009.08.006 [retrieved on 2009-08-14]
- JUAN NUNEZ-IGLESIAS ET AL: "Joint Genome-Wide Profiling of miRNA and mRNA Expression in Alzheimer's Disease Cortex Reveals Altered miRNA Regulation", PLOS ONE, vol. 5, no. 2, 1 February 2010 (2010-02-01), page e8898, XP055187191, DOI: 10.1371/journal.pone.0008898
- ATHANASIOS ZOVOILIS ET AL: "microRNA-34c is a novel target to treat dementias", THE EMBO JOURNAL, vol. 30, no. 20, 23 September 2011 (2011-09-23), pages 4299-4308, XP055047170, ISSN: 0261-4189, DOI: 10.1038/emboj.2011.327
- BHATNAGAR SHEPHALI ET AL: "Increased nnicroRNA-34c abundance in Alzheimer's disease circulating blood plasma", FRONTIERS IN MOLECULAR NEUROSCIENCE, vol. 7, February 2014 (2014-02), XP055187385,
- WANG, W.-X. ET AL.: 'The expression of microRNA miR-107 decreases early in Alzheimer's disease and may accelerate disease progression through regulation of beta-site amyloid precursor protein-cleaving enzyme 1' J NEUROSCI. vol. 28, no. 5, 30 January 2008, pages 1213 - 1223, XP002617832
- HEBERT, S.S. ET AL.: 'Loss of microRNA cluster miR-29a/b-1 in sporadic Alzheimer's disease correlates with increased BACE 1 /beta-secretase expression' PROC NAT ACAD SCI. vol. 105, no. 17, 29 April 2008, pages 6415 - 6420, XP055127732

## Description

### FIELD OF THE INVENTION

Biomarkers of Alzheimer's disease and/or Mild Cognitively Impairment, and use thereof.

### BACKGROUND

Currently, the clinical diagnosis of Alzheimer's disease (AD) and/or mild cognitive impairment (MCI) generally requires an evaluation of medical history and physical examination including neurological, neuropsychological and psychiatric assessment, as well as various biological, radiological and electrophysiological tests, such as for instance measuring brain volume or activity measurements derived from neuroimaging modalities such as magnetic resonance imaging (MRI) or positron emission tomography (PET) of relevant brain regions. However, neuroimaging modalities are expensive, labor-intensive and not universally available. Despite the battery of tests available, a definitive diagnosis of AD can only be achieved by post-mortem brain examination.

Hodges et al (Journal of Alzheimer's Association, 2010, 6, S510) describes plasma microRNA as a new source of biomarker for determining AD. WO2009/025852 describes methods of detecting microRNAs in body fluids for evaluation of in vivo cell death associated with AD. WO2009/009457 and Schipper et al (Gene Regulation and Systems Biology, 2007, 1, 263-274) pertain to AD disease-specific microRNAs. Maes et al (Current Genomics, 2009, 10, 154-168), Wang et al (Brain Research Bulletin, 2009, 80, 268-273), Nunez-Iglesias et al (PLoS ONE, 2010, 5, e8898) and Wang et al (J Neurosci, 2008, 28, 1213-1223) describe links between microRNAs and AD.

### SUMMARY OF INVENTION

The scope of the present invention is defined by the claims and any information that does not fall within the claims is provided for information only.

In one non-limiting broad aspect, there is provided biomarkers of Alzheimer's disease (AD) and/or mild cognitively impairment (MCI), and use thereof.

In one non-limiting broad aspect, the biomarker of AD is a microRNA associated with AD and/or MCI, and/or target protein thereof, wherein the microRNA and/or target protein thereof is a circulating biomarker or an intracellular biomarker.

In one non-limiting broad implementation, there is provided a biomarker which is a microRNA associated with AD and/or MCI, and/or target protein thereof, and use thereof, alone or in combination, as an intracellular reporting system. In one aspect, the reporting system described herein may include a vector adapted for expressing said microRNA in a cell, or a cell comprising such vector, or a host comprising said vector, and the like. Such reporting system may be used in methods for determining, detecting, screening compounds and/or treatments, for identifying or selecting candidate therapeutic compounds and/or treatments for treating and/or preventing AD and/or MCI.

In one aspect, there is provided a method for identifying / selecting a candidate therapeutic compound for prevention and/or treatment of Alzheimer's disease (AD) and/or mild cognitively impairment (MCI), said method comprising contacting a cell expressing a microRNA associated with AD and/or MCI, target protein thereof, with a compound; causing a determination of an intracellular level of said microRNA and/or target protein thereof, obtaining information that is indicative of whether the compound is a candidate therapeutic compound for prevention and/or treatment of AD and/ or MCI, said information obtained from a comparison of the intracellular level of the respective circulating microRNA and/or protein target thereof in presence of the compound to a reference level, and identifying / selecting the candidate therapeutic compound at least partly based on said information.

In one non-limiting broad implementation, there is provided a biomarker which is a circulating microRNA associated with AD and/or MCI, and/or circulating target protein thereof, and use thereof, alone or in combination, in methods for determining, detecting, diagnosing, monitoring, i.e., determining regression, progression, course and/or onset of Alzheimer's disease (AD), and/ or mild cognitively impairment (MCI), or of a likelihood of AD and/ or MCI, or of an increased likelihood of AD and/or MCI, or of prognosis for a conversion from early MCI to late MCI, or of prognosis for a conversion from MCI to *bona fide* AD, and the like.

In a aspect, there is provided a method for identifying / selecting a candidate therapeutic compound for prevention and/or treatment of Alzheimer's disease (AD) and/or mild cognitively impaired (MCI), said method comprising contacting a host with a compound; causing a determination of a level of a circulating microRNA and/or protein target thereof from a host, said microRNA being associated with AD and/or MCI, obtaining information that is indicative of whether the compound is a candidate therapeutic compound for prevention and/or treatment of AD and/or MCI, said information obtained from a comparison of the level of the respective circulating microRNA and/or protein target thereof in presence of the compound compared to a reference level, and identifying / selecting the candidate therapeutic compound at least partly based on said information.

In another aspect, there is provided a method for use in connection with a process for identifying / selecting a candidate therapeutic compound for prevention and/or treatment of Alzheimer's disease (AD) and/ or mild cognitively impaired (MCI), said method comprising determining a level of a circulating microRNA and/or protein target thereof from a host that has been contacted with a compound, said microRNA being associated with AD and/or MCI, processing the level of the respective circulating microRNA and/or target protein thereof at least in part based on a reference level to derive information conveying whether the compound has therapeutic activity, causing conveyance of the information to a recipient for identifying / selecting the candidate therapeutic compound at least partly based on said information.

In yet another aspect, the herein described method is for identifying / selecting a candidate therapeutic compound for reducing risk of an individual having mild stage of Alzheimer's disease progressing to moderate or severe stage, where said information is indicative of whether the compound is a candidate therapeutic compound for reducing said risk.

In yet another aspect, the herein described method is for identifying / selecting a candidate therapeutic compound for reducing risk of an individual having early mild cognitive impairment (EMCI) converting to late mild cognitive impairment (LMCI), where said information is indicative of whether the compound is a candidate therapeutic compound for reducing said risk.

In yet another aspect, the herein described method is for identifying / selecting a candidate therapeutic compound for reducing risk of an individual having late mild cognitive impairment (LMCI) converting to *bona fide* Alzheimer's Disease (AD), where said information is indicative of whether the compound is a candidate therapeutic compound for reducing said risk.

In yet another aspect, there is provided a kit for identifying / selecting a candidate therapeutic compound for (a) prevention and/or treatment of Alzheimer's disease (AD) and/or mild cognitively impaired (MCI), or (b) for reducing risk of an individual having mild AD progressing to moderate or severe stage, or (c) for reducing risk of an individual having early mild cognitive impairment (EMCI) converting to late mild cognitive impairment (LMCI), or (d) for reducing risk of an individual having late mild cognitive impairment (LMCI) converting to *bona fide* Alzheimer's Disease (AD), or (e) for distinguishing between mild AD and MCI, or (f) for distinguishing between EMCI and LMCI, said kit comprising at least one detecting reagent for determining a level of a circulating microRNA and/or target protein thereof, said microRNA being associated with respective (a) severe, moderate, and mild AD, or (b) AD and/or MCI, or (c) EMCI and/or LMCI, or (d) LMCI and/or AD, or (e) mild AD and/or MCI, or (f) EMCI and LMCI. Optionally, said kit further comprises instructions for use.

In yet another aspect, there is provided an apparatus for conveying to a recipient, information that is indicative of whether a candidate compound is a candidate therapeutic compound for (a) prevention and/or treatment of Alzheimer's disease (AD) and/ or mild cognitively impaired (MCI), or (b) for reducing risk of an individual having early mild cognitive impairment (EMCI) converting to late mild cognitive impairment (LMCI), or (c) for reducing risk of an individual having late mild cognitive impairment (LMCI) converting to *bona fide* Alzheimer's Disease (AD), or (d) for distinguishing between mild AD and MCI, or (e) for distinguishing between EMCI and LMCI, where the information is at least partly based on a level of a circulating microRNA in presence of the compound compared to a reference level of the microRNA, said microRNA being associated with respective (a) severe, moderate, or mild AD, or (b) AD and/or MCI, or (c) EMCI and/or LMCI, or (d) LMCI and/or AD, or (e) mild AD and/or MCI, or (f) EMCI and LMCI.

In yet another aspect, there is provided a non transitory computer readable storage medium storing a program element suitable for execution by a CPU, the program element implementing a graphical user interface module for displaying information for identifying / selecting a candidate therapeutic compound for (a) prevention and/or treatment of Alzheimer's disease progression from mild to moderate, and from moderate to severe stage (b) prevention and/or treatment of Alzheimer's disease (AD) and/ or mild cognitively impaired (MCI), or (c) for reducing risk of an individual having early mild cognitive impairment (EMCI) converting to late mild cognitive impairment (LMCI), or (d) for reducing risk of an individual having late mild cognitive impairment (LMCI) converting to *bona fide* Alzheimer's Disease (AD), or (e) for distinguishing between mild AD and MCI, or (f) for distinguishing between EMCI and LMCI, where the information is at least partly based on a level of a circulating microRNA in presence of the compound compared to a reference level of the circulating microRNA, said microRNA being associated with respective (a) severe, moderate and/or mild AD, or (b) AD and/or MCI, or (c) EMCI and/or LMCI, or (d) LMCI and/or AD, or (e) mild AD and/or MCI, or (f) EMCI and LMCI.

In yet another aspect, there is provided a server system implementing a graphical user interface module for displaying information for identifying / selecting a candidate therapeutic compound for (a) prevention and/or treatment of Alzheimer's disease (AD) progression from mild to moderate to severe, and/ or mild cognitively impaired (MCI), or (b) for reducing risk of an individual having early mild cognitive impairment (EMCI) converting to late mild cognitive impairment (LMCI), or (c) for reducing risk of an individual having late mild cognitive impairment (LMCI) converting to *bona fide* Alzheimer's Disease (AD) or (d) for distinguishing between mild AD and MCI, or (e) for distinguishing between EMCI and LMCI. The server system stores a program element for execution by a CPU. The program element includes a plurality of program element components. A first program element component is for receiving at least one first signal which conveys information related to a level of a circulating microRNA in presence of the candidate compound, said level being a first level, and for receiving at least one second signal which conveys information related to the level of the circulating microRNA in absence of the candidate compound, said level being a second level, said microRNA being associated with respective (a) mild, moderate, and severe AD, or (b) AD and/or MCI, or (c) EMCI and/or LMCI, or (d) LMCI and/or AD, or (e) mild AD and/or MCI, or (f) EMCI and LMCI. A second program element component is for processing the first and second signals to derive said first and second levels. A third program element component is for processing said first and second levels for comparing said first and second levels for obtaining information indicative of therapeutic effect of the candidate compound. A fourth program element component is for generating and issuing a signal for displaying a graphical representation information for identifying / selecting a candidate therapeutic compound for (a) prevention and/or treatment of Alzheimer's disease (AD) and/ or mild cognitively impaired (MCI), or (b) for reducing risk of an individual having early mild cognitive impairment (EMCI) converting to late mild cognitive impairment (LMCI), or (c) for reducing risk of an individual having late mild cognitive impairment (LMCI) converting to *bona fide* Alzheimer's Disease (AD) or (d) for distinguishing between mild AD and MCI, or (e) for distinguishing between EMCI and LMCI.

In yet another aspect, there is provided a client-server system for implementing a graphical user interface module for displaying information for identifying / selecting a candidate therapeutic compound for (a) prevention and/or treatment of Alzheimer's disease (AD) and/ or mild cognitively impaired (MCI), or (b) for reducing risk of an individual having mild AD to progress to moderate or severe stage, (c) for reducing risk of an individual having early mild cognitive impairment (EMCI) converting to late mild cognitive impairment (LMCI), or (d) for reducing risk of an individual having late mild cognitive impairment (LMCI) converting to *bona fide* Alzheimer's Disease (AD) or (e) for distinguishing between mild AD and MCI, or (e) for distinguishing between EMCI and LMCI. The client-server system comprises a client system and a server system operative to exchange messages over a data network. The server system stores a program element for execution by a CPU. The program element includes a plurality of program element components. A first program element component is for execution on the server system and is for receiving at least one first signal which conveys information related to a level of a circulating microRNA in presence of the candidate compound, said level being a first level, and for receiving at least one second signal which conveys information related to the level of the circulating microRNA in absence of the candidate compound, said level being a second level, said microRNA being associated with respective (a) severe, moderate, and/or mild AD, or (b) AD and/or MCI, or (c) EMCI and/or LMCI, or (d) LMCI and/or AD, or (e) mild AD and/or MCI, or (f) EMCI and LMCI. A second program element component is for execution on the server system and is for processing the first and second signals to derive said first and second levels. A third program element component is for execution on the server system and is for processing said first and second levels for comparing said first and second levels for obtaining information indicative of therapeutic effect of the candidate compound. A fourth program element component is for execution on the server system and is for sending messages to the client system for causing the client system to display a graphical representation information for identifying / selecting a candidate therapeutic compound for (a) prevention and/or treatment of Alzheimer's disease (AD). and/ or mild cognitively impaired (MCI), or (b) for reducing risk of an individual having mild AD to advance to moderate or severe stage, or (c) for reducing risk of an individual having early mild cognitive impairment (EMCI) converting to late mild cognitive impairment (LMCI), or (d) for reducing risk of an individual having late mild cognitive impairment (LMCI) converting to *bona fide* Alzheimer's Disease (AD) or (e) for distinguishing between mild AD and MCI, or (f) for distinguishing between EMCI and LMCI.

In another aspect, the herein described methods can be used to differentiate between mild, moderate and severe AD, at least partly based on the microRNA level in a sample. In another aspect, the herein described methods can be used to differentiate between MCI and mild AD, at least partly based on the microRNA level in a sample.

In another aspect, the herein described methods involving determination in a sample of the microRNA level or the target protein level can be used alone or in combination with one another, for example as companion diagnostics.

In yet another aspect, there is provided a method for determining therapeutic efficacy of an anti-Alzheimer's disease (AD) treatment and/ or anti-mild cognitively impaired (MCI) treatment, said method comprising contacting a subject with said treatment; causing a determination of a level of a circulating microRNA and/or target protein thereof from the subject, said microRNA being associated with AD and/or MCI, obtaining information that is indicative of the treatment therapeutic efficacy, said information obtained from a comparison of the level of the respective circulating microRNA and/or target protein thereof in presence of the treatment compared to a reference level, and determining therapeutic efficacy of the treatment at least partly based on said information.

In yet another aspect, there is provided a method for use in connection with an anti-Alzheimer's disease (AD) and/ or anti-mild cognitively impaired (MCI) treatment, said method comprising: determining for a subject having received the treatment, a level of a circulating microRNA and/or target protein thereof, said microRNA being associated with AD and/or MCI; processing the level of the respective circulating microRNA and/or target protein thereof at least in part based on a reference level to derive information conveying a level of therapeutic efficacy associated with the treatment; and causing conveyance of the information to a recipient for determining therapeutic efficacy of the treatment at least partly based on said information.

In another aspect, there is provided a kit for determining therapeutic efficacy of an anti-Alzheimer's disease (AD) and/ or anti-mild cognitively impaired (MCI) treatment, said kit comprising at least one detecting reagent for determining a level of a circulating microRNA and/or target protein thereof, said microRNA being associated with AD and/or MCI. Optionally, said kit further comprises instructions for use.

In another aspect, there is provided an apparatus for conveying to a recipient, information that is indicative of therapeutic efficacy of an anti-Alzheimer's disease (AD) treatment, where the information is at least partly based on a level of a circulating microRNA and/or target protein thereof, said microRNA being associated with AD.

In yet another aspect, there is provided a computer readable storage medium storing a program element suitable for execution by a CPU, the program element implementing a graphical user interface module for displaying information for determining therapeutic efficacy of an anti-Alzheimer's disease (AD) and/ or anti-mild cognitively impaired (MCI) treatment, where the information is at least partly based on a level of a circulating microRNA and/or target protein thereof in presence of the anti-AD and/or MCI treatment compared to a reference level, said microRNA being associated with AD and/or MCI.

In yet another aspect, there is provided a server system implementing a graphical user interface module for displaying information for determining therapeutic efficacy of an anti-Alzheimer's disease (AD) and/ or anti-mild cognitively impaired (MCI) treatment. The server system stores a program element for execution by a CPU. The program element includes a plurality of program element components. A first program element component is for receiving at least one signal which conveys information related to a level of circulating microRNA and/or target protein thereof from a subject having received the anti-AD and/or anti-MCI treatment, said level being a first level, and for receiving at least one second signal which conveys information related to a reference level, said level being a second level. A second program element component is for processing the first and second signals to derive said first and second levels. A third program element component is for processing said first and second levels for comparing said first and second levels for obtaining information indicative of therapeutic efficacy of said anti-AD and/or anti-MCI treatment. A fourth program element component is for generating and issuing a signal for displaying a graphical representation information for determining the therapeutic efficacy of said anti-AD and/or anti-MCI treatment.

In yet another aspect, there is provided a client-server system for implementing a graphical user interface module for displaying information for determining therapeutic efficacy of an anti-Alzheimer's disease (AD) and/ or anti-mild cognitively impaired (MCI) treatment. The client-server system comprises a client system and a server system operative to exchange messages over a data network. The server system stores a program element for execution by a CPU. The program element includes a plurality of program element components. A first program element component is for execution on the server system and is for receiving at least one signal which conveys information related to a level of circulating microRNA and/or target protein thereof from a subject having received the anti-AD and/or anti-MCI treatment, said level being a first level, and for receiving at least one second signal which conveys information related to a reference level, said level being a second level. A second program element component is for execution on the server system and is for processing the first and second signals to derive said first and second levels. A third program element component is for execution on the server system and is for processing said first and second levels for comparing said first and second levels for obtaining information indicative of therapeutic efficacy of said anti-AD and/or anti-MCI treatment. A fourth program element component is for execution on the server system and is for sending messages to the client system for causing the client system to display a graphical representation information for determining the therapeutic efficacy of said anti-AD and/or anti-MCI treatment.

In one aspect, there is provided a method for determining presence of Alzheimer's disease (AD) and/or mild cognitively impairment (MCI) in a subject, said method comprising: causing a determination of a level of a circulating microRNA and/or target protein thereof from the subject, said microRNA being associated with AD and/or MCI, obtaining information conveying a level of likelihood of presence of AD and/or MCI, said information obtained from a comparison of the level of the respective circulating microRNA and/or target protein thereof to a reference level, and determining the presence of AD and/or MCI at least partly based on said information.

In one aspect, there is provided a method for use in connection with a process for determining presence of Alzheimer's disease (AD) and/or mild cognitively impairment (MCI) in a subject, said method comprising determining a level of a circulating microRNA and/or target protein thereof from the subject, said microRNA being associated with AD and/or MCI, processing the level of the respective circulating microRNA and/or target protein thereof at least in part based on a reference level to derive information conveying a level of likelihood of presence of AD and/or MCI, and causing conveyance of the information to a recipient for determining the presence of AD and/or MCI.

In one aspect, there is provided a kit for determining presence of Alzheimer's disease (AD) and/or mild cognitively impairment (MCI) in a subject, said kit comprising at least one detecting reagent for determining the level of a circulating microRNA and/or target protein thereof, said microRNA being associated with AD and/or MCI. Optionally, said kit further comprises instructions for use.

In yet aspect, there is provided an apparatus for conveying to a recipient, information that is indicative of presence of Alzheimer's disease (AD) and/or mild cognitively impairment (MCI) in a subject, where the information is at least partly based on a level of a circulating microRNA and/or target protein thereof, said microRNA being associated with AD and/or MCI.

In yet aspect, there is provided a non transitory computer readable storage medium storing a program element suitable for execution by a CPU, the program element implementing a graphical user interface module for displaying information that is indicative of presence of Alzheimer's disease (AD) and/or mild cognitively impairment (MCI) in a subject, where the information is at least partly based on a level of a circulating microRNA and/or target protein thereof, said microRNA being associated with AD and/or MCI.

In one aspect, there is provided a server system implementing a graphical user interface module for displaying information that is indicative of presence of Alzheimer's disease (AD) and/or mild cognitively impairment (MCI) in a subject. The server system stores a program element for execution by a CPU. The program element includes a plurality of program element components. A first program element component is for receiving at least one signal which conveys information related to a level of circulating microRNA and/or target protein thereof from a subject, said microRNA being associated with AD and/or MCI. A second program element component is for processing the signal to derive said circulating microRNA level. A third program element component is for processing the level of the circulating microRNA at least in part based on a reference level to derive information conveying a level of likelihood of presence of AD and/or MCI. A fourth program element component is for generating and issuing a signal for displaying a graphical representation information that is indicative of the presence of AD and/or MCI in the subject.

In yet another aspect, there is provided a client-server system for implementing a graphical user interface module for displaying information that is indicative of presence of Alzheimer's disease (AD) and/or mild cognitively impairment (MCI) in a subject. The client-server system comprises a client system and a server system operative to exchange messages over a data network. The server system stores a program element for execution by a CPU. The program element includes a plurality of program element components. A first program element component is for execution on the server system and is for receiving at least one signal which conveys information related to a level of circulating microRNA and/or target protein thereof from the subject, said microRNA being associated with AD and/or MCI. A second program element component is for execution on the server system and is for processing the signal to derive said respective circulating microRNA and/or target protein thereof level. A third program element component is to be executed on the server system and is for processing the level of the circulating microRNA at least in part based on a reference level to derive information conveying a level of likelihood of presence of AD and/or MCI. A fourth program element component is to be executed on the server system and is for sending messages to the client system for causing the client system to display a graphical representation information that is indicative of the presence of AD and/or MCI.

In one non-limiting aspect, the herein described methods, kits, apparatuses, computer readable medium, server system, or client-server system, for detecting the presence of AD and/or MCI also includes monitoring AD and/or MCI disease progression in the subject, e.g. a change from one MMSE group to another, i.e., from severe to moderate AD, moderate to mild AD, mild AD to normal elderly control (NEC) and the like.

In one aspect, the herein described recipient is the subject, or is an individual from a medical staff.

In a specific non-limiting implementation, the herein described client-server system includes a plurality of client systems operative to exchange messages with the server system over a data network. The data network may be of any suitable network configuration including Intranets and the Internet. The client systems may be embodied in any suitable computing device including, but not limited to, desktop computers, laptop computers and personal digital assistants (PDAs).

### BRIEF DESCRIPTION OF THE DRAWINGS

A detailed description of aspects is provided herein below, by way of example only, with reference to the accompanying drawings.

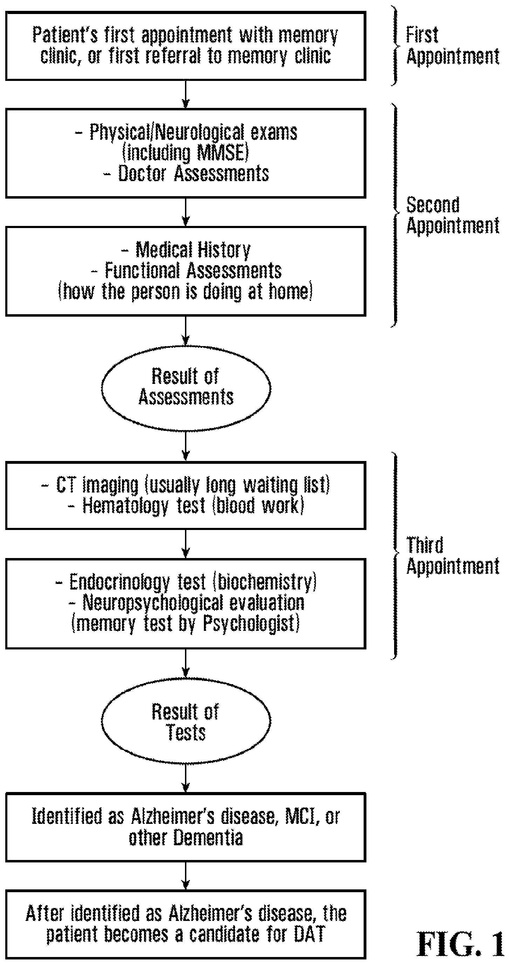

- Figure 1: (A) Flowchart of an illustrative and general Dementia of Alzheimer's Type (DAT) candidate identification process for the purposes of establishing an AD population. (B) Flowchart of an illustrative and general Normal Elderly Control (NEC) candidate identification process for the purposes of establishing an NEC population.

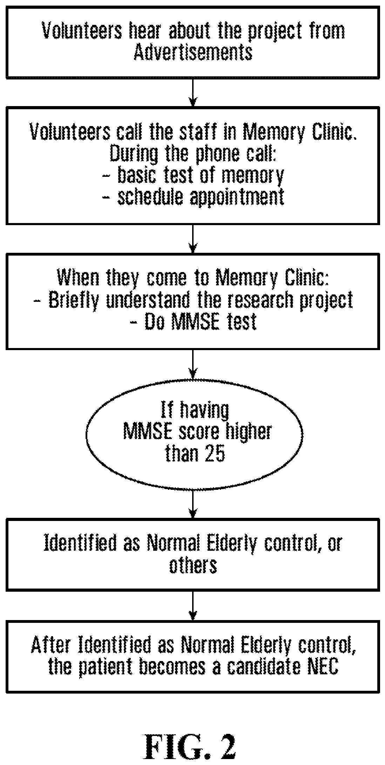

- Figure 2: Flowchart of an illustrative and general process for obtaining blood sample from the candidates.

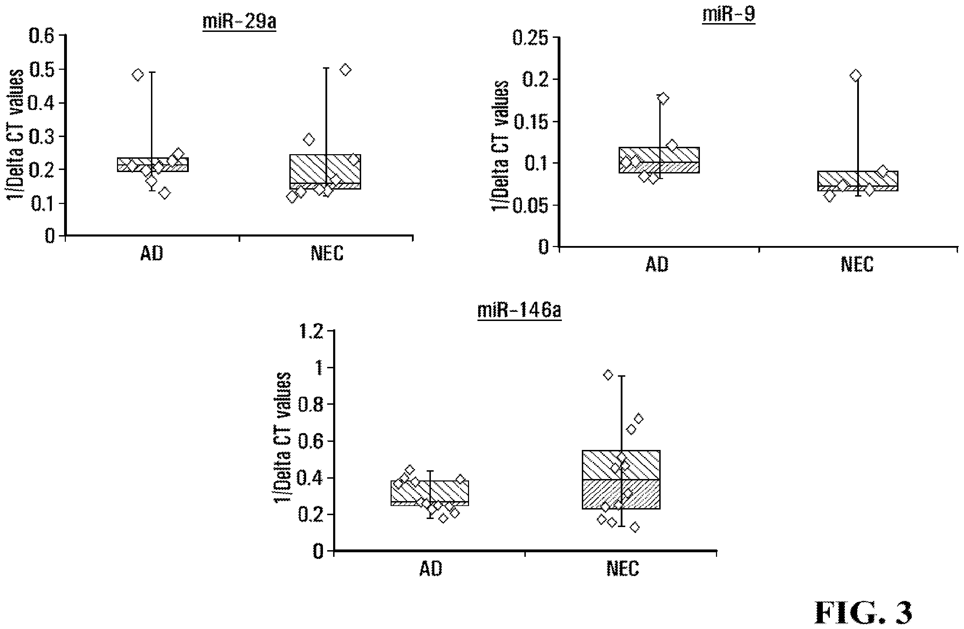

- Figure 3: An aspect of illustrative non-limiting box plot of qPCR results of three microRNAs in plasma samples of AD and NEC individuals.

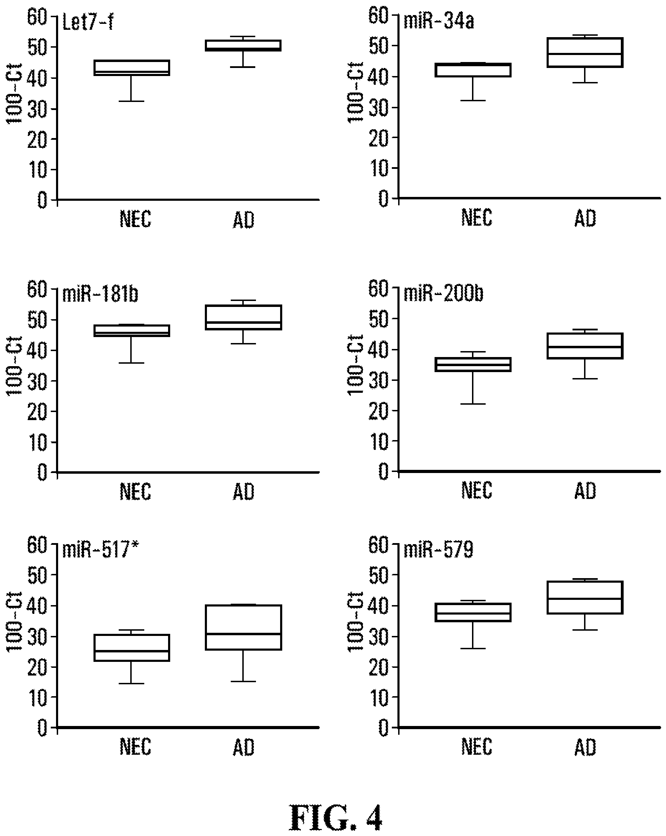

- Figure 4: An aspect of illustrative non-limiting box plot showing qPCR results for six miRNA in plasma samples of 7 AD individuals and 7 NEC individuals.

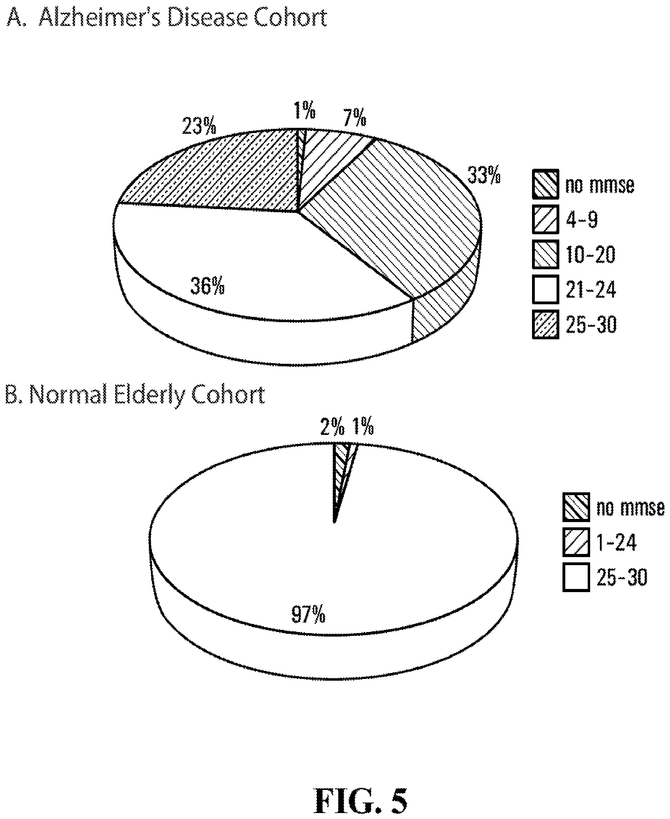

- Figure 5: Non-limiting and illustrative pie distribution of Mini-Mental Status Exam (MMSE) scores within (A) 114 AD individuals (No MMSE: 1; MMSE 4-9: 8; MMSE 10-20: 36; MMSE 21-24: 40; MMSE 25-30: 26) and (B) 124 NEC individuals (No MMSE: 2; MMSE 1-24: 1; MMSE 25-30: 121).

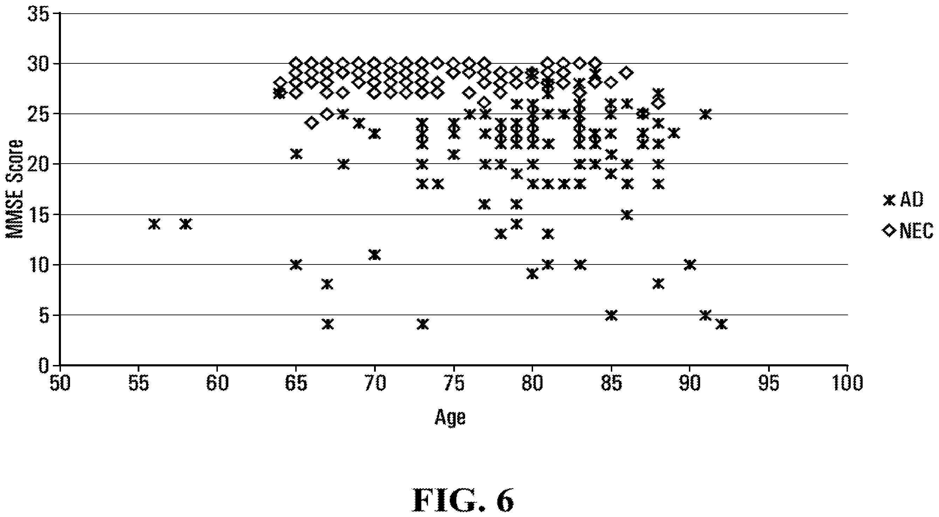

- Figure 6: An aspect of illustrative non-limiting results of MMSE scores distribution of the total sample population on the y axis and the age distribution on the x axis.

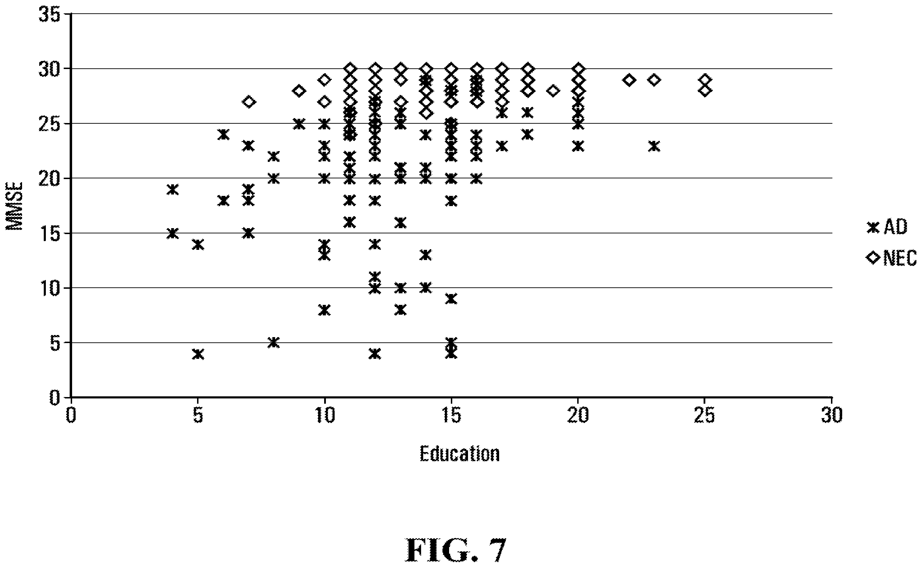

- Figure 7: An aspect of illustrative non-limiting results of MMSE scores distribution of the total sample population on the y axis and years of education on the x axis.

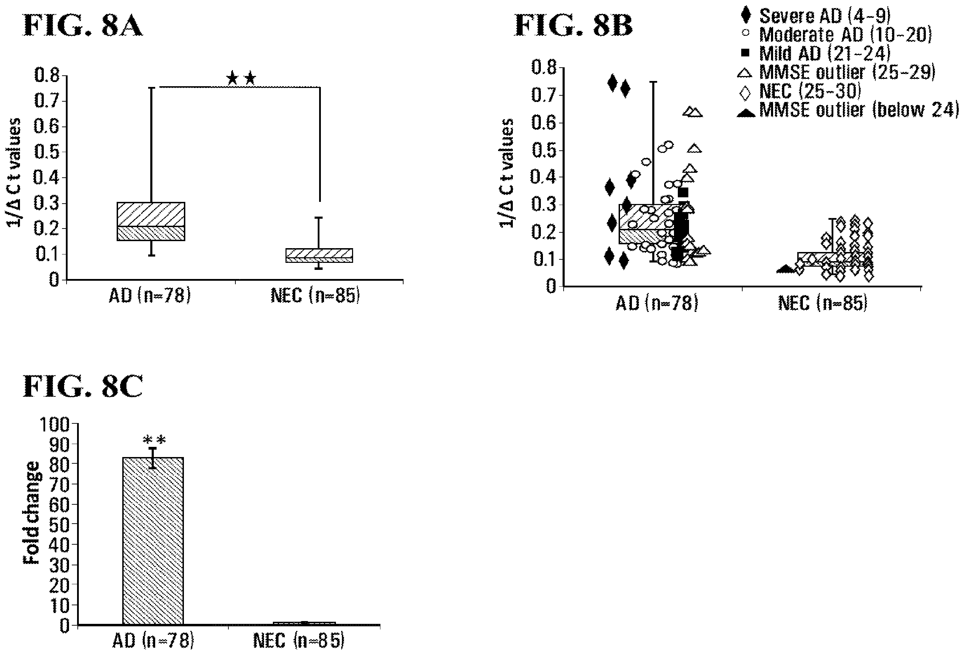

- Figure 8: Circulating miR-34c level is associated with AD status. (A) An aspect of illustrative non-limiting results of Box plot distribution of qPCR results from 160 plasma samples: 78 AD and 85 NEC. There is a significant difference between plasma level of miR-34c in AD and in NEC (** represents p < 0.01). p value for the box plot is calculated using 1 / delta C_{T} values. (B) Box plot as in (A) showing individual results. (C) Histogram showing the fold change from the results in (A) of AD relative to NEC. p-value for the fold change (2^{-delta deltaC}t) is calculated using delta C_{T} values. There is a significant difference for the fold change values. (** represents p < 0.01).

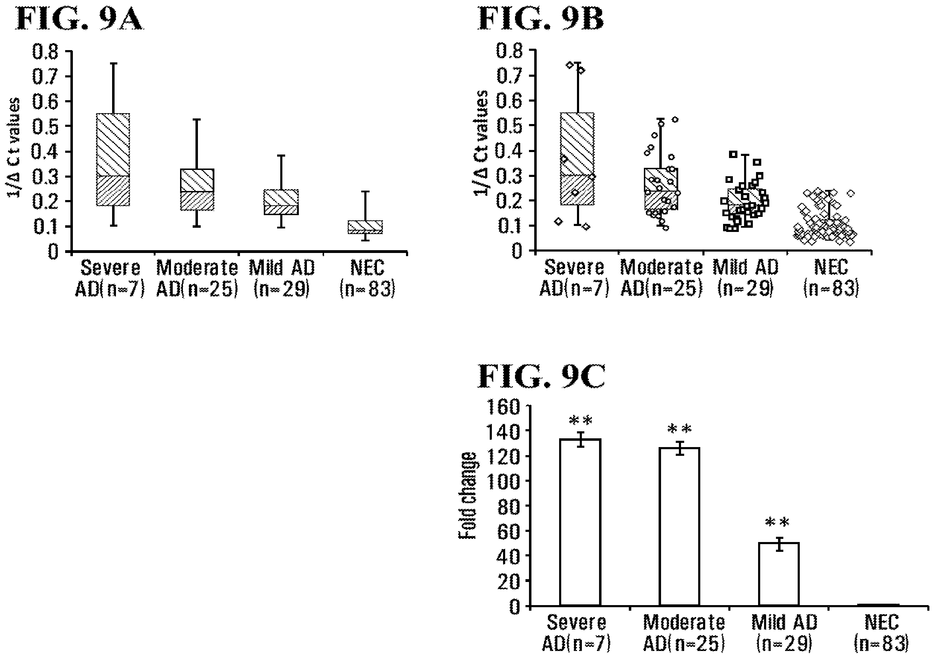

- Figure 9: Circulating miR-34c level is associated with AD status. (A) An aspect of illustrative non-limiting results of Box plot distribution of qPCR results from 7 severe AD (MMSE 4-9), 25 moderate (MMSE 10-20), 29 mild AD (MMSE 21-24), and 83 NEC individuals. p value for the box plot is calculated using 1 / delta C_{T} values. (B) Box plot as in (A) showing individual results. (C) Histogram showing the fold change from the results in (A) of AD relative to NEC. p-value for the fold change (2^{-delta deltaC}t) is calculated using delta C_{T} values. There is a significant difference for the fold change values. (** represents p<0.01).

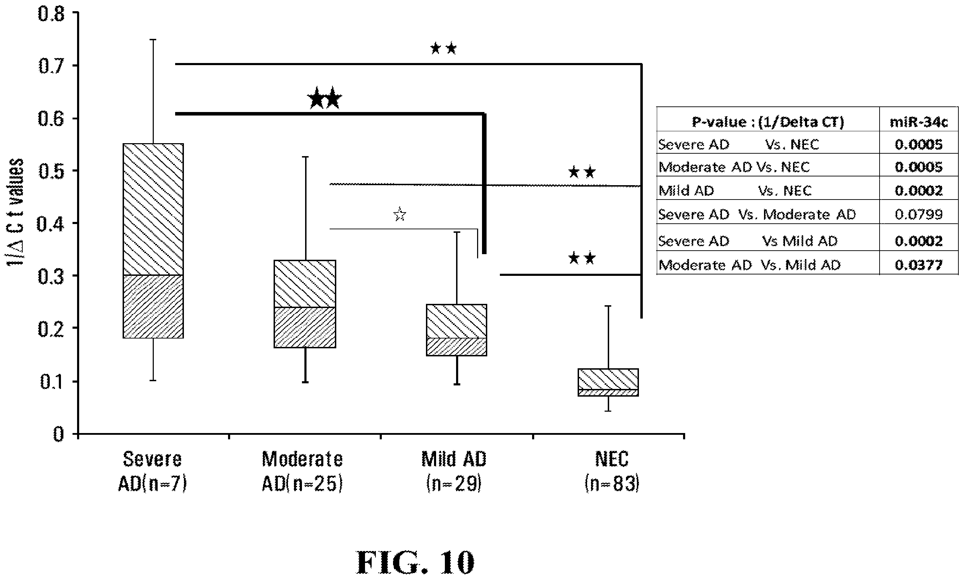

- Figure 10: Circulating miR-34c level is associated with MMSE score. An aspect of illustrative non-limiting results of Box plot distribution from Figure 9 (A) where the 1/delta C_{T} value is used to determine the p value, shown in the box on the right. (** represents p < 0.01 and * represents p < 0.05.)

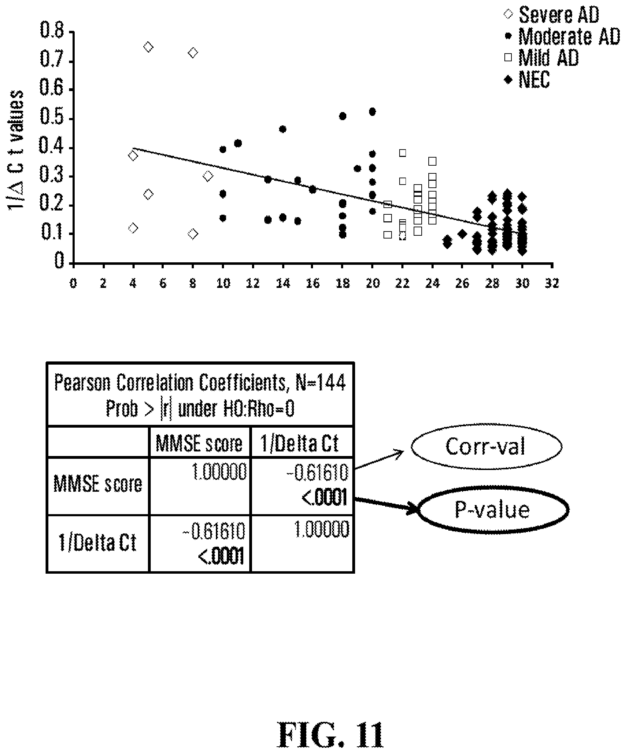

- Figure 11: Correlation between plasma level of miR-34c and MMSE scores. The results from Fig. 9 are plotted against the MMSE score obtained by the individual from which was obtained the plasma sample. The curve has the following equation: y = -0.011x + 0.441 with an R² value of 0.379, indicating that the decrease of MMSE score is correlated with an increase of inverted delta C_{T} value of plasma miR-34c. On the bottom is shown the Pearson Correlation Coefficients.

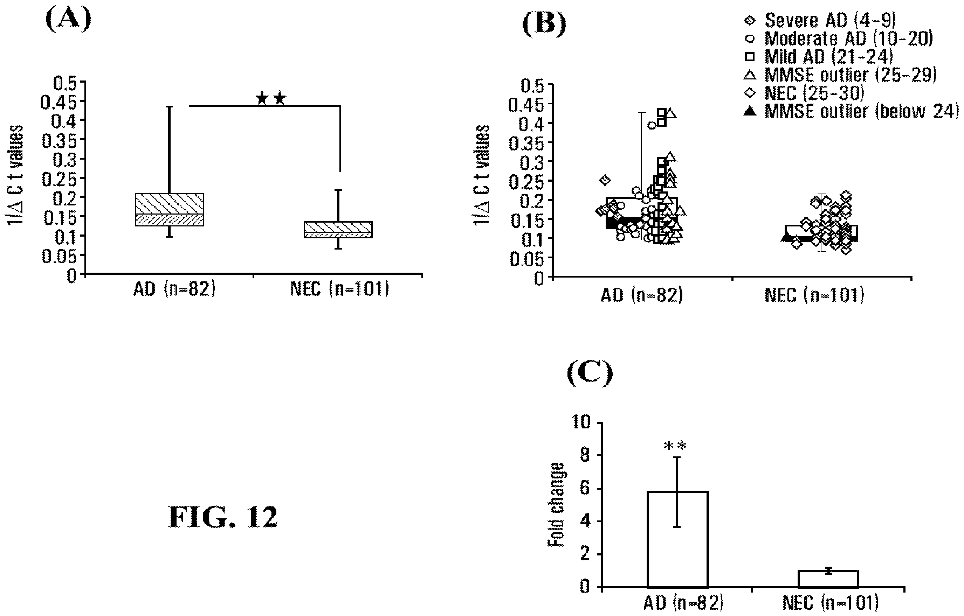

- Figure 12: Circulating miR-34a level is associated with AD status. (A) An aspect of illustrative non-limiting results of Box plot distribution of qPCR results from 183 plasma samples: 82 AD and 101 NEC. There is a significant difference between plasma level of miR-34a in AD and in NEC (p < 0.01). p value for the box plot is calculated using 1 / delta C_{T} values. (B) Box plot as in (A) showing individual results. (C) Histogram showing the fold change from the results in (A) of AD relative to NEC. p-value for the fold change (2^{-delta deltaC}t) is calculated using delta C_{T} values. There is a significant difference for the fold change values. (** represents p < 0.01).

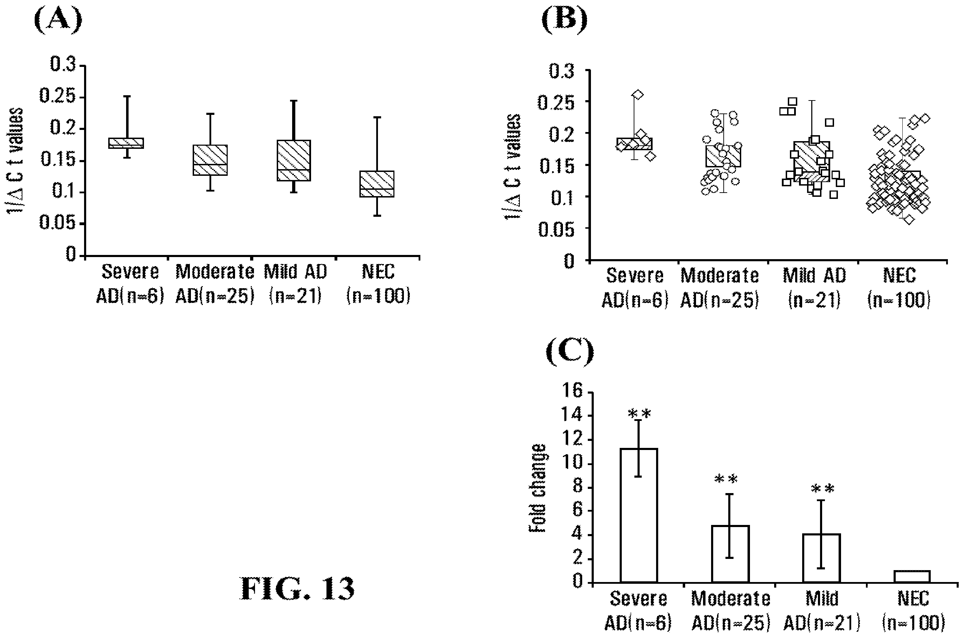

- Figure 13: Circulating miR-34a level is associated with AD status. (A) An aspect of illustrative non-limiting results of Box plot distribution of qPCR results from 6 severe AD (MMSE 4-9), 25 moderate (MMSE 10-20), 21 mild AD (MMSE 21-24), and 100 NEC individuals. p value for the box plot is calculated using 1 / delta C_{T} values. (B) Box plot as in (A) showing individual results. (C) Histogram showing the fold change from the results in (A) of AD relative to NEC. p-value for the fold change (2^{-delta deltaC}t) is calculated using delta C_{T} values. There is a significant difference for the fold change values. (** represents p < 0.01).

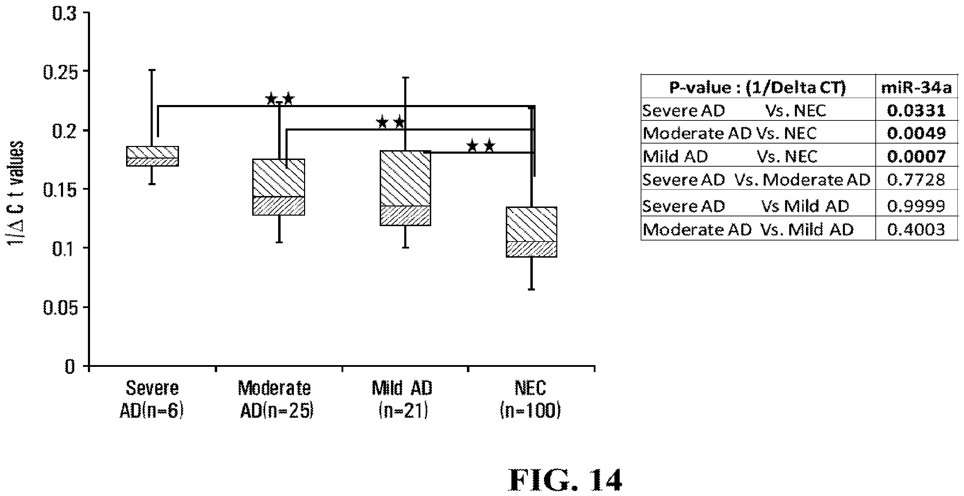

- Figure 14: Circulating miR-34a level is associated with MMSE score. Box plot distribution from Figure 13 (A) where the 1/delta C_{T} value is used to determine the p value, shown in the box on the right. (** represents p<0.01)

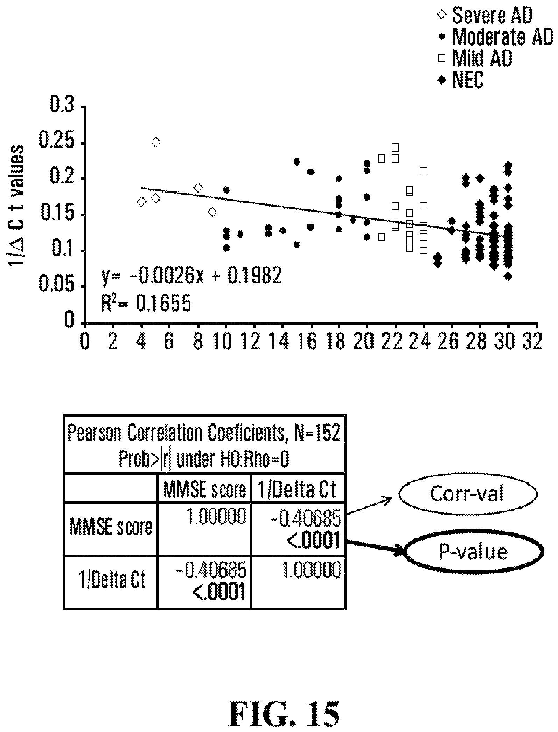

- Figure 15: Correlation between plasma level of miR-34a and MMSE scores. The results from Fig. 13 are plotted against the MMSE score obtained by the individual from which was obtained the plasma sample. The curve has the following equation: y = -0.002x + 0.198 with an R² value of 0.165, indicating that the decrease of MMSE score is correlated with an increase of inverted delta C_{T} value of plasma miR-34a and that the relation between the two variables is negatively associated using the Scheffe multiple comparison method for 1/delta C_{T} values. On the bottom is shown the Pearson Correlation Coefficients.

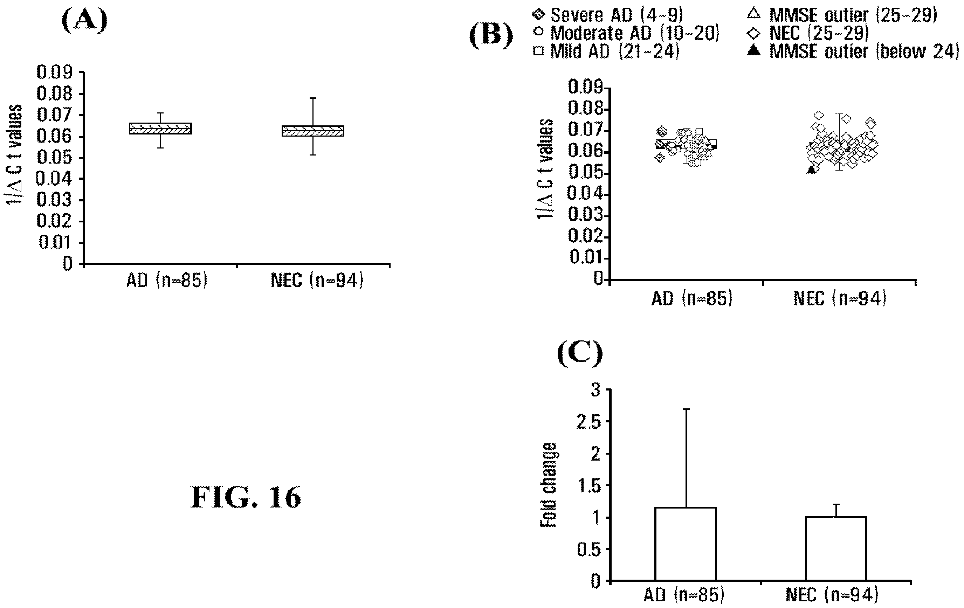

- Figure 16: No correlation between PBMC level of miR-34c and AD status. (A) An aspect of illustrative non-limiting results of Box plot distribution of qPCR results from 85 AD and 94 NEC. No significant difference. The p value is calculated using 1/delta C_{T} values. (p > 0.05). (B) Box plot as in (A) showing individual results. (C) Histogram showing the fold change from the results in (A) of AD relative to NEC. p-value for the fold change (2^{-delta deltaC}t) is calculated using delta C_{T} values. There is no significant difference for the fold change values. (p > 0.05).

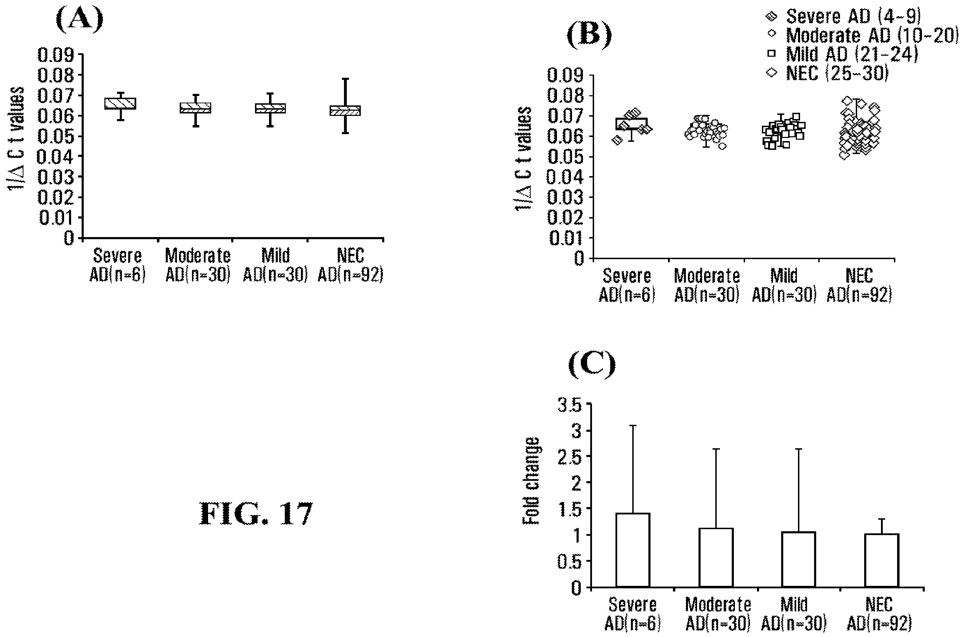

- Figure 17: No correlation between PBMC level of miR-34c and MMSE scores. (A) An aspect of illustrative non-limiting results of Box plot distribution of qPCR results from 6 severe AD (MMSE 4-9), 30 moderate (MMSE 10-20), 30 mild AD (MMSE 21-24), and 92 NEC individuals. p value for the box plot is calculated using 1 / delta C_{T} values. There is no significant change (p > 0.05). (B) Box plot as in (A) showing individual results. (C) Histogram showing the fold change from the results in (A) of AD relative to NEC. p-value for the fold change (2^{-delta deltaC}t) is calculated using delta C_{T} values. There is no significant difference for the fold change values (p > 0.05).

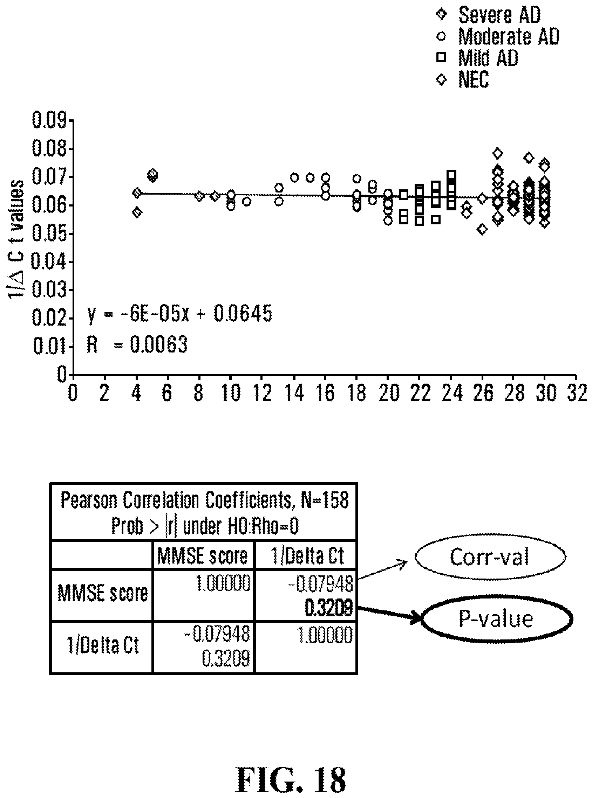

- Figure 18: No correlation between PBMC level of miR-34c and MMSE scores. The results from Fig. 16 are plotted against the MMSE score obtained by the individual from which was obtained the PBMC sample. The curve has the following equation: y = -0.00006x + 0.064 with an R² value of 0.006, indicating that there is no relation / association between the two variables, and the relation is not significant since the p value is 0.3209 (p > 0. 05). (using the Bonferroni multiple comparison method for 1 / delta C_{T} values).

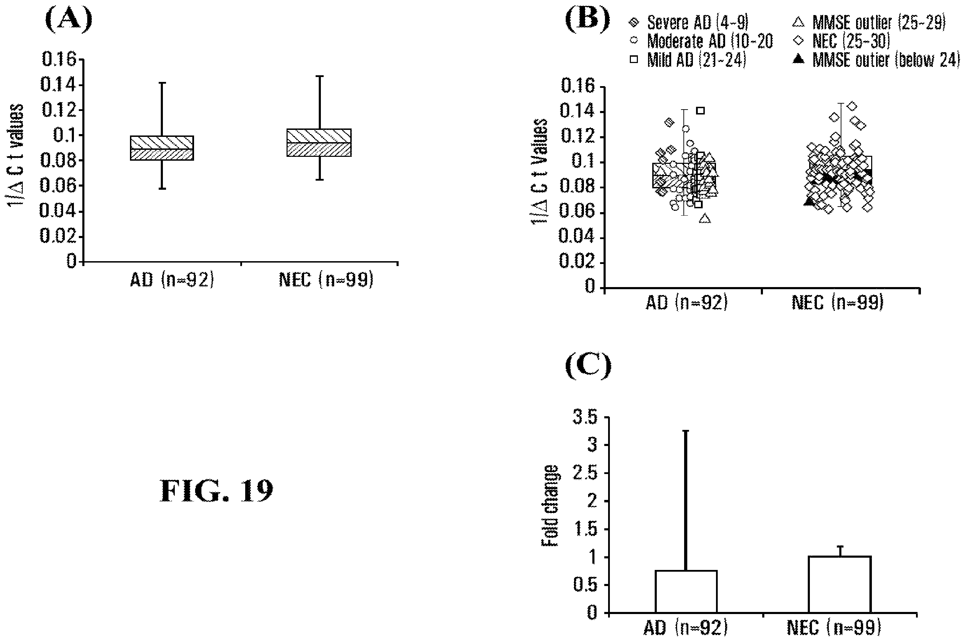

- Figure 19: No correlation between PBMC level of miR-34a and AD status. (A) An aspect of illustrative non-limiting results of Box plot distribution of qPCR results from 92 AD and 99 NEC. No significant difference. The p value is calculated using 1/delta C_{T} values. (p > 0.05). (B) Box plot as in (A) showing individual results. (C) Histogram showing the fold change from the results in (A) of AD relative to NEC. p-value for the fold change (2^{-delta deltaC}t) is calculated using delta C_{T} values. There is no significant difference for the fold change values. (p > 0.05).

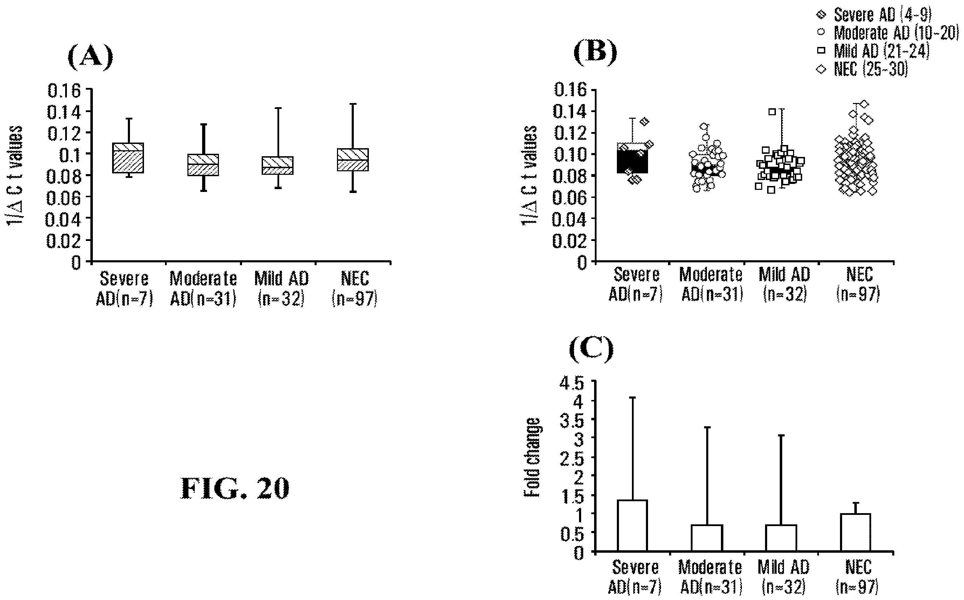

- Figure 20: No correlation between PBMC level of miR-34a and MMSE scores. (A) An aspect of illustrative non-limiting results of Box plot distribution of qPCR results from 7 severe AD (MMSE 4-9), 31 moderate (MMSE 10-20), 32 mild AD (MMSE 21-24), and 97 NEC individuals. p value for the box plot is calculated using 1 / delta C_{T} values. There is no significant change (p > 0.05). (B) Box plot as in (A) showing individual results. (C) Histogram showing the fold change from the results in (A) of AD relative to NEC. p-value for the fold change (2^{-delta deltaC}t) is calculated using delta C_{T} values. There is no significant difference for the fold change values (p > 0.05).

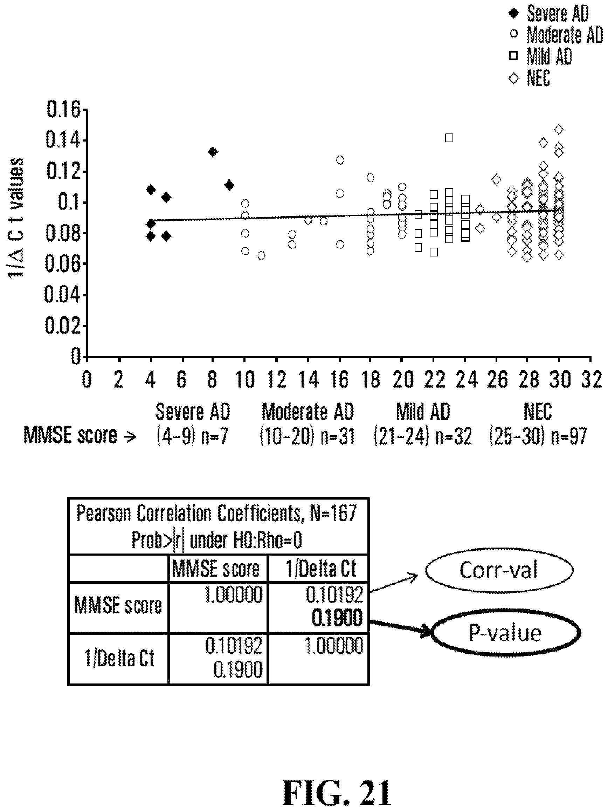

- Figure 21: No correlation between PBMC level of miR-34a and MMSE scores. The results from Fig. 19 are plotted against the MMSE score obtained by the individual from which was obtained the PBMC sample. The curve has the following equation: y = -0.000x + 0.087 with an R² value of 0.010, indicating that there is no relation / association between the two variables, and the relation is not significant since the p value is 0.1900 (p > 0. 05). (using the Bonferroni multiple comparison method for 1 / delta C_{T} values).

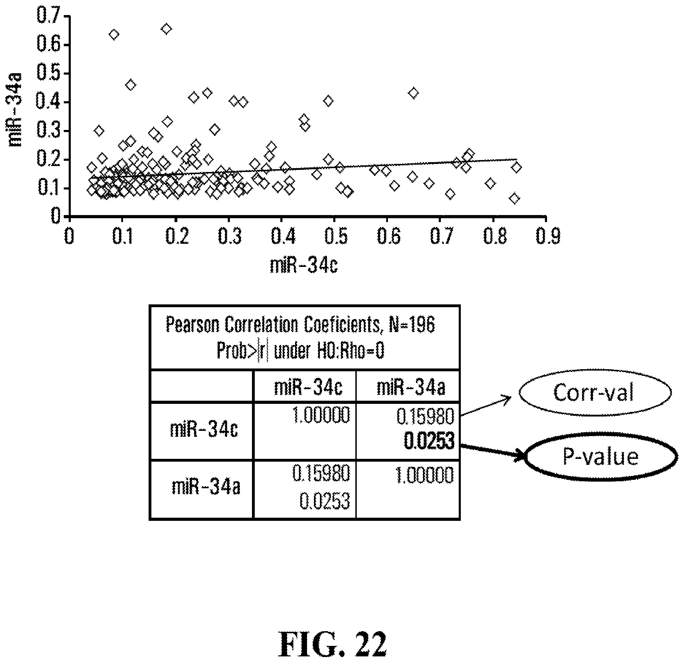

- Figure 22: Weak correlation between plasma level of miR-34a and miR-34c. The results from figures 8 to 15 are grouped using level of miR-34c on the x axis and level of miR-34a on the y axis. The MMSE scores are not considered in this figure. The data used only includes samples from which both miR-34a and miR-34c levels could be determined. The curve has the following equation: y = 0.080x + 0.134 with an R² value of 0.025, indicating that there is a weak positive relation between the two variables, and the relation is significant since the p value is of 0.0253 (p < 0.05).

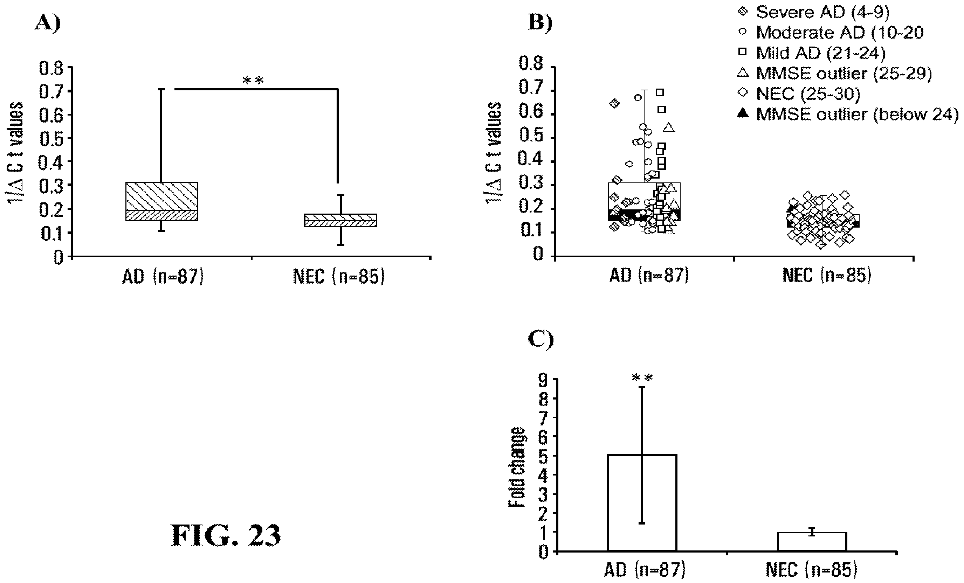

- Figure 23: Circulating level of miR-181b is associated with AD status. (A) An aspect of illustrative non-limiting results of Box plot distribution of qPCR results from 87 AD and 85 NEC. The p value for the box plot is calculated using 1 / delta C_{T} values. (** represents p < 0.01). (B) Box plot as in (A) showing individual results. (C) Histogram showing the fold change from the results in (A) of AD relative to NEC. The p-value for the fold change (2^{-delta deltaC}t) is calculated using delta C_{T} values. There is a significant difference for the fold change values. (** represents p < 0.01).

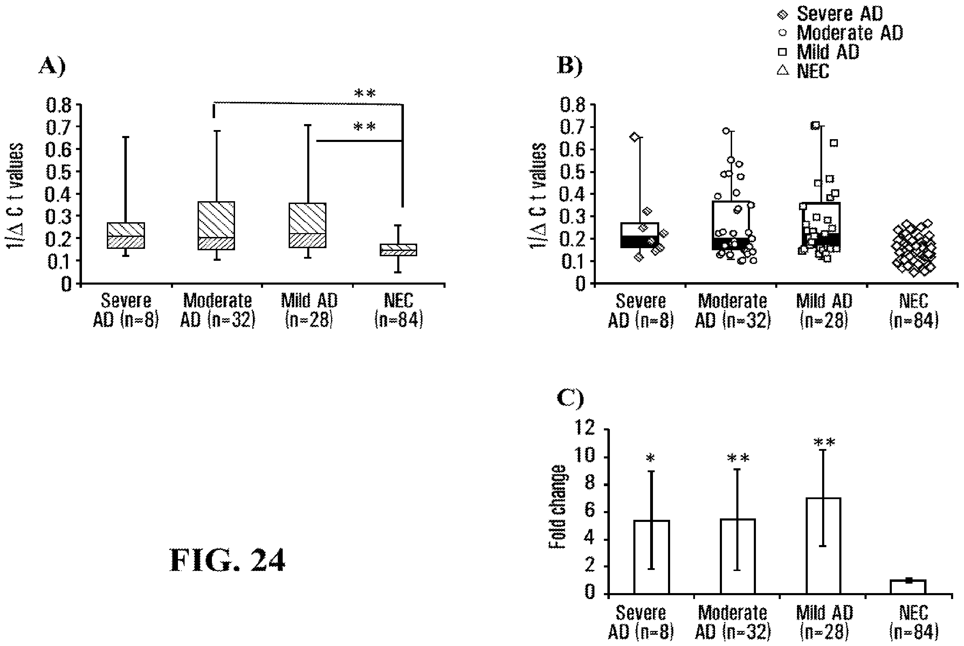

- Figure 24: Circulating level of miR-181b is associated with AD status. (A) An aspect of illustrative non-limiting results of Box plot distribution of qPCR results from 8 severe AD (MMSE 4-9), 32 moderate (MMSE 10-20), 28 mild AD (MMSE 21-24), and 84 NEC individuals. The p value for the box plot is calculated using 1 / delta C_{T} values. (** represents p < 0.01). (B) Box plot as in (A) showing individual results. (C) Histogram showing the fold change from the results in (A) of AD relative to NEC. p-value for the fold change (2^{-delta deltaC}t) is calculated using delta C_{T} values. There is a significant difference for the fold change values. (** represents p < 0.01 and * represents p < 0.05).

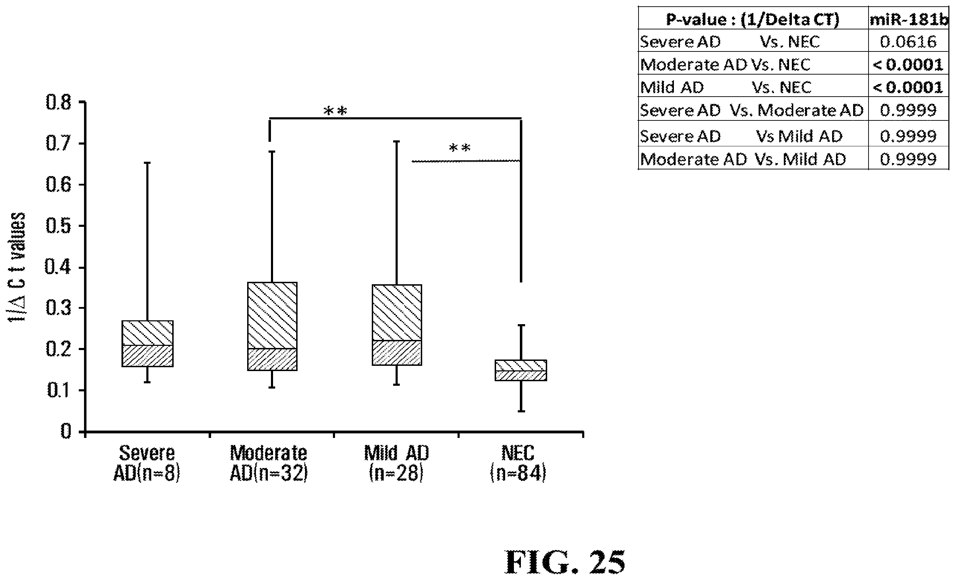

- Figure 25: Circulating level of miR-181b is associated with MMSE score. Box plot distribution from Figure 23 (A) where the 1/delta C_{T} value is used to determine the p value, shown in the box on the right. (** represents p < 0.01)

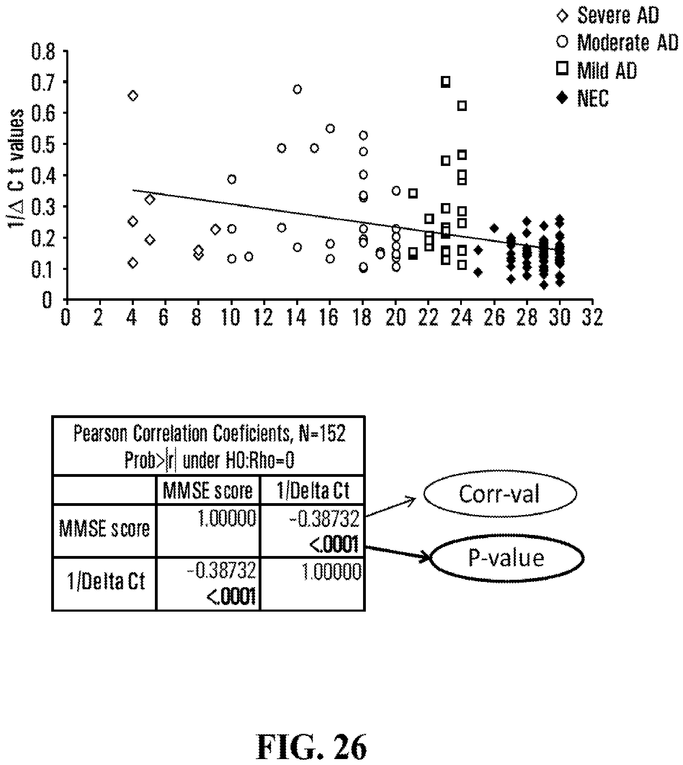

- Figure 26: Correlation between plasma level of miR-181b and MMSE scores. The results from Fig. 23 are plotted against the MMSE score obtained by the individual from which was obtained the plasma sample. The curve has the following equation: y = -0.007x + 0.382 with an R² value of 0.15, indicating that the decrease of MMSE score is correlated with an increase of inverted delta C_{T} value of plasma miR-181b and that the relation between the two variables is negatively associated, and that the relation / association between them is significant since the p value using the Scheffe multiple comparison method for 1/delta C_{T} values is < 0.001. On the bottom is shown the Pearson Correlation Coefficients.

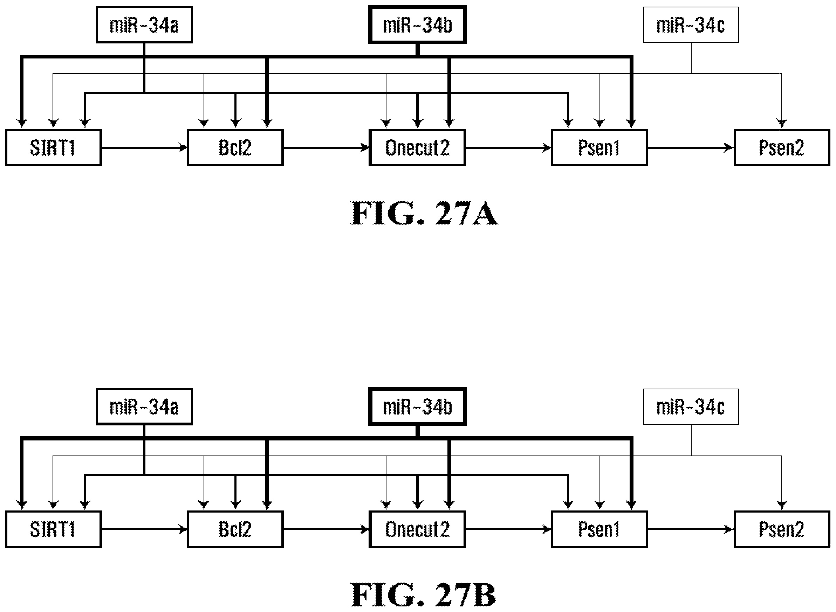

- Figure 27: Bioinformatic predictive shared target genes of miR-34a, b and c: Sirt1, Bcl2, Onecut2, and Presenilins 1 and 2, in sequence comparison analysis in (A) mouse and (B) human.

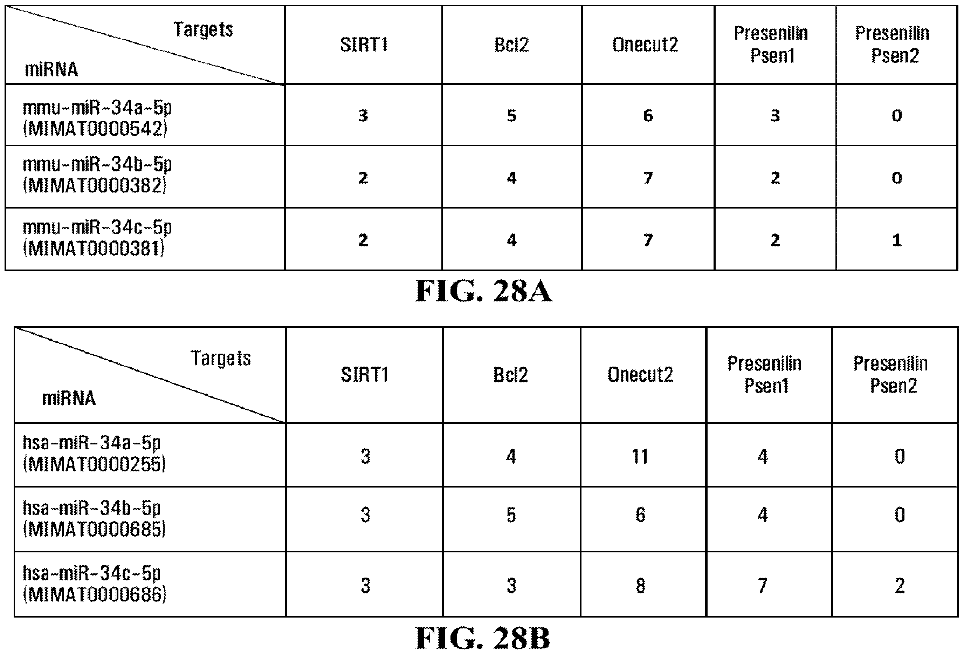

- Figure 28: Numbers of 'hits' per target gene in (A) mouse and (B) human.

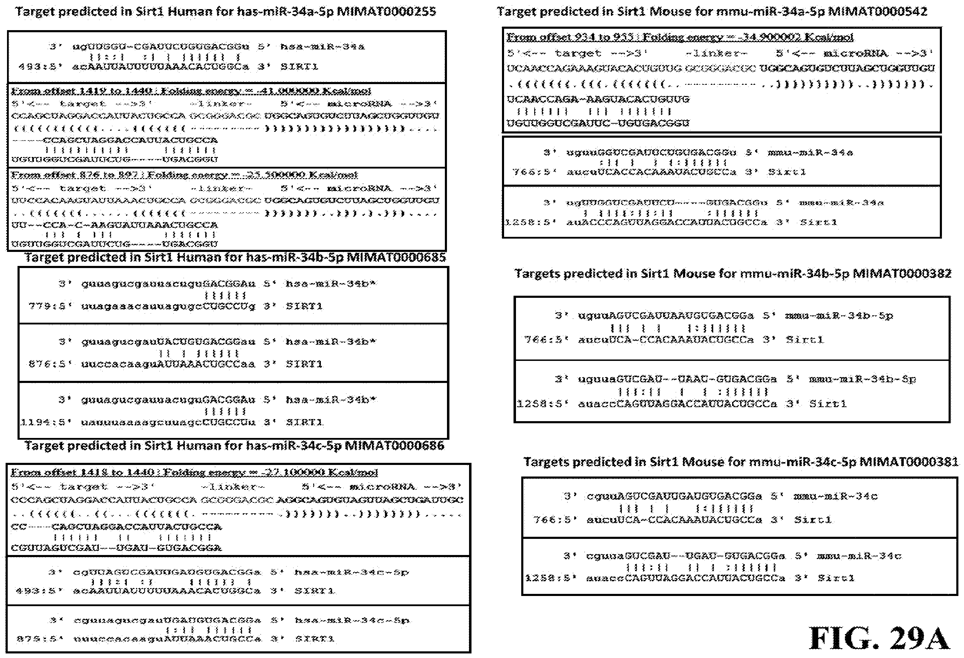

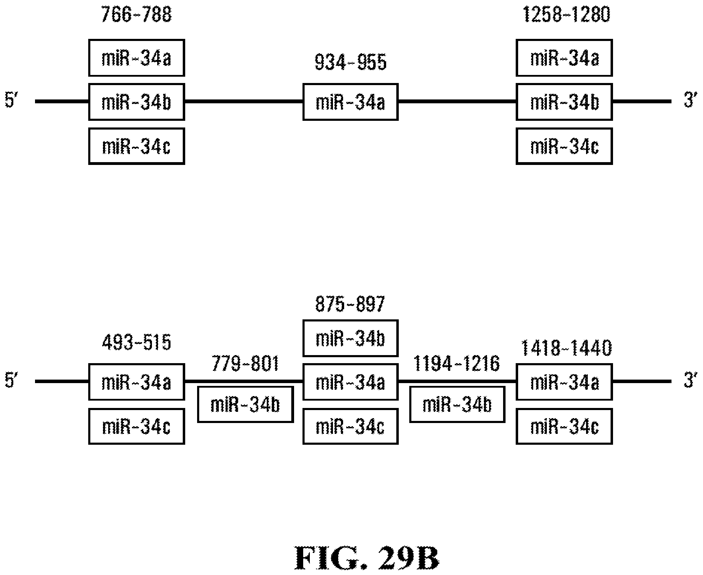

- Figure 29: (A) Putative binding sequences in mouse and in human for SIRT1 3' UTR. (B) Schematic diagrams of the locations of these binding sequences in mouse (top) and human (bottom).

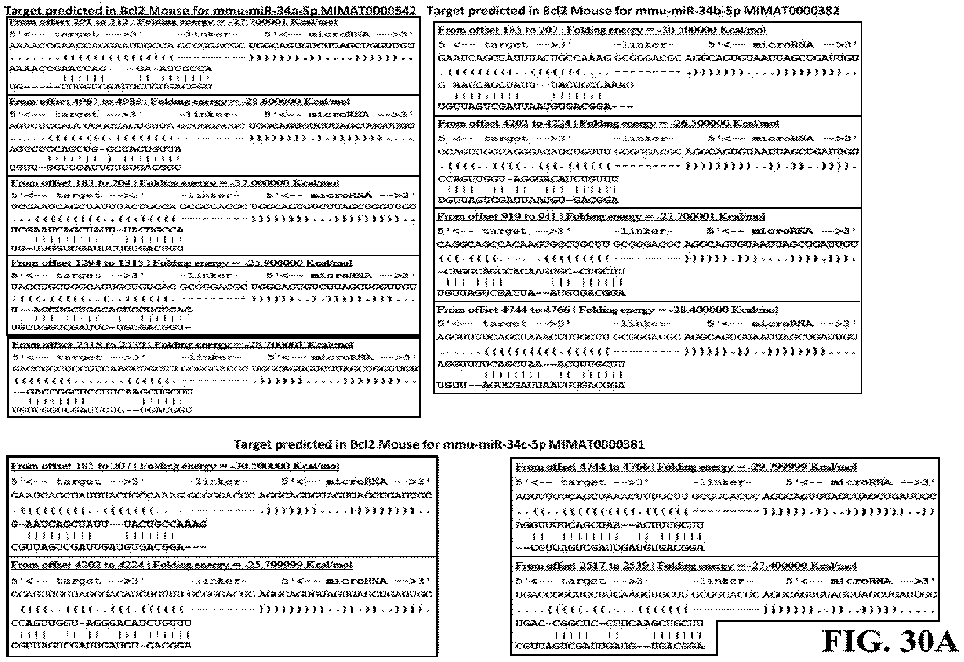

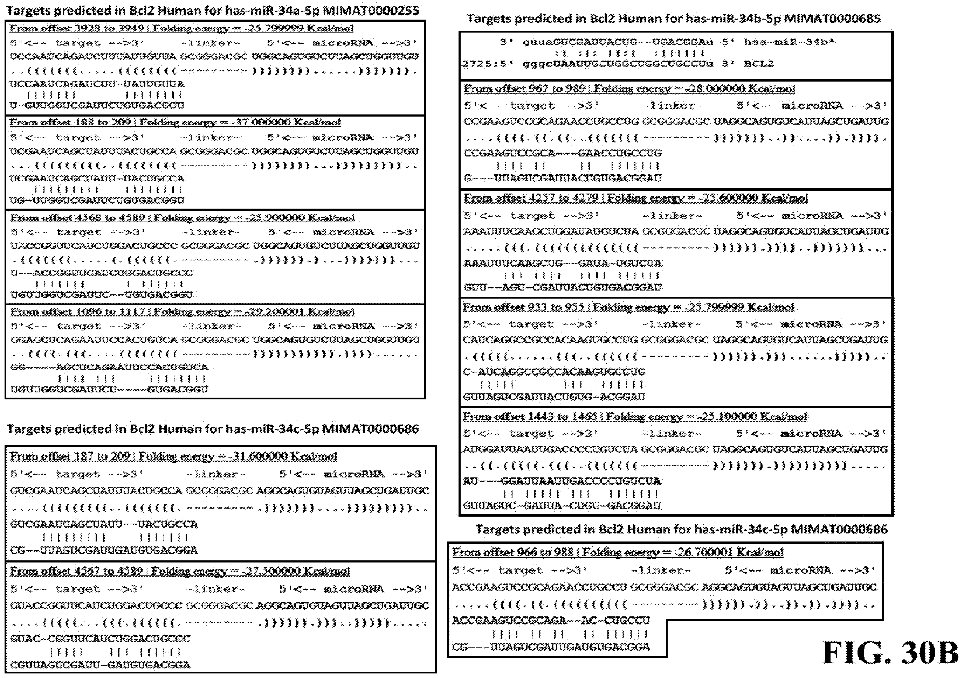

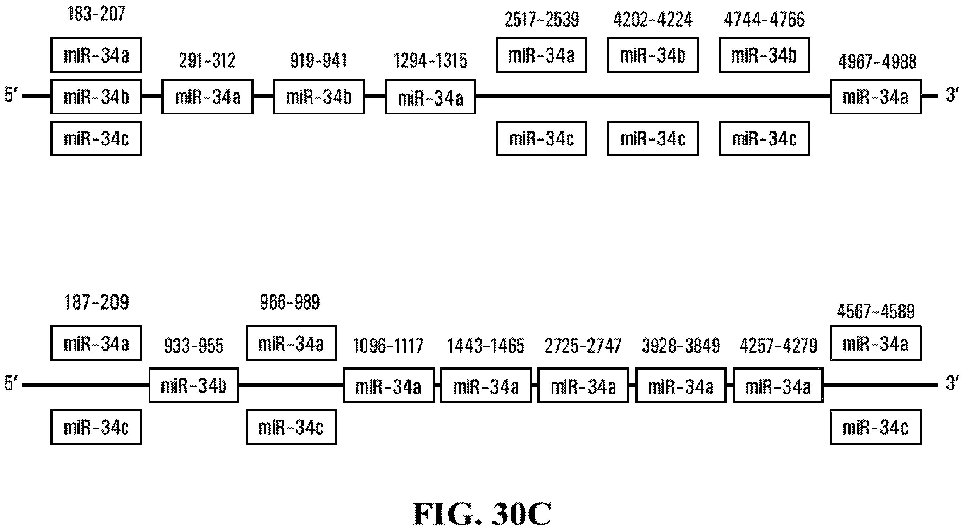

- Figure 30: (A) and (B) Putative binding sequences in mouse and in human for Bcl2 3' UTR. (C) Schematic diagrams of the locations of these binding sequences in mouse (top) and human (bottom).

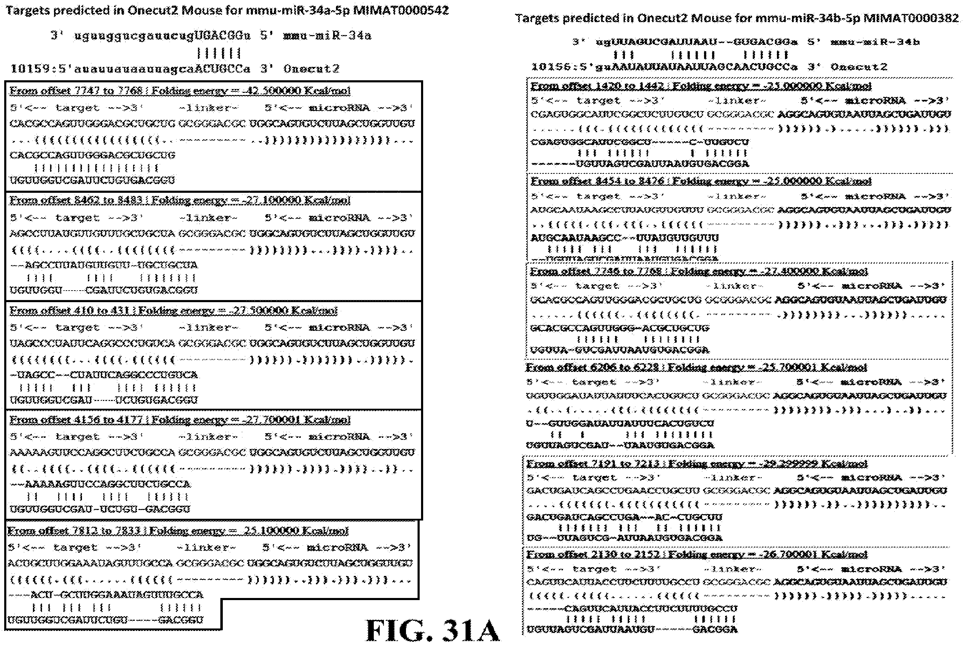

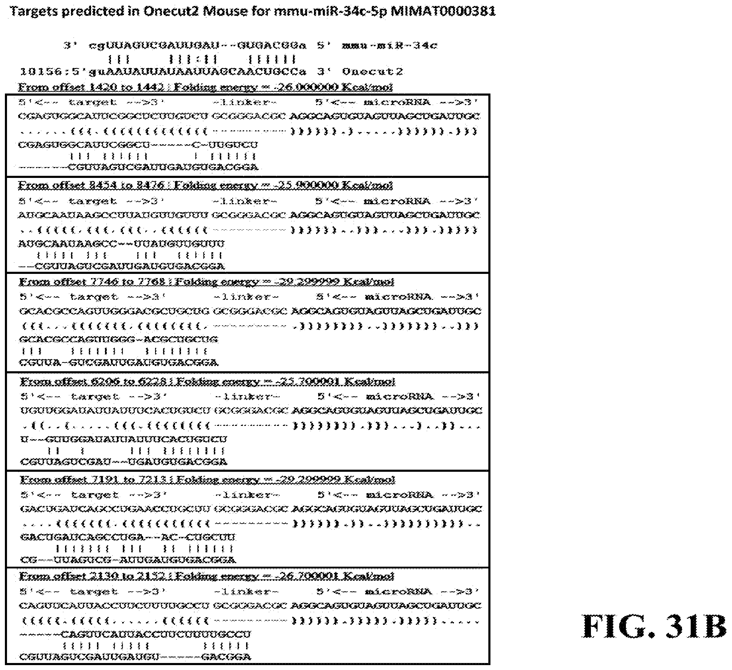

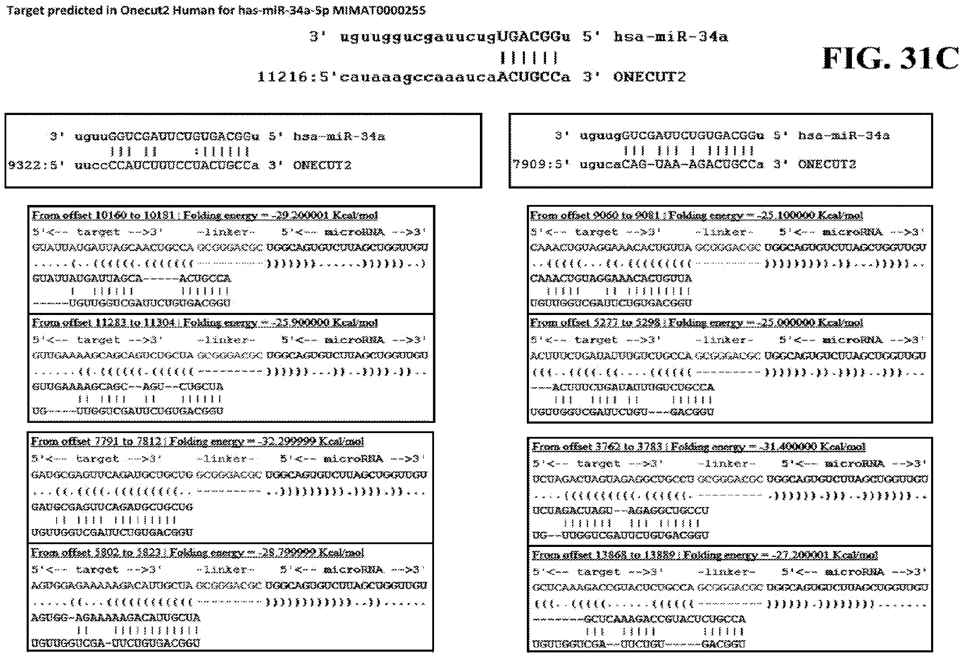

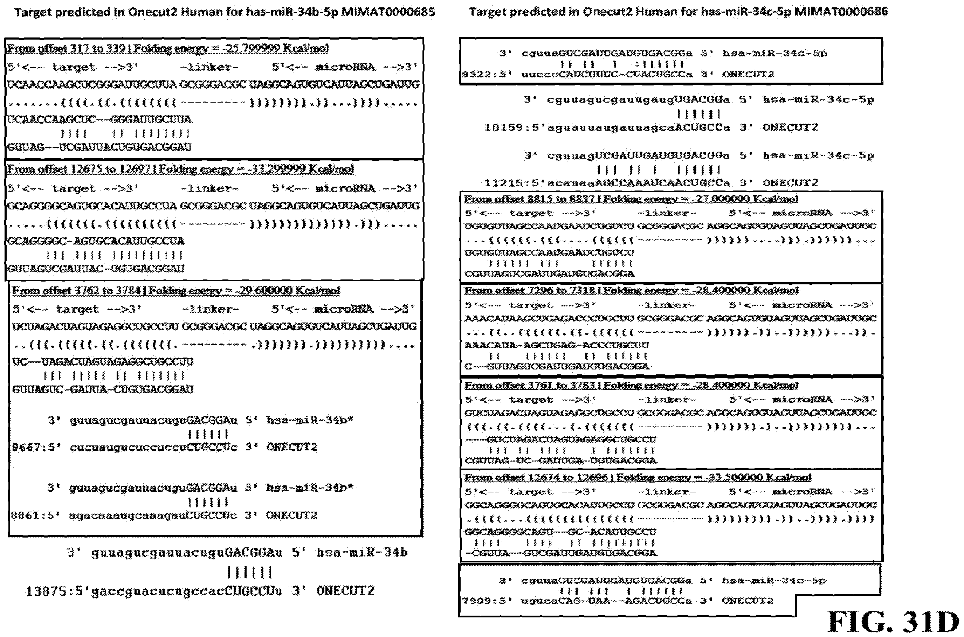

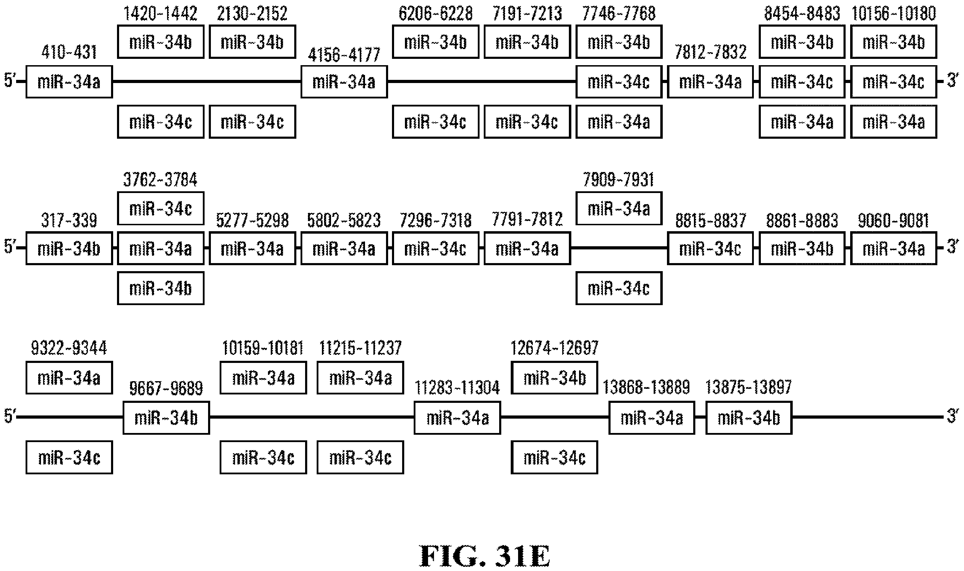

- Figure 31:: (A), (B), (C) and (D) Putative binding sequences in mouse and in human for Onecut2 3' UTR. (E) Schematic diagrams of the locations of these binding sequences in mouse (top) and human (bottom).

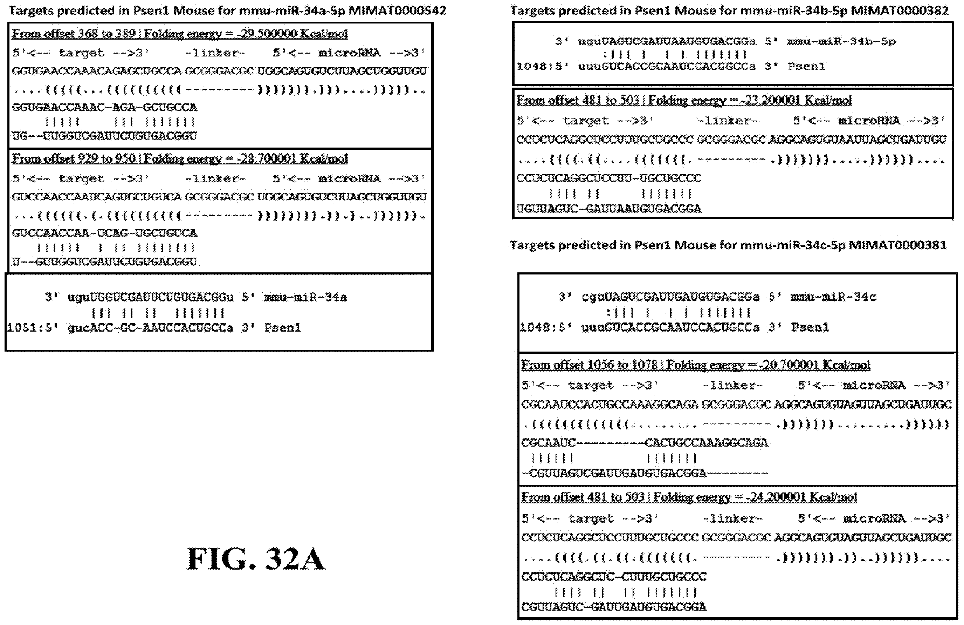

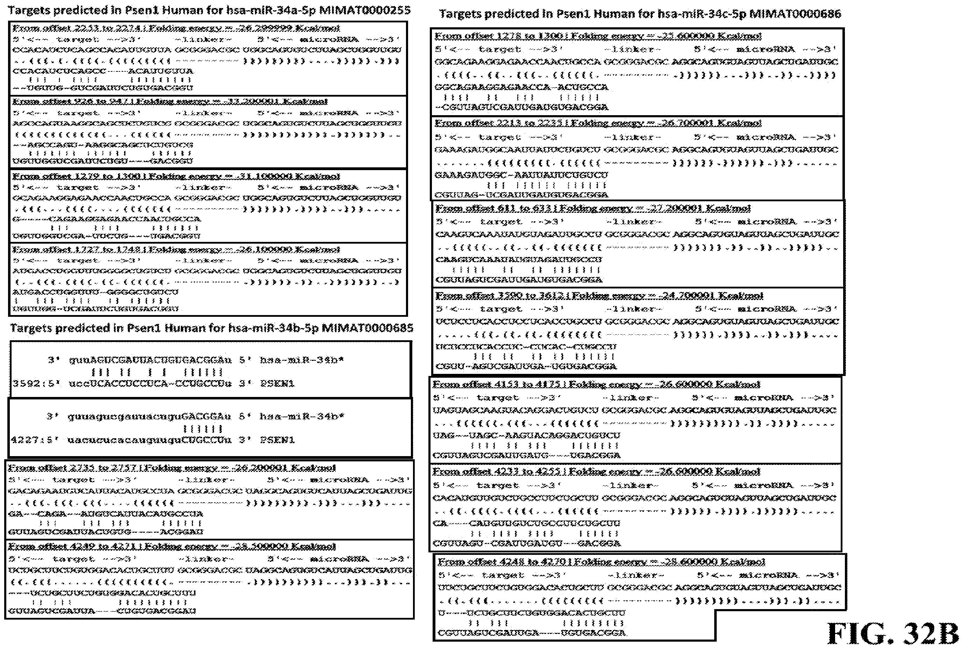

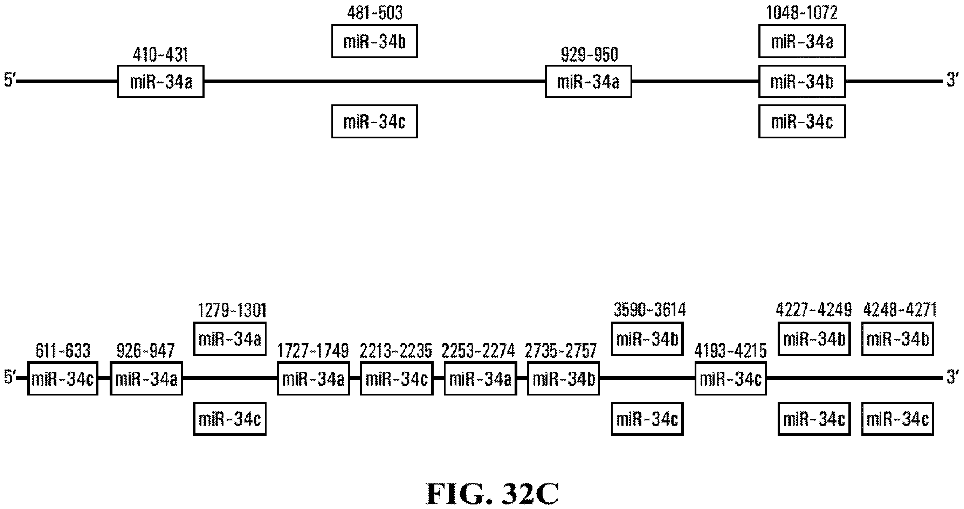

- Figure 32: (A) and (B) Putative binding sequences in mouse and in human for Psen1 3' UTR. (C) Schematic diagrams of the locations of these binding sequences in mouse (top) and human (bottom).

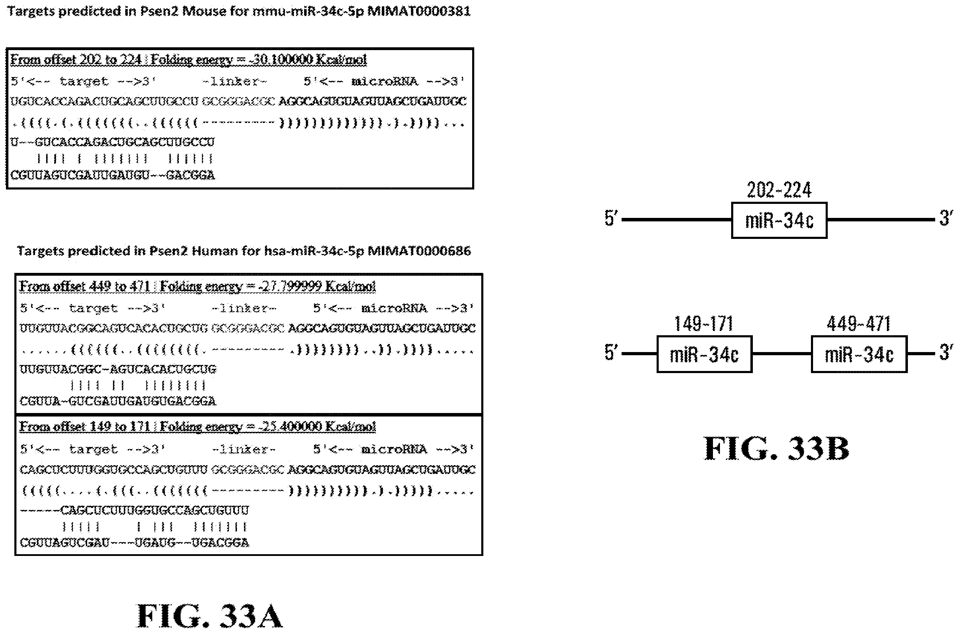

- Figure 33: (A) Putative binding sequences in mouse and in human for Psen2 3' UTR. (B) Schematic diagrams of the locations of these binding sequences in mouse (top) and human (bottom).

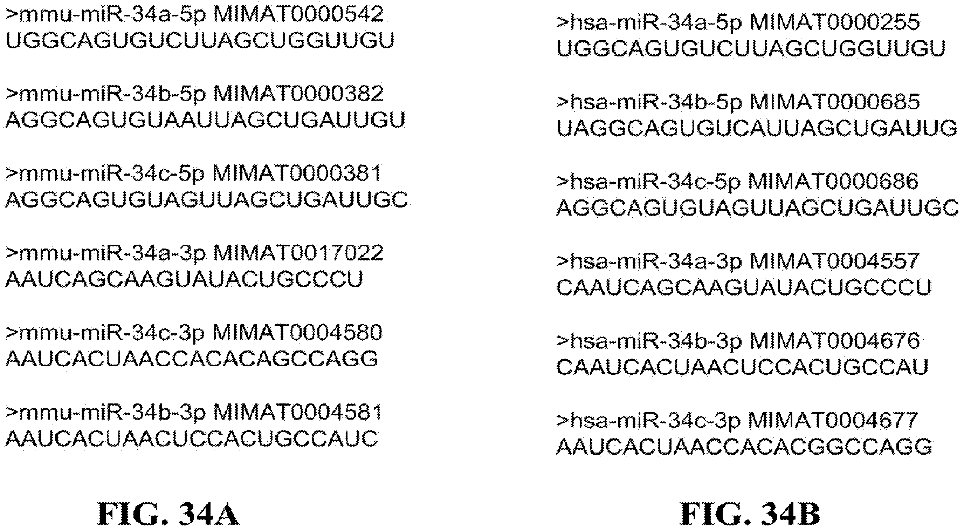

- Figure 34: Nucleotide sequences from mouse (A) and human (B) of mature miR-34a, miR-34b and miR-34c. (5p is the sense strand; 3p is the antisense strand).

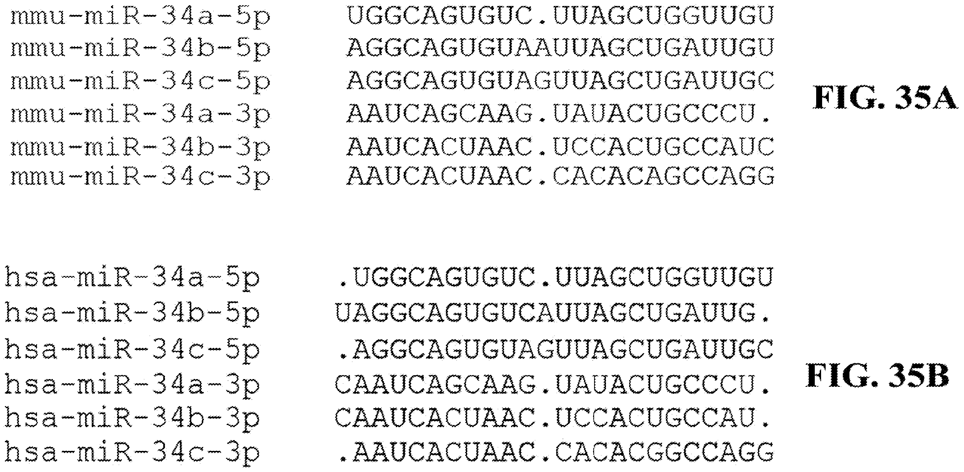

- Figure 35: Intra-species comparison of the three miR-34a, miR-34b and miR-34c microRNAs in mouse (A) and human (B). (5p is the sense strand; 3p is the antisense strand).

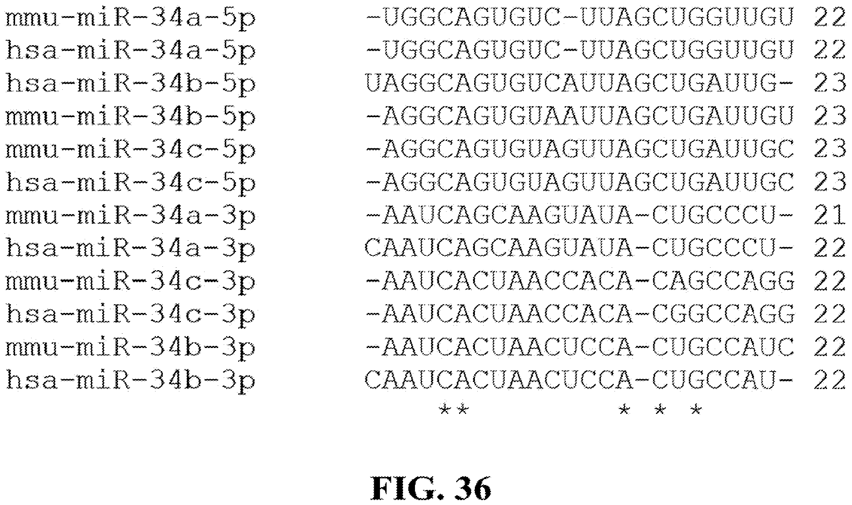

- Figure 36: Cross-species comparison of the sequence of each of the three miR-34a, miR-34b and miR-34c microRNAs between mouse (mmu) and human (hsa). (5p is the sense strand; 3p is the antisense strand).

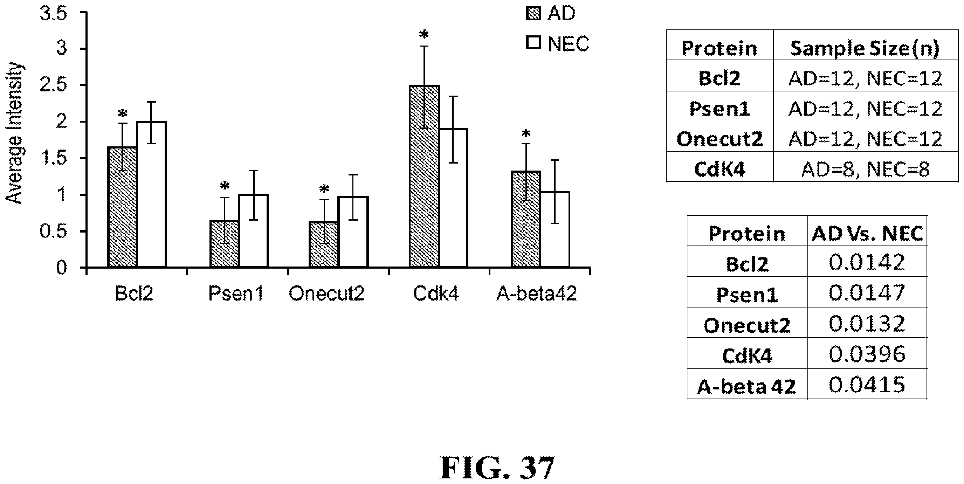

- Figure 37: An aspect of illustrative non-limiting histogram of circulating putative target proteins of miR-34c in a randomly selected first group of AD and NEC individuals. The putative target proteins are Bcl2, Psen1, Onecut2, Cdk4 and A-beta 42. All histograms represent the mean +/- standard deviation (SD) and * represents p < 0.05. All the protein samples are normalized with the ponceau S stained band. The sample size is shown in the box on the right while the AD relative to NEC p values are shown in the bottom box.

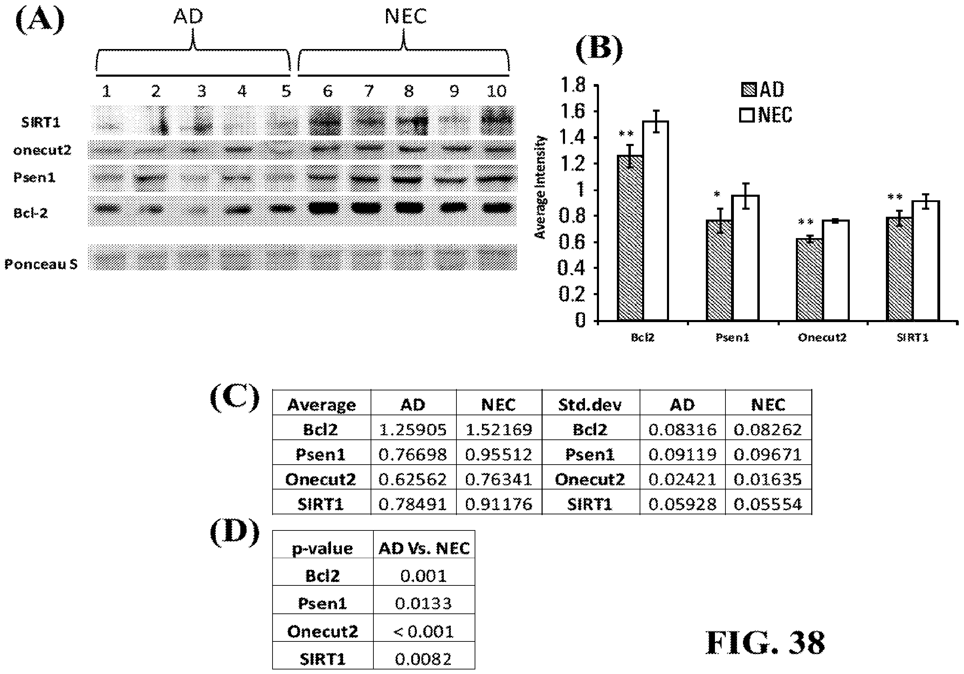

- Figure 38: 5 AD individuals having the highest circulating level of miR-34c and 5 NEC individuals having the lowest circulating level of miR-34c from the populations tested in Figure 37. (A) An aspect of illustrative non-limiting Western blot for the putative target protein. (B) Histogram showing the normalized circulating level of protein of (A), representing the mean +/- SD (n=5) and where ** represents p < 0.01 and * represents p < 0.05. (C) The raw data for the histograms in (B). (D) The p value for AD vs. NEC.

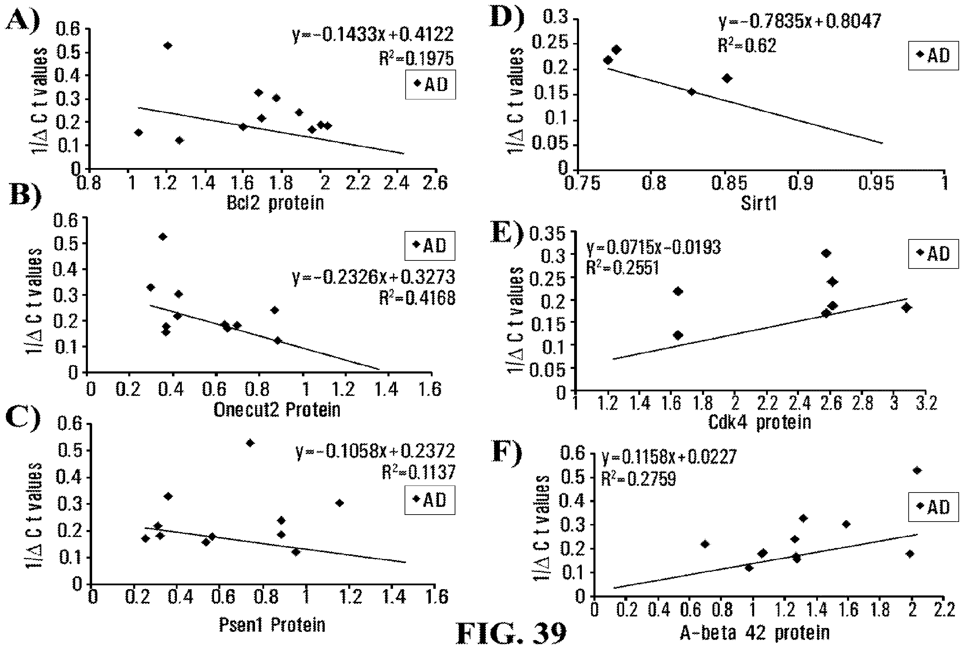

- Figure 39: Correlation between plasma level of miR-34c and putative target protein thereof. The curve equation is shown on each panel. Decreased target protein levels of miR-34c (Panels A, B, C, & D), inverse to delta C_{T} levels, but positive trends for increased protein levels of Cdk4 & A-beta 42 (Panels E & F), in AD human plasma samples.

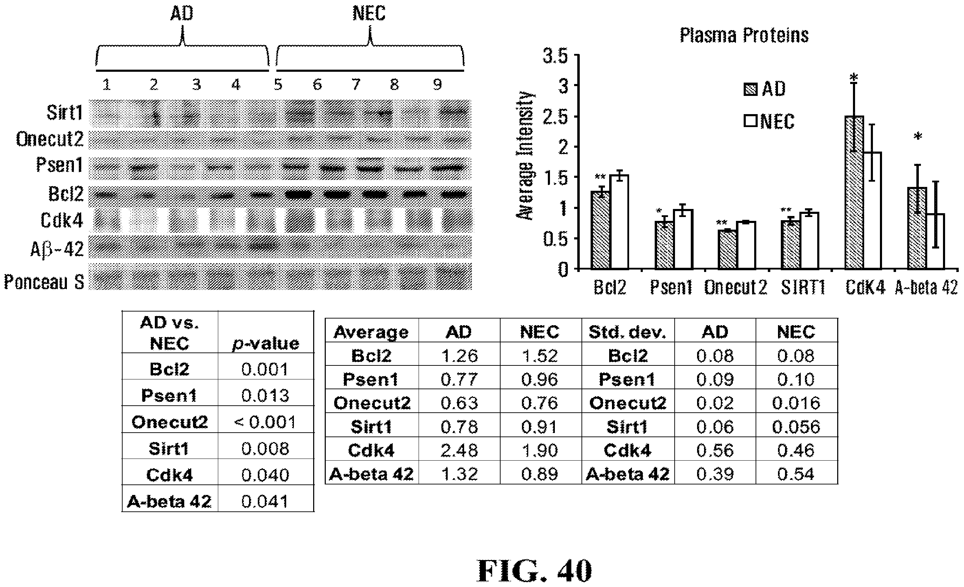

- Figure 40: An aspect of illustrative non-limiting Western blot for the putative target protein of microRNAs miR-34a and miR-34c. In the same individuals, pro-cell death protein Cdk4 and Aβ-42 are increased. Top panel: left, Western blot image; right, histogram of band level from (A). Bottom panel: left, p-value for comparison between plasma level of protein in AD relative to NEC samples; right, raw data of level of protein derived from western blot image. * represents p < 0.05 , ** represents p < 0.01. All protein samples in plasma are normalized with the Ponceau S-stained band.

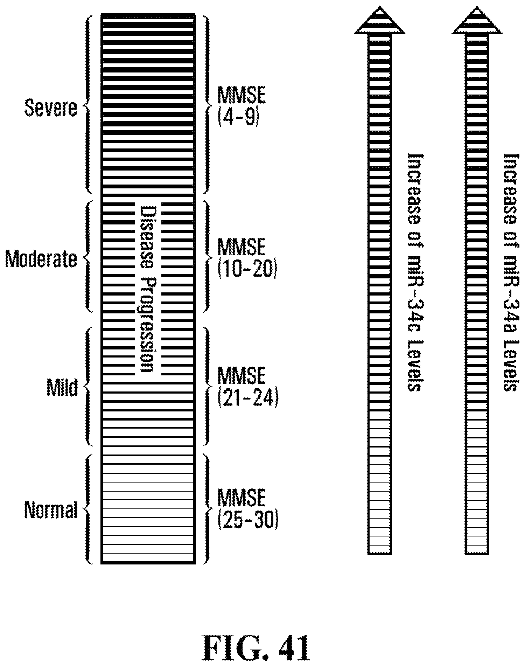

- Figure 41: Illustration of a working model which shows AD disease progression from normal (NEC) to mild, moderate, and severe cognitive decline, as marked by the decrease of MMSE scores with correlated increase in circulating level of miR-34a and/or miR-34c.

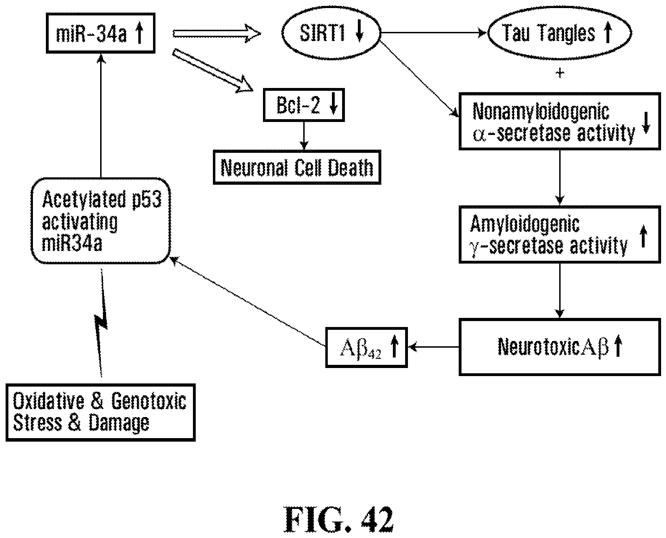

- Figure 42: Illustration of a possible mechanism underlying AD patho-physiological signs, such as Tau tangles, Aβ₄₂ and neuronal death induced by elevated level of miR-34a.

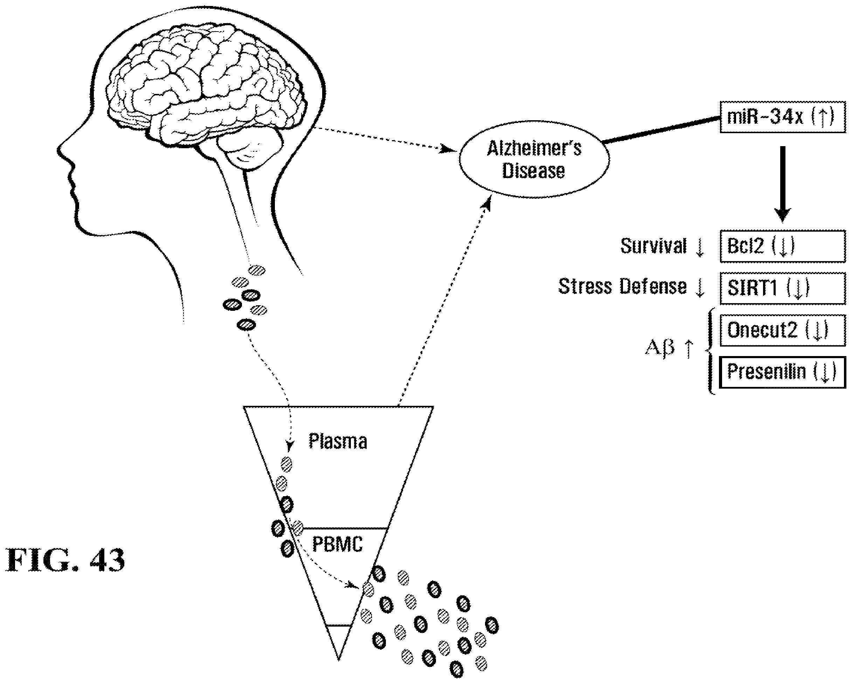

- Figure 43: Illustration of a possible triple effect subsequent to an increase in miR-34x (including miR-34a and/or miR-34b and/or miR-34c) in Alzheimer's disease.

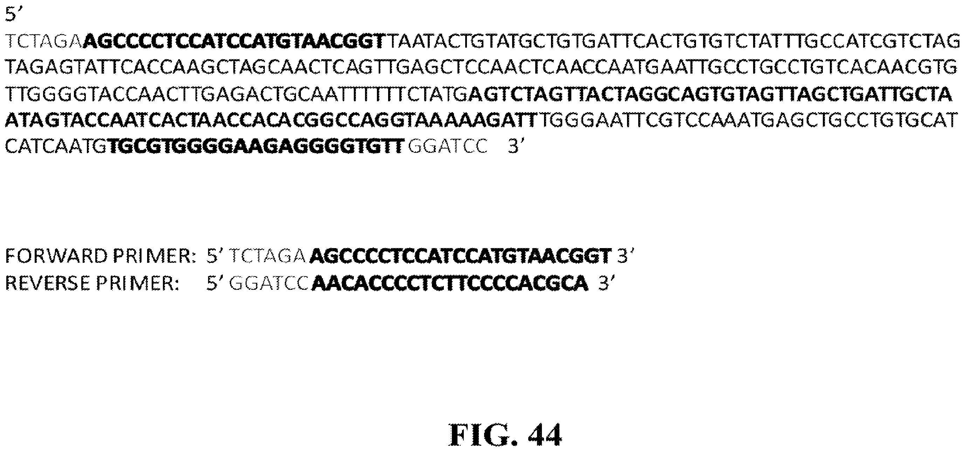

- Figure 44: Sequence description of human miR-34c-GFP construct. The precursor has-miR34c sequence is amplified from genomic DNA from HEK293 cells using the illustrated forward and reverse primers.

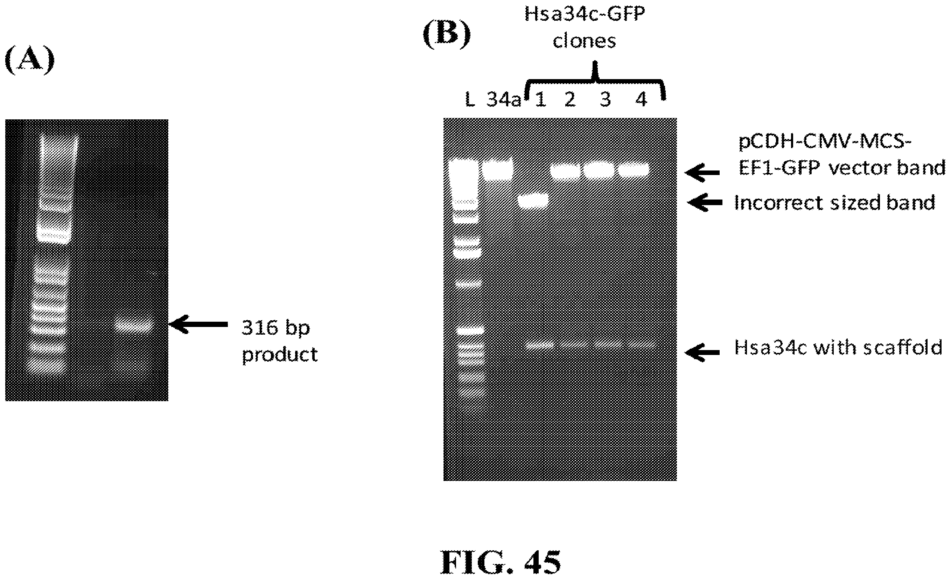

- Figure 45: Agarose gel image of PCR amplification results for cloning of has-miR-34c using the primers from Figure 44. (A) The PCR amplified band from genomic DNA.

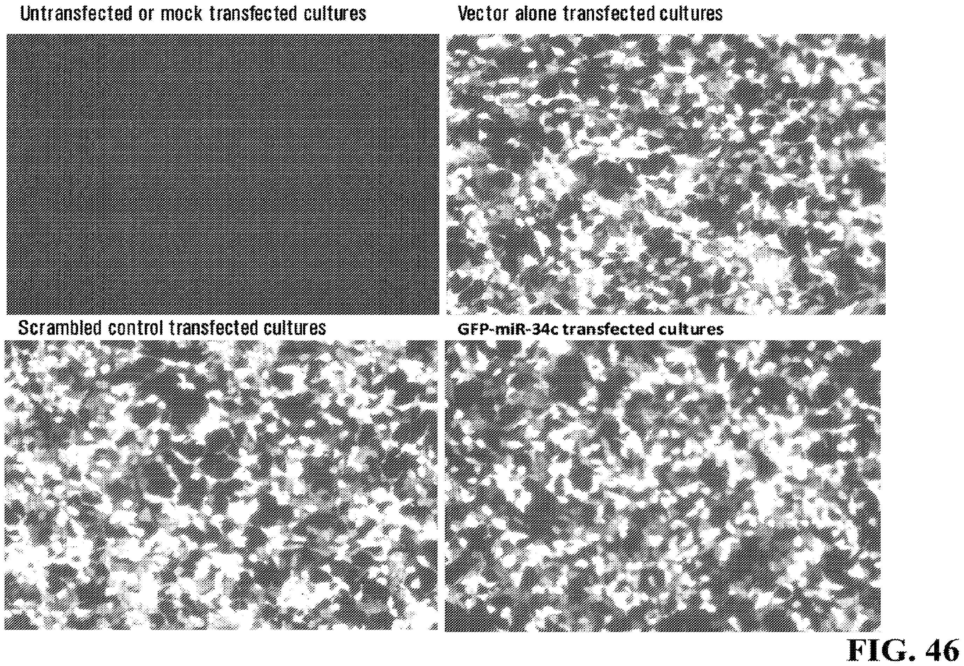

- Figure 46: The PCR amplification product from figure 45 was cloned into pCDH-CMV-MCS-EF1-copGFP (CD511B-1) and the resulting was transfected into cultured HEK293 cells.

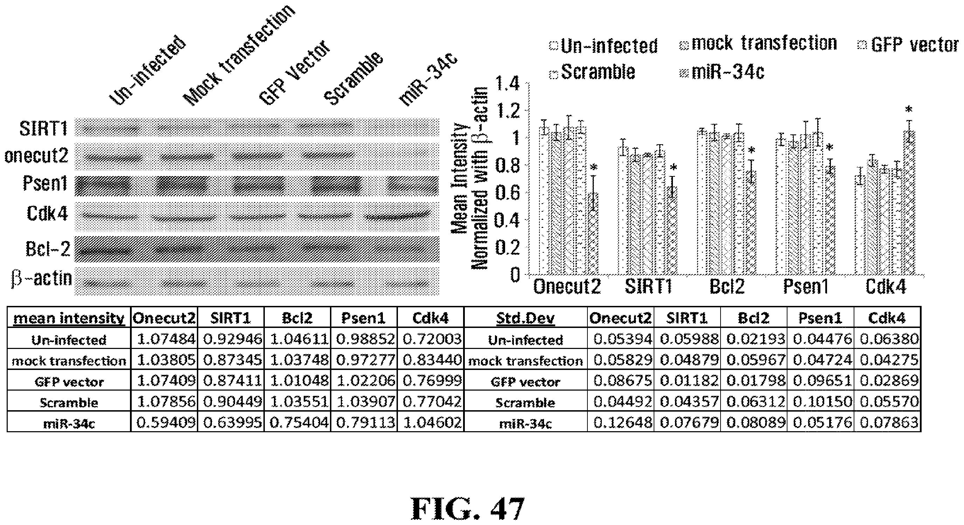

- Figure 47: A non-limiting aspect Western blot results of HEK293 cells transfected with GFP constructs expressing miR-34c, scrambled sequence or no sequence, or mock transfected. On the left panel is shown the Western blot gel image and on the right panel is shown the histogram that illustrates the quantification of the band intensities normalized with β-action.

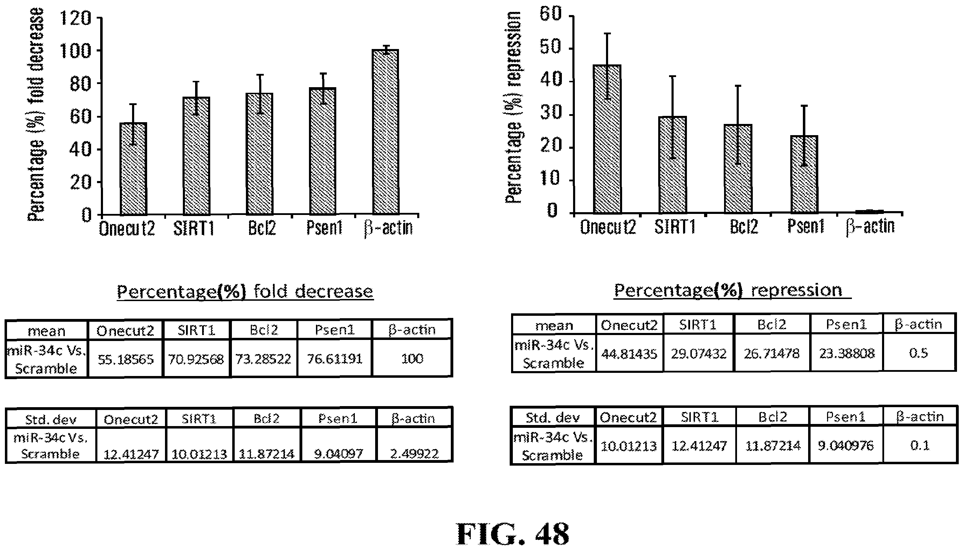

- Figure 48: A non-limiting aspect Western blot results of HEK293 cells transfected with GFP constructs expressing miR-34c or scrambled control. On the left panel, is shown the ratios of Onecut2, Sirt1, Bcl2 and Psen1 expression compared to β-actin. On the right panel, is shown the repression levels for the four proteins

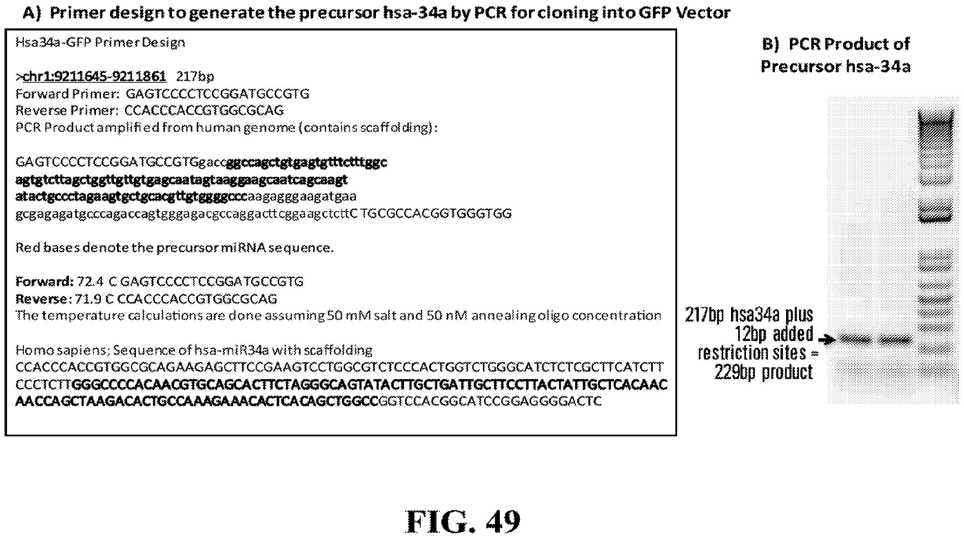

- Figure 49: Left, primers used to generate the precursor has-miR-34a by PCR on genomic DNA of HEK293 cells. Right, agarose gel image of PCR amplification results for cloning of has-miR-34c using the primers from left. The PCR amplified band is indicated by the arrow.

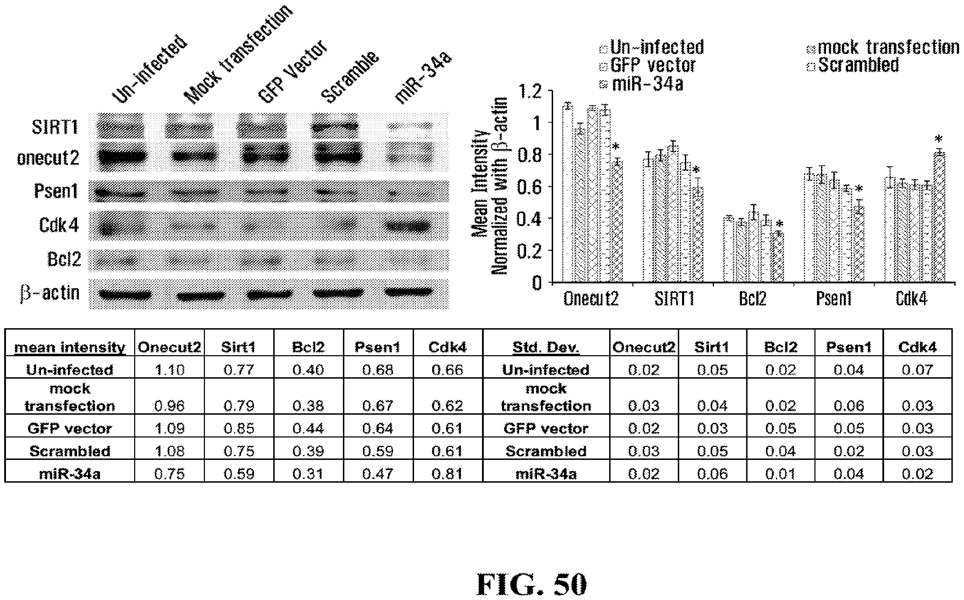

- Figure 50: A non-limiting aspect Western blot results of HEK293 cells transfected with GFP constructs expressing miR-34a or scrambled control. Top panel: left, western blot image; right, histogram obtained from the protein band level of left with normalized values. β-Actin was used as an internal control. (*: p < 0.05; all histograms represent average ± SD; n = 3, three samples from each of three experiments). Bottom panel: numerical value of band level intensities.

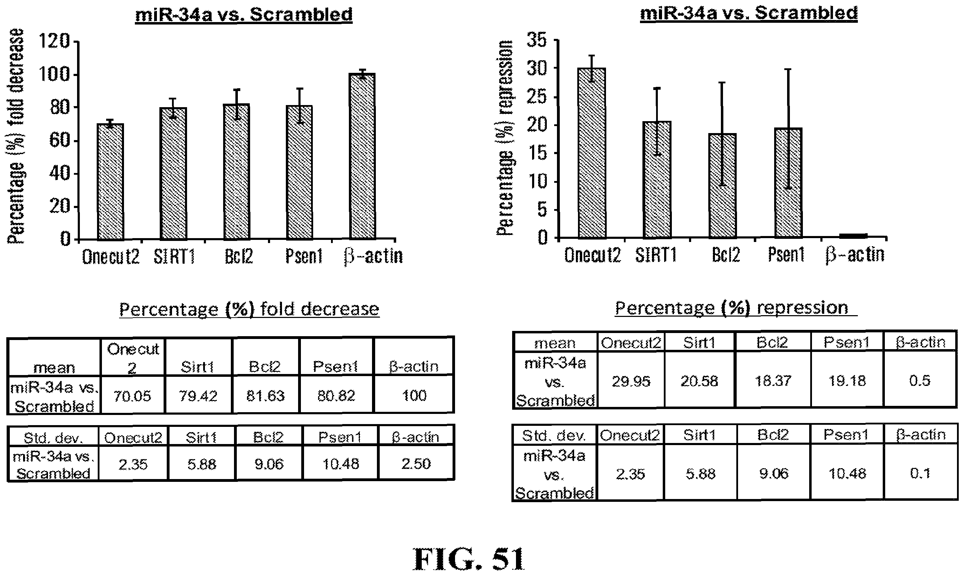

- Figure 51: Histograms illustrating the statistical analysis of the results obtained in Figure 50. Top panel, left: percentage of fold decrease for each putative protein target; right: percentage of repression. Bottom panel, numerical values from the histograms.

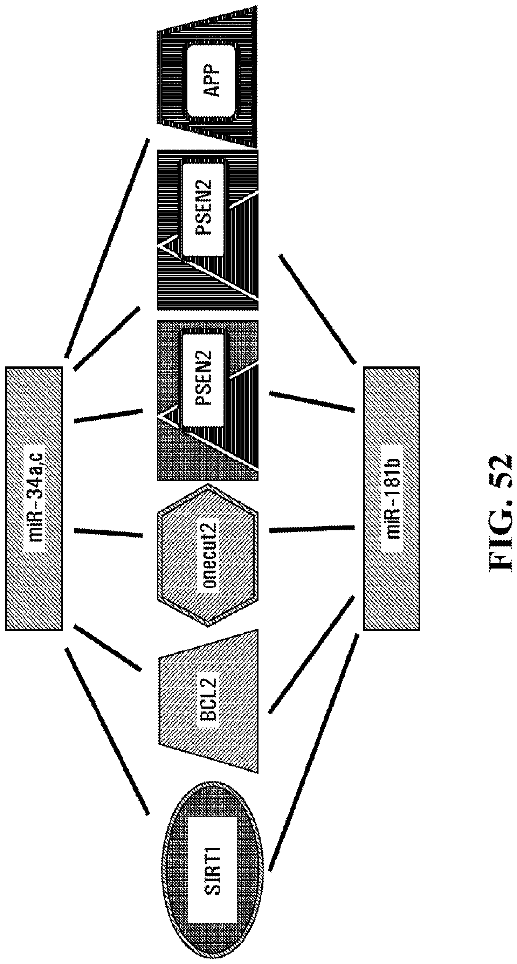

- Figure 52: A non-limiting aspect that illustrates putative target proteins of miR-34a,c and miR-181b.

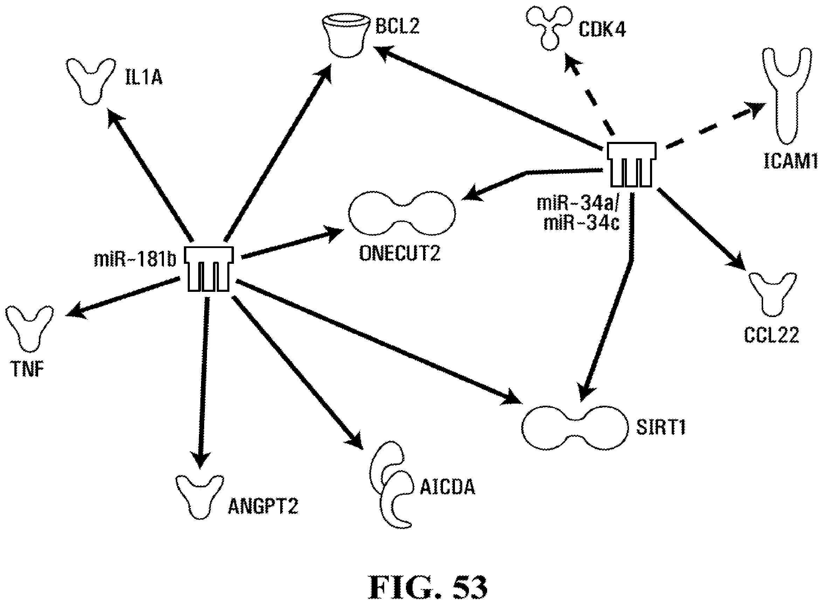

- Figure 53: A non-limiting aspect that illustrates putative shared target pathways of miR-34a,c and miR-181b.

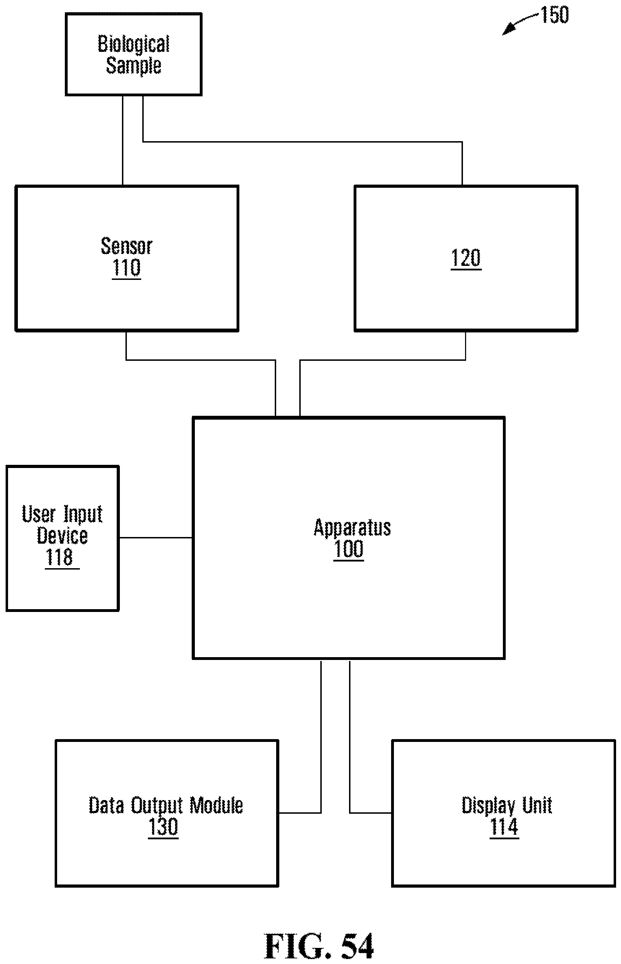

- Figure 54: High-level functional block diagram of a system including an apparatus implementing a user interface for displaying information that is indicative in a subject of the AD and/or MCI status, or the likelihood of AD and/or MCI, or the increased likelihood of AD and/or MCI, and the like, in accordance with a specific example of implementation.

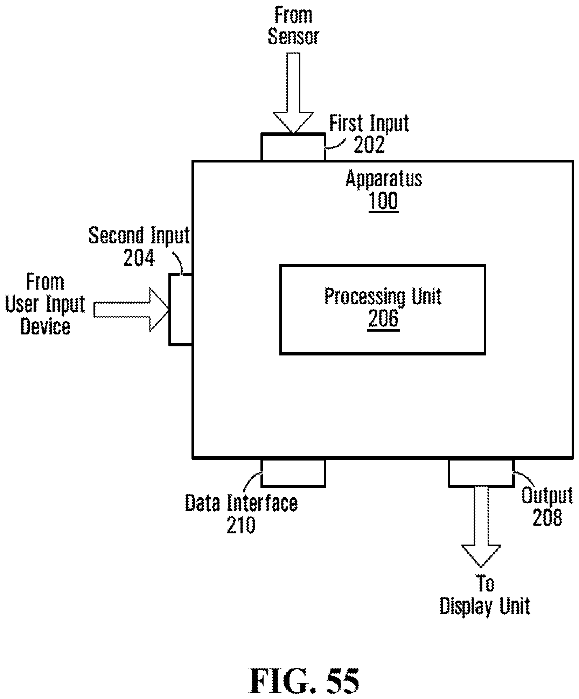

- Figure 55: Functional block diagram of an apparatus for providing information that is indicative in a subject of the AD and/or MCI status, or the likelihood of AD and/or MCI, or the increased likelihood of AD and/or MCI and the like in accordance with a specific example of implementation.

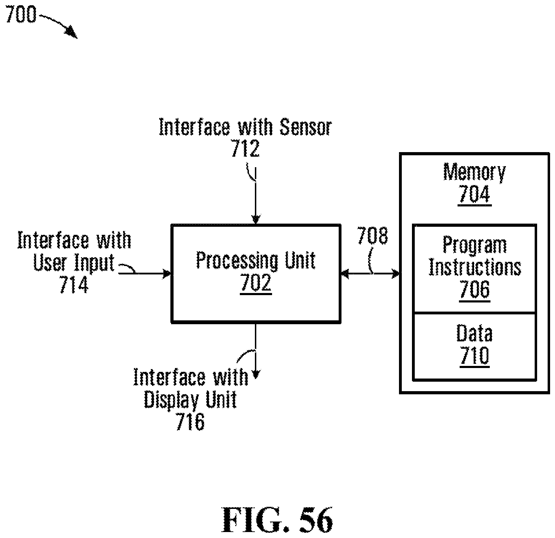

- Figure 56: Functional block diagram of an apparatus for providing information that is indicative in a subject of the AD and/or MCI status, or the likelihood of AD and/or MCI, or the increased likelihood of AD and/or MCI and the like in accordance with a specific non-limiting example of implementation.

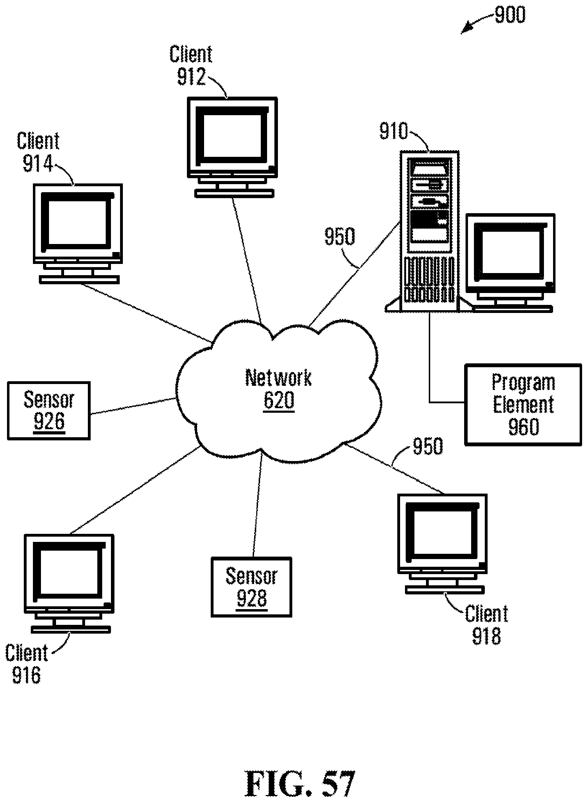

- Figure 57: Functional block diagram of a client-server system for providing information that is indicative in a subject of the AD and/or MCI status, or the likelihood of AD and/or MCI, or the increased likelihood of AD and/or MCI, and the like, in accordance with an alternative specific example of implementation.

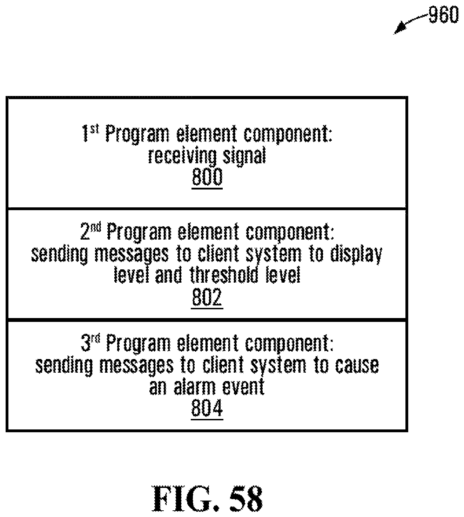

- Figure 58: High level conceptual block diagram of a program element for implementing a graphical user interface in accordance with a specific example of implementation.

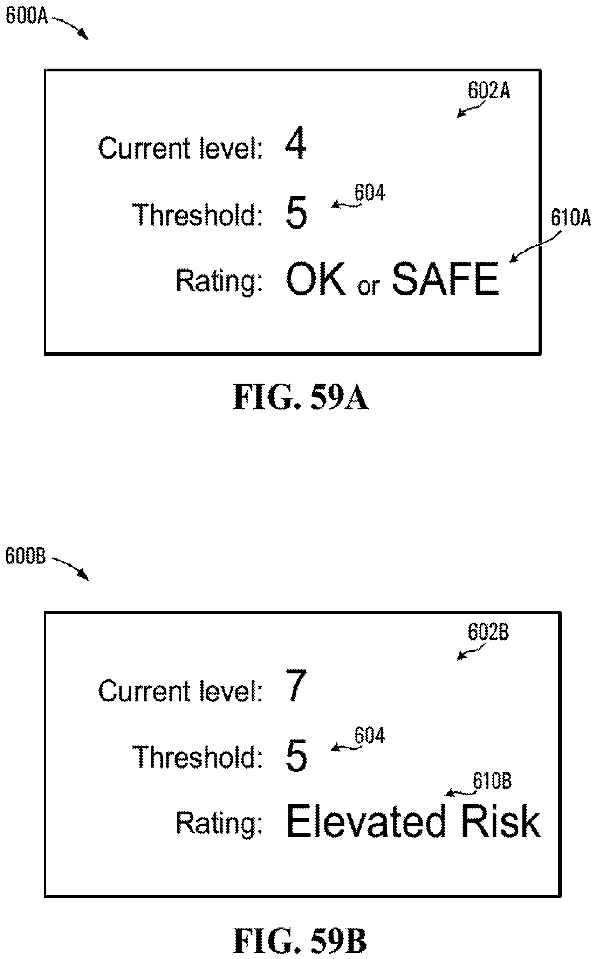

- Figure 59: (A) and (B) Specific example of implementation of a graphical user interface implemented by the system shown in Figure 54 for displaying information that is indicative in a subject of the AD and/or MCI status, or the likelihood of AD and/or MCI, or the increased likelihood of AD and/or MCI, and the like, in accordance with a specific example of implementation.

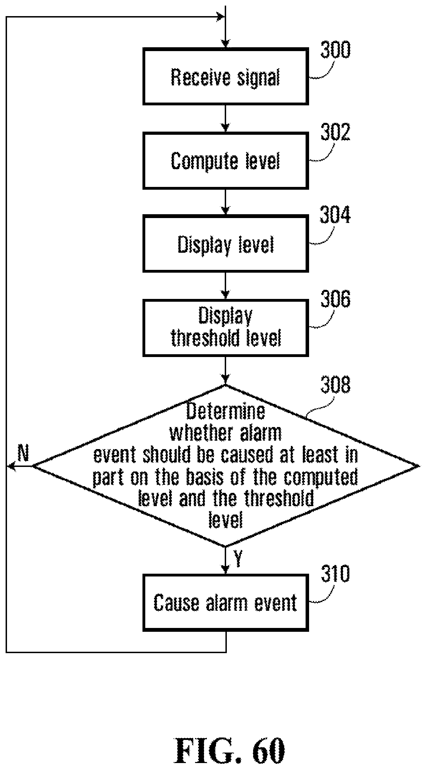

- Figure 60: Flow diagram of a process for displaying information that is indicative in a subject of the AD and/or MCI status, or the likelihood of AD and/or MCI, or the increased likelihood of AD and/or MCI, and the like, in accordance with a specific example of implementation.

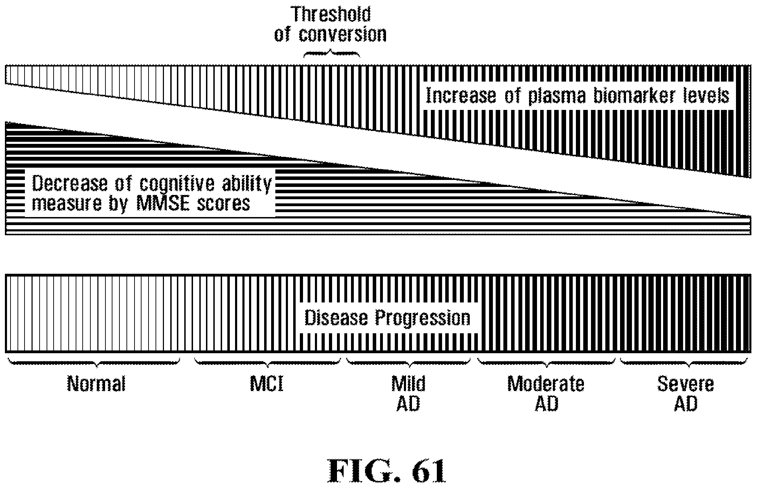

- Figure 61: A specific example of implementation of a graphical user interface implemented by the system shown in Figure 54 for displaying AD and/or MCI status, or the likelihood of AD and/or MCI, or the increased likelihood of AD and/or MCI, and the like, e.g., increasing plasma microRNA level and corresponding decreasing cognitive ability for MCI and AD, in accordance with a specific example of implementation.

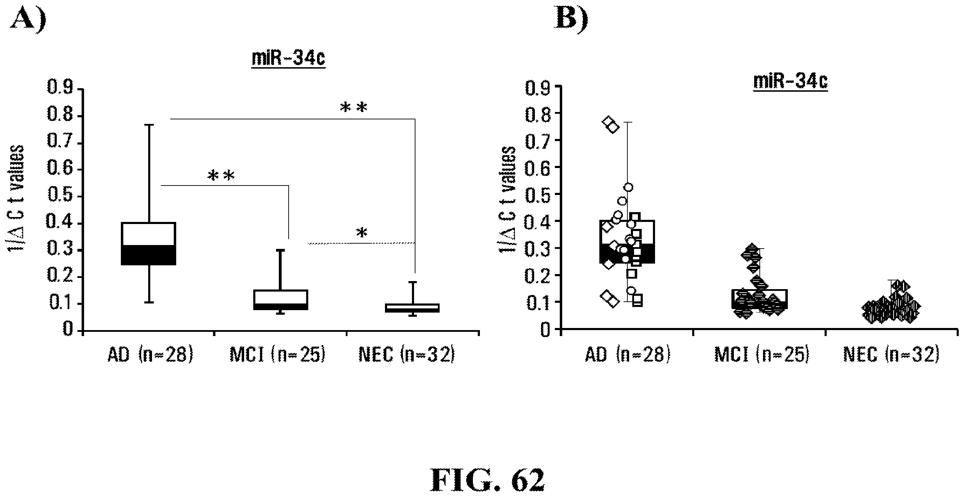

- Figure 62: A non-limiting illustrative quantitative PCR results for miR-34c level in plasma of 25 mild cognitive impaired (MCI) individuals, compared with 28 Alzheimer's disease patients (AD) and 32 normal elderly controls (NEC). The levels in plasma are significantly different, comparing all three groups by p-values. Panel A: Box plot with significance tests by p-values; Panel B: distribution of individuals' miR-34c levels in each of the 3 clinical populations. * represents p < 0.05 and ** represents p < 0.01. The p-values for box plot are calculated using 1/Δ C_{T} values.

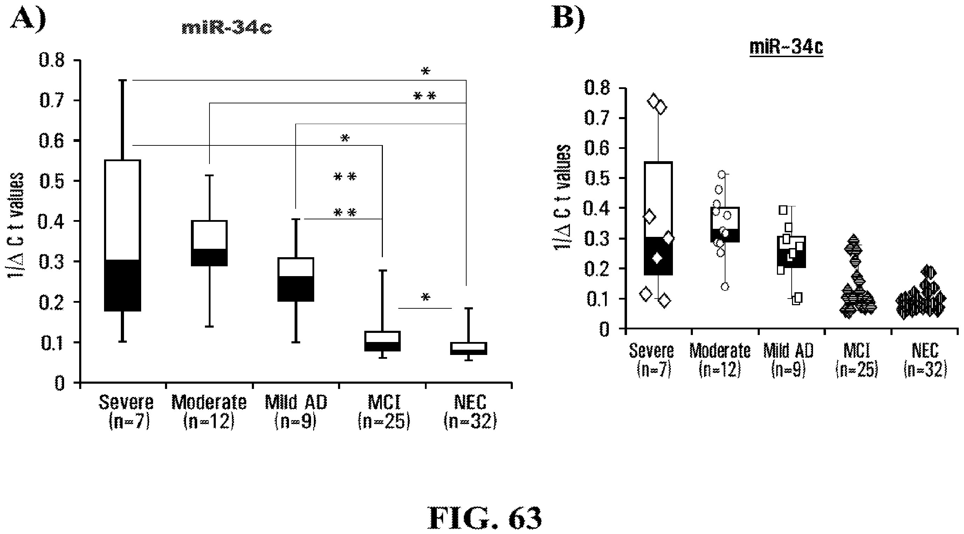

- Figure 63: A non-limiting illustrative quantitative PCR results for miR-34c level in plasma for MCI intermediate between mild AD and normal elderly controls (NEC). Panel A: Box plot with significance tests by *p-*values, showing that miR-34c levels can be used to distinguish between all five groups, MCI, NEC, mild, moderate and severe AD; Panel B: distribution of individuals' miR-34c levels in each of the 5 clinical cohorts. * represents p < 0.05 and ** represents p < 0.01. Severe AD (defined by MMSE scores in the range 4-9); Moderate AD (MMSE 10-20); Mild AD (MMSE 21-24); MCI; NEC (MMSE scores in the range 25-30).

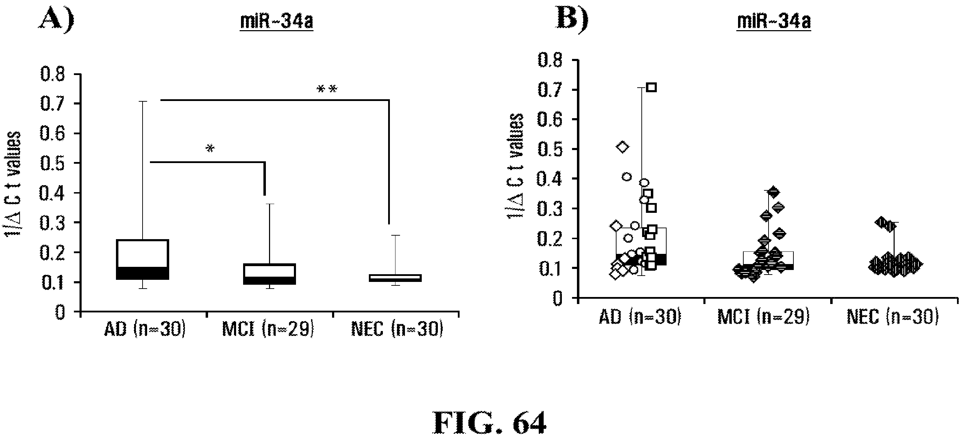

- Figure 64: A non-limiting illustrative quantitative PCR results for miR-34a level in plasma of 29 mildly cognitive impaired (MCI) individuals compared with 30 Alzheimer's disease patients (AD) and 30 normal elderly controls (NEC). The plasma levels are only significantly different comparing NEC or MCI with AD groups by p-values. There is no difference between MCI and NEC groups, suggesting that this microRNA level in plasma cannot be used to differentiate these two groups, but can be used to differentiate MCI from AD. Panel A: Box plot with significance tests by p-values; Panel B: distribution of individuals' miR-34a levels in each of the 3 clinical cohorts. * represents p < 0.05 and ** represents p < 0.01. The *p-*values for box plot are calculated using 1/Δ C_{T} values.

### DETAILED DESCRIPTION

MCI (also known as incipient dementia, or isolated memory impairment) is a brain-function syndrome involving the onset and evolution of cognitive impairments beyond those expected based on the age and education of the individual, but which are not significant enough to interfere with their daily activities. (See, e.g., Petersen et al. (1999), Arch. Neurol. 56 (3): 303-8.) It is often found to be a transitional stage between normal aging and dementia. Although MCI can present with a variety of symptoms, when memory loss is the predominant symptom it is termed "amnestic MCI" (now also called "late MCI") and is frequently seen as a prodromal stage of Alzheimer's disease. (See, e.g.,Grundman et al. (2004) Arch. Neurol. 61 (1): 59-66.)

AD can be diagnosed with reasonable accuracy at the dementia stage. In fact, a recent evidence based medicine review of the literature by the American Academy of Neurology documented that clinicians were quite accurate when the clinical diagnoses were subsequently compared to neuropathological findings (Knopman, DeKosky et al. 2001). However, as one identifies the disease process at an earlier point in the clinical continuum, the precision of the diagnosis is reduced. An important challenge is to try to identify the process at the pre-dementia stage and enhance the specificity of the clinical diagnosis through the use of imaging and other biomarkers. This approach assumes an underlying cascade of pathological events that lend themselves to intervention (Jack, Knopman et al. 2010; Petersen 2010). Biochemical and neuroimaging biomarkers can provide a window on the underlying neurobiology, facilitating early identification and intervention.

Accumulation of Aβ42 occurs early in the disease. This can be detected by molecular imaging techniques such as PET scanning using an amyloid-specific ligand or through measurement of cerebrospinal fluid (CSF) Aβ42. The next event involves neuronal injury and dysfunction which may be detected by FDG-PET measures of regional metabolic activity, elevated levels of CSF tau indicating neuronal damage, phospho-tau indicating accumulating tangle pathology, and MRI volumetric changes. When a threshold of neuronal dysfunction is reached, cognitive and then functional manifestations of AD accelerate. However, by this point in the continuum, it is likely that considerable damage has occurred in the central nervous system, and some of this may be irreversible. Consequently, most investigators believe that early intervention is preferable to waiting to treat individuals when the full dementia syndrome is present.

Amyloid biomarker abnormalities are present during the asymptomatic stage. Ideally, one would like to intervene at this point to have the greatest impact on subsequent neuronal damage. However, there is a tremendous challenge to identifying subjects at risk with sufficient sensitivity and specificity in the asymptomatic stage to allow intervention at this point. The currently available data suggests that MCI is an identifiable entity with a predictable progression to clinical dementia. (See, e.g., Gauthier S, Reisberg B, et al. Lancet 2006;367(9518):1262-1270.)

The present inventor has discovered that microRNAs (microRNAs) and their target proteins, which are expressed differentially in the brain of AD patients relative to non-AD brain, also circulate outside the brain, and therefore can be useful biomarkers for AD and/or MCI.

It was quite unexpected to detect such biomarkers outside the brain and to find that their levels outside the brain correlate with AD and/or MCI. At present, but without being limited by a particular theory, there are at least two possible general explanations for the presence of circulating microRNAs.

One non-limiting hypothesis is that passive release may occur during tissue injury. For example, microRNA-208 was shown to be exclusively expressed in the heart and was measured in the serum after heart tissue injury [Ji et al., Clin Chem. 2009 Nov;55(11): 1944-9]. The same nonspecific release could also happen in cancer, since the high rate of proliferation and cell lysis in tumors might contribute to the abundance of microRNAs in the blood stream. In contrast to these situations, to the inventor's knowledge, there has been no suggestion that AD involves tissue injury or tumors, so AD-associated microRNAs should not be released non-specifically.

A second non-limiting hypothesis is that microRNAs may be contained in small particles which may allow transfer of mRNA and microRNA between cells through microvesicles [Valadi et al., Nat Cell Biol. 2007 Jun;9(6):654-9]. These are small (50 nm to 100 nm) particles, shed from the cell plasma membrane into the extracellular space and into the blood stream [Caby et al., Int Immunol. 2005 Ju1;17(7):879-87]. Microvesicles are derived from different cell types, e.g. reticulocytes, dendritic cells, B/T cells and mast cells [Brase et al. Mol. Cancer. 2010; 9:306]. However, to the inventor's knowledge, there has been no suggestion that neurons in AD patients would shed microvesicles.

Furthermore, in addition to the above, it is generally believed that the central nervous system (CNS) is an enclosed system, [see, e.g. Krutzfeldt, et al., Nature, 438(7068): 685-9, 1 December 2005] and that the blood-brain barrier (BBB) restricts the movement of CNS pathoetiological factors from the rest of the body.

The advent of a biological marker differentiating AD/MCI from normal aging and other dementing illnesses may be useful in at least evaluating, monitoring, screening for candidate therapeutic compounds for treating and/or managing and/or preventing this debilitating disorder. A substantially accurate, minimally-invasive biological marker of AD/MCI as described herein may serve the public interest, for instance, by facilitating patient and family counseling, optimizing stratification of sub-groups for enrolment in clinical drug trials, interpreting treatment outcome measures, and the like. The herein described biomarkers may also prove useful in situations where concurrent medical conditions preclude or confound cognitive and neuropsychological testing. Examples of the latter include patients with major depression, delirium, suppressed consciousness, or otherwise uncooperative for detailed cognitive testing [Schipper (2007) Alzheimer's and Dementia 3: 325-332].

In one aspect, the herein described expression "determining" a "level of circulating" biomarker refers to an assessment performed on a biological sample. In one aspect, the herein described biological sample is selected from, but without being limited thereto, blood and fractions thereof, blood serum, blood plasma, urine, excreta, semen, seminal fluid, seminal plasma, prostatic fluid, pre-ejaculatory fluid (Cowper's fluid), pleural effusion, tears, saliva, sputum, sweat, biopsy, ascites, amniotic fluid, lymph, vaginal secretions, endometrial secretions, gastrointestinal secretions, bronchial secretions, breast secretions, and the like.

In another aspect, the herein described biological sample is selected from blood serum and blood plasma.

In one aspect, the herein described sample can be obtained by any known technique, for example by drawing, by non-invasive techniques, or from sample collections or banks, etc.

In one aspect, the herein described methods and/or kits may employ, for example, a dipstick, a membrane, a chip, a disk, a test strip, a filter, a microsphere, a slide, a multi-well plate, an optical fiber, and the like, or any other variant available to the person skilled in the art.

In one aspect, the person skilled in the art may use either (i) "fresh" biological samples, such as blood plasma or serum, or (ii) frozen (stored) and subsequently thawed biological sample, such as plasma or serum.

In another aspect, frozen (stored) biological samples, such as plasma or serum, are maintained at storage conditions of -20 to -70 degrees centigrade until thawed and used. In another aspect, "fresh" biological samples, such as plasma or serum, are maintained at room temperature, refrigerated or maintained on ice until used.

In the specific example of a serum/plasma sample, blood can be drawn by standard methods into a collection recipient, such as a tube. In one aspect, the collection recipient is made of siliconized glass. In one aspect, the blood is drawn either without anticoagulant for preparation of serum, or with EDTA, sodium citrate, heparin, or similar anticoagulants for preparation of plasma.

In one aspect, the plasma or serum is optionally first fractionated from whole blood prior to being frozen. This reduces the burden of extraneous intracellular RNA released from lysis of frozen and thawed cells, which might reduce the sensitivity of the amplification assay or interfere with the amplification assay through release of inhibitors to PCR such as porphyrins and hematin. "Fresh" plasma or serum may be fractionated from whole blood by centrifugation, using for instance gentle centrifugation at about 300-800 x g for about five to about ten minutes, or fractionated by other standard methods.

In one non-limiting aspect, there is provided a method as herein described which additionally or alternatively includes determining a level of a target protein, wherein the target protein is encoded by an mRNA which is a target of the circulating microRNA associated with AD and/or MCI. The target protein level may be a circulating protein level or an intracellular target protein level. In one aspect, the herein described target protein is selected from an apoptosis marker (e.g., Bcl2, etc.), synaptosis pathway marker (e.g., PS1, Onecut2, etc.), homeo-box pathway marker (e.g., Onecut 2, etc.), and any combinations thereof.

In one aspect, the herein described target protein is selected from Onecut2, Prenisilin1, SIRT1, Cdk4, Bcl2, and any combinations thereof. For example, in one aspect, the herein described target protein is Onecut2, alternatively or additionally, the herein described target protein is Prenisilin1, alternatively or additionally, the herein described target protein is SIRT1, alternatively or additionally, the herein described target protein is Cdk4, alternatively or additionally, the herein described target protein is Bcl2. The person skilled in the art may select a suitable target protein.

In one aspect, the herein described microRNA is selected from a microRNA that targets at least a messenger of apoptosis (e.g., Bcl2, etc.), synaptosis pathway (e.g., PS1, Onecut2, etc.), homeo-box pathway (Onecut 2, etc.), and any combinations thereof. The person skilled in the art may select a suitable microRNA.

In one aspect, the herein described microRNA is selected from a microRNA the level of which is associated in the brain with AD and/or MCI, for instance, but without being limited thereto: miR-9, miR-15a, miR-15b, miR-17-5p, miR20a, miR-20b, miR-29a/b/c, miR-30c, miR-34a/b/c, let-7i, let-7f, miR-101, miR-103, miR-106a/b, miR-107, miR-124, miR-146a, miR-148b, miR-181a/b/c/d, miR-195, miR-200a, miR-200b, miR298, miR-328, miR-371, miR-495, miR-517*, miR-520h, miR-579 and miR-709. (See, e.g. Yao et al., PLoS One. 2010 Dec 16;5(12):e15546; Smith et al., J Neurochem. 2011 Jan;116(2):240-7; Li et al., Neurosci Lett. 2011 Jan 3;487(1):94-8; Wang et al., Brain Res. 2010 Oct 21;1357:166-74; Hébert et al., Hum Mol Genet. 2010 Oct 15;19(20):3959-69; Schonrock et al., PLoS One. 2010 Jun 11;5(6):e11070; and Wang et al., Brain Res Bull. 2009 Oct 28;80(4-5):268-73.). The sequence information of these and other microRNA can be obtained from the Sanger database located at: http://www.mirbase.org/index.shtml. The person skilled in the art may select a suitable circulating microRNA.

In one aspect, the herein described microRNA is selected from miR-34a/b/c, let-7f, miR-181a/b/c/d, miR-517*, miR-579, miR-200b and any combinations thereof.

In one aspect, the herein described microRNA is miR-34c, or miR-181b, or miR-34a.

In one aspect, the herein described kit may include at least one detecting agent which is "packaged". As used herein, the term "packaged" can refer to the use of a solid matrix or material such as glass, plastic, paper, fiber, foil and the like, capable of holding within fixed limits the at least one detection reagent. Thus, in one aspect, the kit may include the at least one detecting agent "packaged" in a glass vial used to contain microgram or milligram quantities of the at least one detecting agent. In another aspect, the kit may include the at least one detecting agent "packaged" in a microtiter plate well to which microgram quantities of the at least one detecting agent has been operatively affixed. In another aspect, the kit may include the at least one detecting agent coated on microparticles entrapped within a porous membrane or embedded in a test strip or dipstick, etc. In another aspect, the kit may include the at least one detecting agent directly coated onto a membrane, test strip or dipstick, etc. which contacts the sample fluid. Many other possibilities exist and will be readily recognized by those skilled in this art.

In one aspect, the level of microRNA is detected by hybridization. In one aspect, the hybridization is accomplished using a support, for example a solid support, having probes bound to a surface, e.g., to capture the microRNA by hybridization to the probe. Non-limiting examples of types of supports for immobilization of the probe include controlled pore glass, glass plates, polystyrene, avidin-coated polystyrene beads, cellulose, nylon, acrylamide gel and activated dextran.

Where a support is used, the probe may be attached to the support in a variety of manners. For example, the probe may be attached to the support through a 3' or 5' terminal nucleotide of the probe. In some aspects, the probe is attached to the support by a linker that serves to distance the probe from the support surface. The linker can be at least 15-30 atoms in length. The required length of the linker will depend on the particular solid support used. For example, a six atom linker is generally sufficient when high cross-linked polystyrene is used as the solid support.

A wide variety of linkers are known in the art which may be used to attach the probe to the support. The linker may be formed of any compound which does not significantly interfere with the hybridization of the target sequence to the probe attached to the support. The linker may be formed of a homopolymeric oligonucleotide which can be readily added on to the linker by automated synthesis. Alternatively, polymers such as functionalized polyethylene glycol can be used as the linker. Such polymers are preferred over homopolymeric oligonucleotides because they do not significantly interfere with the hybridization of probe to the target oligonucleotide. Polyethylene glycol is particularly preferred. The linkages between the support, the linker and the probe are normally not cleaved during removal of base protecting groups under basic conditions at high temperature. Examples of preferred linkages include carbamate and amide linkages.

### 1. Detection / quantification of biomarkers

Generally, microRNAs can be detected, and their level can be determined, using any of the techniques available to the person skilled in the art. For example, these techniques include, but are not limited thereto, different variants of the Northern blot methodology (see, e.g., Valoczi et al. 2004, Nucleic Acids Res. 2004 Dec 14;32(22):e175; Ramkissoon et al. 2006, Mol Cell Probes. 2006 Feb;20(1):1-4), real-time PCR (see, e.g., Schmittgen et al. 2004 Nucleic Acids Res. 2004 Feb 25;32(4):e43; Chen et al. Nucleic Acids Res. 2005 Nov 27;33(20):e179; Raymond et al. 2005 RNA. Nov;11(11):1737-44), confocal laser-induced fluorescence detection (Neely et al. Nat Methods. 2006 Jan;3(1):41-6), oligo-array-based technologies (see, e.g., Babak et al. RNA. 2004 Nov;10(11):1813-9; Nelson et al. Nat Methods. 2004 Nov;1(2):155-61; or Thomson et al. Nat Methods. 2004 Oct;1(1):47-53), and the like. The person skilled in the art will be able to select and use a suitable technique.

In the specific case of real-time PCR, this technique can generally be done according to published protocols and variants thereof, which can be designed by the person skilled in the art. For one or more specific microRNA sequences in a sample, Real Time-PCR enables both detection and quantification. The quantity can be either an absolute number of copies or a relative amount when normalized to a control RNA input or additional normalizing RNAs.

The procedure usually follows the following general principles. In the illustrative example where one starts from a biological sample, one proceeds with total RNA extraction from the biological sample using, e.g., TRI Reagent™ (Molecular Research Centre, Inc.) or TRIzol™ reagent (Invitrogen) following the manufacturer's recommendations. RNA quality may be examined for instance on an Agilent 2100 Bioanalyzer with the RNA 6000 Nano Kits (Agilent, Germany) or by visual inspection of the 28S/18S ribosomal bands in an agarose gel. RNA quantity can be measured,for example, on a Nanodrop 1000 Spectrophotometer (Thermo Scientific, USA). Total RNA or a fraction thereof is then used for cDNA synthesis using reverse transcriptase enzyme and suitable reagents, e.g. buffers, dNTP, primers, etc., as described in several publications known to the person skilled in the art (see, e.g., Schmittgen et al. 2004 Nucleic Acids Res. 2004 Feb 25;32(4):e43; Chen et al. Nucleic Acids Res. 2005 Nov 27;33(20):e179; Raymond et al. 2005 RNA. Nov;11(11):1737-44).

Since plasma samples do not carry cell-associated 28 and 18S RNAs, the isolated RNA fraction is in general of small RNA fraction. The RNA quality is evaluated by RNA integrity number (RIN) on the Bioanalyzer 2100 (Agilent, Germany), and the RIN quality is based on the sharp peak quality in range of less than 150 nucleotide (nt).

Once with the extracted total RNA or fraction thereof in hand, one can proceed with the real-time PCR procedure following the general principles of polymerase chain reaction; its key feature is that the amplified DNA is detected as the reaction progresses in real time. This is a new approach compared to standard PCR, where the product of the reaction is detected at its end. Two common methods for detection of products in real-time PCR are: (1) non-specific fluorescent dyes that intercalate with any double-stranded DNA, and (2) sequence-specific DNA probes consisting of oligonucleotides that are labelled with a fluorescent reporter which permits detection only after hybridization of the probe with its complementary DNA target.

Relative concentrations of DNA produced by the PCR reaction present during the exponential phase of the reaction are determined by plotting fluorescence against cycle number on a logarithmic scale (so that an exponentially-increasing quantity will show as a straight line). A threshold for detection of fluorescence above background is determined. The cycle at which the fluorescence from a sample crosses the threshold is called the cycle threshold, Cₜ. The quantity of DNA theoretically doubles every cycle, during the exponential phase, and relative amounts of DNA can be calculated, e.g. a sample whose Cₜ is 3 cycles earlier than another's has 2³ = 8 times more template. Since all sets of primers don't work equally well, one has to calculate the reaction efficiency first. Thus, by using this as the numeric base and the cycle difference Cₜ as the exponent, the precise difference in starting template can be calculated (in previous example, if efficiency was 98%, then the sample would have 2(^{3 * 98%}) = (2^{98%})³ = 7.67 times more template).

The microRNA level is then generally determined by comparing the results to a standard curve produced by real-time PCR of serial dilutions (e.g. undiluted, 1:4, 1:16, 1:64) of a known amount of RNA or DNA.

In one aspect, to accurately quantify the level of microRNA of interest, the measured level of microRNA is divided by the amount of RNA from a control housekeeping gene measured in the same sample or to a spiked in control RNA to normalize for possible variation in the amount and quality of RNA between different samples. This normalization permits accurate comparison of expression of the gene of interest between different samples, provided that the control RNA used in the normalization is very similar across all the samples.

Real-time PCR can be used to quantify nucleic acids by two methods: relative quantification and absolute quantification. Relative quantification is based on internal reference genes to determine fold-differences in the level of the target microRNA. Absolute quantification gives the exact number of target microRNA molecules by comparison with standards [see, e.g., Dhanasekaran et al. (2010 Mar). Immunol Methods. 354 (1-2): 34-9].

Generally proteins can be detected and their expression level may be assayed using antibodies with commonly used methods known in the art, such as but without being limited thereto, Western blotting or enzyme-linked immunosorbent assay (ELISA). Western blotting typically begins with an electrophoresis step, where proteins from a biological sample of interest are separated on the basis of size and electromagnetic charge by sodium dodecyl sulfate-polyacrylamide gel electrophoresis (SDS-PAGE), according to standard methods known in the art. See, e.g., SAMBROOK ET AL., 3 MOLECULAR CLONING: A LABORATORY MANUAL A8.40-A8.45 (2001) (describing various reagents and methods for electrophoresis of proteins by SDS-PAGE). The contents of the gel are then transferred to nitrocellulose, nylon, PVDF, or other membrane or filter suitable for fixation and Western blotting by standard methods also known in the art. The transfer may be by immersion, semi-dry blotting, or by other comparable methods known in the art. Next, the filters or membranes are fixed to prevent loss of the target proteins during the several hybridization, washing, and staining steps typically included in Western blotting. Fixation may be accomplished by heat, cross-linking with ultraviolet light, or by other comparable methods known in the art. See, e.g., SAMBROOK ET AL., 3 MOLECULAR CLONING: A LABORATORY MANUAL A8.52-A8.55 (describing various reagents and methods for immunoblotting and detection of antigen/antibody complexes).

Non-specific antibody binding sites on the fixed filter or membrane are generally blocked with buffered solutions (e.g., phosphate-buffered saline ("PBS") or the like) containing a blocking agent such as, for example, 0.5% (w/v) low-fat dry milk or 5% (w/v) bovine serum albumin (BSA). After blocking, the filter or membrane then undergoes primary antibody incubation. After primary antibody incubation, the filter or membrane is washed, and the presence of antibody-antigen complexes detected using a secondary antibody labeled with chromogenic, fluorogenic, or chemiluminescent means. Antibody-antigen complexes are then detected colorimetrically (e.g., with horseradish peroxidase and TMB), or by autoradiography (e.g., alkaline phosphatase). If detected colorimetrically, or by chemiluminescence, the amount of color of fluorescence may be measured using a luminometer, a spectrophotometer, or other similar instruments. If detected autoradiographically, the amount of bound antibody may be measured from the exposed x-ray film using a densitometer, or similar instrument. See, e.g., SAMBROOK ET AL., 3 MOLECULAR CLONING: A LABORATORY MANUAL A8.52-A8.55.

Secondary antibodies used in Western blotting, whether polyclonal or monoclonal, may be labeled with a ligand (such as biotin) or a detectable marker (such as a fluorescent group or an enzyme) using conventional techniques. Suitable labels include fluorophores, chromophores, electron-dense reagents (e.g., silver or gold), enzymes, and ligands having specific binding partners. Enzymes such as horseradish peroxidase or alkaline phosphatase are typically detected by their activity. For example, horseradish peroxidase can be detected by its ability to convert tetramethylbenzidine (TMB) to a blue pigment, quantifiable with a spectrophotometer. Other suitable ligands and/or detectable markers include biotin and avidin or streptavidin, IgG and protein A, and the numerous additional receptor-ligand couples known in the art. Other permutations and possibilities will be readily apparent to those of ordinary skill in the art.

Ponceau red staining can be used as loading control for plasma samples. In one aspect, one may use, if desired, an additional processing step of using affinity columns to substantially remove plasma serum albumin from plasma samples, for example plasma serum albumin levels can be reduced to < 10% of starting value using the Vivapure™ anti-human albumin serum (HAS)/IgG kit (Sartorius Stedim Biotech GMbH, Gottingen, Germany). Alternatively, serum albumin could be removed by the Thermo Scientific Pierce Blue Albumin Removal kit composed of an agarose resin bed which can bind to the serum albumin for processing gel/western blotting assays. Similar affinity depletion cartridges for removal of human serum albumin are also available from Applied Biosystems. Negative controls such as minus primary antibody and normal serum may also be employed if desired. Intensities on Western blots can be quantified by a computer image scanner system, allowing the determination of levels of protein bands for each blot, calculated as a ratio to the major band intensity (plasma protein samples).

In the specific case of an ELISA, this technique typically begins with an antigen adsorption step, where the target antigen or antigens are adsorbed to the wells of a microtiter plate. See, e.g., KIERKEGAARD & PERRY LABORATORIES, INC., TECHNICAL GUIDE FOR ELISA 9-13 (2003). The most commonly used adsorption buffers for antibodies are 50 mM Carbonate, pH=9.6; 10 mM Tris-HCl, pH=8.5; and 10 mM PBS, pH=7.2. These buffers work well for many proteins. If the target antigens are not readily adsorbed to the surface of the microtiter plate, plates with surfaces modified or derivatized to permit covalent linkage of proteins to their surface by a variety of chemical means are widely available from commercial suppliers. Time and temperature are factors affecting the amount of protein adsorbed.

Once the wells of a microtiter plate are coated with the desired antigen or antigens, they are typically washed with a blocking buffer to block non-specific antibody binding and to minimize false positive results. See, e.g., id. at 13-14 (discussing methods and reagents for blocking microtiter plates). Commonly used blocking agents are either protein solutions, such as BSA (typically used at concentrations between 1% and 5% (w/v) in PBS, pH=7.0), non-fat dry milk, casein (the main protein component of non-fat dry milk), or caseinate (a more soluble version of casein, produced by partial digestion with sodium hydroxide), normal serum (typically used at concentrations between 1% and 5% (v/v)), and gelatin (normally used at concentrations between 1% and 5% (w/v)), or non-ionic detergents, such as TWEEN-20™ and TRITON X-100™.

Washing reagents are selected for their ability to disrupt low-affinity interactions between various reaction components that can affect the ability to detect specific antigen-antibody interactions. See, e.g., id. at 14-15 (discussing methods and reagents for washing microtiter plates). Wash solutions commonly contain a physiological buffer to prevent denaturation of antigens and their cognate antibodies, and to preserve enzyme activity. Buffers such as PBS, Tris-saline, or imidizole-buffered saline at neutral pH are widely used. Specific buffers are typically selected based on the method of detection to be employed in a particular assay. Wash buffers should also include non-ionic detergents such as TWEEN 20, TRITON X-100, or the like, at concentrations of between 0.01% to 0.05% (v/v), in order to disrupt low-affinity, non-specific interactions between reaction components.

After the blocking step, the wells of the microtiter plate are typically washed. The adsorbed antigen then undergoes the primary antibody incubation, after which it is typically washed again. Antibody/antigen complexes are then detected using a secondary antibody labeled with chromogenic (e.g., horseradish peroxidase and TMB), fluorescent or chemiluminescent (e.g., alkaline phosphatase) means. See, e.g., id. at 15-21 (discussing antibody preparation and use, as well as commonly used detection molecules). The amount of color or fluorescence may be measured using a luminometer, a spectrophotometer, or other similar instruments. There are many common variations on the standard ELISA protocol, including competitive ELISA, sandwich ELISA, and numerous others. One of ordinary skill in the art will select the appropriate protocol to use, depending on the antigen to be detected, the source of antigen and/or primary antibody used in the assay, and any other relevant experimental parameters.

The herein described kit may be prepared by techniques known to one skilled in the art. By way of example, the probes (or detection agents of microRNAs) may be labeled, using labeling techniques that are known to one skilled in the art, to facilitate detection, including but not limited to radioisotope labels or fluorescent labels. The probes can hybridize to nucleic acid molecules that are either or both strands of the double stranded nucleic acid molecule portion of the microRNA.

Nucleic acid hybridization is affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringency conditions depend on the length and base composition of the nucleic acid, which can be determined by techniques well known in the art. Generally, stringency can be altered or controlled by, for example, manipulating temperature and salt concentration during hybridization and washing. For example, a combination of high temperature and low salt concentration increases stringency. Such conditions are known to those skilled in the art and can be found in, for example, Strauss, W. M. "Hybridization With Radioactive Probes," in Current Protocols in Molecular Biology 6.3.1-6.3.6, (John Wiley & Sons, N.Y. 2000). Both aqueous and nonaqueous conditions as described in the art can be used.

An example of stringent hybridization conditions is hybridization in 0.1 x SSC (15 mM sodium chloride/1.5 mM sodium citrate) at 50 °C. or higher. Another example of stringent hybridization conditions is hybridization overnight at 42 °C. in 50% formamide, 1 x SSC (150 mM NaCl, 15 mM sodium citrate), 50 mM sodium phosphate (pH 7.6), 5 x Denhardt's solution, 10% (w/v) dextran sulfate, and 20 µg/ml denatured, sheared salmon sperm DNA, followed by washing in 0.1 x SSC at about 65 °C. Highly stringent conditions can include, for example, aqueous hybridization (e.g., free of formamide) in 6 x SSC (where 20 x SSC contains 3.0 M NaCl and 0.3 M sodium citrate), 1% (w/v) sodium dodecyl sulfate (SDS) at 65 °C. for about 8 hours (or more), followed by one or more washes in 0.2 x SSC, 0.1 % SDS at 65 °C.

Moderately stringent hybridization conditions permit a nucleic acid to bind a complementary nucleic acid that has at least about 60%, at least about 75%, at least about 85%, or greater than about 90% identity to the complementary nucleic acid. Stringency of hybridization is generally reduced by decreasing hybridization and washing temperatures, adding formamide to the hybridization buffer, or increasing salt concentration of the washing buffer, either individually or in combination. Moderately stringent conditions can include, for example, aqueous hybridization (e.g., free of formamide) in 6 x SSC, 1% (w/v) SDS at 65 °C. for about 8 hours (or more), followed by one or more washes in 2 x SSC, 0.1% SDS at room temperature. Another exemplary hybridization under moderate stringency comprises hybridization in 6 x SSC, 5 x Denhardt's reagent, 0.5% (w/v) SDS, and optionally 100 µg/ml sonicated salmon or herring sperm DNA, at about 42 °C., followed by washing in 2 x SSC, 0.1 % (w/v) SDS at 65 °C. Other permutations and possibilities will be readily apparent to those of ordinary skill in the art.

These and many other permutations will be readily apparent to those of ordinary skill in the art.

### 2. Definitions

As used herein, the expressions "circulating microRNA" and "circulating protein" generally refer to a microRNA or protein found outside the brain, for example in a biological sample, such as plasma.

As used herein, the expression "biological sample" generally refers to a sample obtained from a biological subject, including samples of biological tissue or fluid origin, obtained, reached, or collected in vivo or in situ.

As used herein, a "substantially cell free" biological sample is a biological sample which has been processed, for instance but without being limited thereto, by centrifugation, sedimentation, cell sorting, and the like, in order to substantially remove cells, such that when one aims to detect the level of circulating microRNA, this detected level reflects circulating levels and minimizes detection of microRNA molecules which would be released from cell lysis. In the particular case of fluids such as plasma and serum, these are generally presumed to be cell-free; however in the practical sense, particularly under conditions of routine clinical fractionation, plasma and serum may occasionally be contaminated by cells. Nonetheless, plasma and serum are considered for the purposes of this invention as "substantially cell free" biological samples.

As used herein, microRNAs (microRNAs) are small (e.g., 18-25 nucleotides in length), noncoding RNAs that influence gene regulatory networks by post-transcriptional regulation of specific messenger RNA (mRNA) targets via specific base-pairing interactions. This ability of microRNAs to inhibit the production of their target proteins results in the regulation of many types of cellular activities, such as cell-fate determination, apoptosis, differentiation, and oncogenesis.

As used herein, a microRNA and/or target protein thereof that is "differentially expressed" or "differentially present" is when the level thereof is "increased" or "decreased" relative to a reference level. In one aspect, the difference in level can be determined qualitatively, such as the visualization of the presence or absence of a signal. I another aspect, the difference in level can be determined quantitatively. In one aspect, the level may be compared to a diagnostic cut-off value, beyond which a skilled person is capable of determining the statistical significance of this level. In another aspect, the microRNA and/or target protein thereof is differentially present if, for example, the mean or median level of the microRNA and/or target protein thereof in a sample is calculated to be statistically significant from a reference level. Common tests for statistical significance include, among others, t-test, ANOVA, Kruskal-Wallis, Wilcoxon, Mann-Whitney and odds ratio, which are known to the person skilled in the art. In another aspect, the herein described "differentially present" represents a differential level of the biomarker of, e.g., at least 1.1, 1.2, 1.3, 1.4, 1.5, 1.6, 1.7, 1.8, 1.9, 2.0, 2.3, or more fold between the tested sample and a reference level. In one aspect, the comparison of the herein described biomarkers level relative to a diagnostic cut-off level allows the person skilled in the art to determine the AD status of a subject, or distinguish between early MCI and late MCI, or distinguish between MCI and mild AD, and the like.

In one aspect, the comparison of the herein described biomarker level relative to a reference level allows the person skilled in the art to select a candidate therapeutic compound at least partly based on the effect of the tested compound on the biomarker level. For example, the level of a microRNA and/or target protein thereof in a sample can be "increased" when a host is contacted with the tested compound, for example, by an increase of about 5%, 10%, 20%, 30%, 40%, 50%, 60%, 70%, 90%, 100%, 200%, 300%, 500%, 1,000%, 5,000% or more relative to a reference level (e.g., in absence of the tested compound, or relative to a NEC, etc.)). Alternatively, the level of a microRNA and/or target protein thereof in a sample can be "decreased" when the host is contacted with the tested compound, for example, by a decrease of about 99%, 95%, 90%, 80%, 70%, 60%, 50%, 40%, 30%, 20%, 10%, 1% or less relative to a reference level (e.g., in absence of the tested compound, or relative to a NEC, etc.) As used herein, a "low" level of a biomarker (a microRNA or target protein thereof) in a sample can be a level that is less than the level of the biomarker in a pool from a non-patient population. A "low" level of a biomarker in a sample can also refer to a level that is decreased in comparison to the level of the biomarker reached upon treatment, for example with an anti-AD compound. A "low" level of a biomarker can also refer to a level that is present in comparison to an individual that does not have AD (e.g., a NEC). In certain cases, the low level of a biomarker in a sample can be an indication of MCI or AD, or of AD status, or of progression of AD, and the like.

As used herein, a "high" level of a biomarker (microRNA or target protein thereof) in a sample can be a level that is elevated in comparison to the level of a biomarker in a pool from a non-patient population. A "high" level of a biomarker in a sample can also refer to a level that is elevated in comparison to the level of the biomarker reached upon treatment, for example with an anti-AD compound. A "high" level of a biomarker can also refer to a level that is present in comparison to an individual that does not have AD (e.g., a NEC). In certain cases, a high level of the biomarker in a sample can be an indication of MCI or AD, or of AD status, or of progression of AD, and the like.

As used herein, the terms "individual," "subject," and "patient," generally refer to a human subject, unless indicated otherwise, e.g., in the context of a non-human mammal useful in an in vivo model (e.g., for testing drug toxicity), which generally refers to murines, simians, canines, felines, ungulates and the like (e.g., mice, rats, other rodents, rabbits, dogs, cats, swine, cattle, sheep, horses, primates, etc.).

As used herein, the term "array" and the equivalent term "microarray" generally refers to an ordered array of capture agents for binding to aqueous analytes, such as microRNA, or to an ordered array for enclosing cell culture populations comprising a plurality of cell cultures. An "array" includes any two-dimensional or substantially two-dimensional (as well as a three-dimensional) arrangement of spatially addressable regions (i.e., "features"). In the specific case of an array containing capture agents, such addressable regions include the capture agents, such as polynucleotides, and the like. Any given support may carry one, two, four or more arrays disposed on a surface of a support. A typical array may contain one or more, including more than two, more than ten, more than one hundred, or more than one thousand features, in an area of less than about 100 cm², less than 20 cm², less than 10 cm², less than 5 cm², less than 1 cm², less than 1 mm², less than 100 µm², or even smaller. For example, features may have widths in the range from 10 µm to 1.0 cm. In other aspects each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. Inter-feature regions can be present which do not carry any nucleic acids. Such inter-feature areas are typically present where the arrays are formed by processes involving drop deposition of reagents but may not be present when, for example, photolithographic array fabrication processes are used.

The terms "determining," "measuring," "evaluating," "assessing," and "assaying," as used herein, generally refer to any form of measurement, and include determining if an element is present or not in a biological sample. These terms include both quantitative and/or qualitative determinations, which require sample processing and transformation steps of the biological sample. Assessing may be relative or absolute. The phrase "assessing the presence of" can include determining the amount of something present, as well as determining whether it is present or absent.

The term "stringent assay conditions" generally refers to conditions that are compatible to produce binding pairs of nucleic acids, e.g., probes and target microRNAs, of sufficient complementarity to provide for the desired level of specificity in the assay while being generally incompatible to the formation of binding pairs between binding members of insufficient complementarity to provide for the desired specificity. The term "stringent assay conditions" generally refers to the combination of hybridization and wash conditions.

A "label" or a "detectable moiety" in reference to a nucleic acid, generally refers to a composition that, when linked with a nucleic acid, renders the nucleic acid detectable, for example, by spectroscopic, photochemical, biochemical, immunochemical, or chemical means. Exemplary labels include but are not limited to radioactive isotopes, magnetic beads, metallic beads, colloidal particles, fluorescent dyes, enzymes, biotin, digoxigenin, haptens, and the like. A "labeled nucleic acid or oligonucleotide probe" is generally one that is bound, either covalently, through a linker or a chemical bond, or noncovalently, through ionic bonds, van der Waals forces, electrostatic attractions, hydrophobic interactions, or hydrogen bonds, to a label such that the presence of the nucleic acid or probe can be detected by detecting the presence of the label bound to the nucleic acid or probe.

In one aspect, the herein described detection agent comprises a nucleic acid primer (or probe) having a sequence of 6-50, or 10-30, or 15-30, or 20-30 contiguous nucleotides of the microRNA and/or target mRNA thereof, including any length between the stated ranges. Such primer may be present, if desired, on a microarray.

Primers (or probes) are usually single-stranded for maximum efficiency in amplification / hybridization, but may alternatively be double-stranded. If double-stranded, the primers (or probes) are usually first treated to separate the strands before use; this denaturation step is typically done by heat, but may alternatively be carried out using alkali, followed by neutralization.

By way of a non-limiting example, the primers (or probes) for detecting a circulating microRNA may be labeled, using labeling techniques that are known to one skilled in the art, to facilitate detection, including but not limited to radioisotope labels or fluorescent labels. The primers (or probes) can hybridize to nucleic acid molecules that are either or both strands of the double stranded nucleic acid molecule portion of the microRNA.

A "label" or a "detectable moiety" in reference to a detecting agent, in particular in the case of primers (or probes), generally refers to a compound that, when linked with at least one detecting agent, renders it detectable, for example, by spectroscopic, photochemical, biochemical, immunochemical, or chemical means. An example of a "label" or a "detectable moiety" includes but is not limited to radioactive isotopes, magnetic beads, metallic beads, colloidal particles, fluorescent dyes, enzymes, biotin, digoxigenin, haptens, and the like. In this context, "labeled" primers (or probe) includes primers (or probe) that are bound, either covalently, through a linker or a chemical bond, or noncovalently, through ionic bonds, van der Waals forces, electrostatic attractions, hydrophobic interactions, or hydrogen bonds, to a label such that the presence of the primers (or probe) can be detected by detecting the presence of the label bound to the primers (or probe).

A detectable label may be included in an amplification reaction. Suitable labels include fluorochromes, e.g., fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may be a two stage system, where the amplified DNA is conjugated to biotin, haptens, etc. having a high affinity binding partner, e.g., avidin, specific antibodies, etc., where the binding partner is conjugated to a detectable label. The label may be conjugated to one or both of the primers. Alternatively, the pool of nucleotides used in the amplification may be labeled, so as to incorporate the label into the amplification product. All of these and other labels are well known in the art and one can select corresponding suitable means for detecting such labels.

Hybridization primers (or probes) may be coupled to labels for detection. As with amplification primers, several methods and compositions for derivitizing oligonucleotides with reactive functionalities that permit the addition of a label are known in the art. For example, several approaches are available for biotinylating probes so that radioactive, fluorescent, chemiluminescent, enzymatic, or electron dense labels can be attached via avidin. See, e.g., Broken et al., Nucl. Acids Res. (1978) 5:363-384 which discloses the use of ferritin-avidin-biotin labels; and Chollet et al. Nucl. Acids Res. (1985) 13:1529-1541 which discloses biotinylation of the 5' termini of oligonucleotides via an aminoalkylphosphoramide linker arm. Several methods are also available for synthesizing amino-derivatized oligonucleotides which are readily labeled by fluorescent or other types of compounds derivatized by amino-reactive groups, such as isothiocyanate, N-hydroxysuccinimide, or the like, see, e.g., Connolly (1987) Nucl. Acids Res. 15:3131-3139, Gibson et al. (1987) Nucl. Acids Res. 15:6455-6467 and U.S. Pat. No. 4,605,735 to Miyoshi et al. Methods are also available for synthesizing sulfhydryl-derivatized oligonucleotides which can be reacted with thiol-specific labels, see, e.g., U.S. Pat. No. 4,757,141, Connolly et al. (1985) Nuc. Acids Res. 13:4485-4502 and Spoat et al. (1987) Nucl. Acids Res. 15:4837-4848. A comprehensive review of methodologies for labeling DNA fragments is provided in Matthews et al., Anal. Biochem. (1988) 169:1-25.

For example, probes may be fluorescently labeled by linking a fluorescent molecule to the non-ligating terminus of the probe. Guidance for selecting appropriate fluorescent labels can be found in Smith et al., Meth. Enzymol. (1987) 155:260-301; Karger et al., Nucl. Acids Res. (1991) 19:4955-4962; Haugland (1989) Handbook of Fluorescent Probes and Research Chemicals (Molecular Probes, Inc., Eugene, Oreg.). In one aspect, fluorescent labels include fluorescein and derivatives thereof, such as disclosed in U.S. Pat. No. 4,318,846 and Lee et al., Cytometry (1989) 10:151-164, and 6-FAM, JOE, TAMRA, ROX, HEX-1, HEX-2, ZOE, TET-1 or NAN-2, and the like.

Additionally, probes can be labeled with an acridinium ester (AE). Current technologies allow the AE label to be placed at any location within the probe. See, e.g., Nelson et al. (1995) "Detection of Acridinium Esters by Chemiluminescence" in Nonisotopic Probing, Blotting and Sequencing, Kricka L. J. (ed) Academic Press, San Diego, Calif.; Nelson et al. (1994) "Application of the Hybridization Protection Assay (HPA) to PCR" in The Polymerase Chain Reaction, Mullis et al. (eds.) Birkhauser, Boston, Mass.; Weeks et al., Clin. Chem. (1983) 29:1474-1479; Berry et al., Clin. Chem. (1988) 34:2087-2090. An AE molecule can be directly attached to the probe using non-nucleotide-based linker arm chemistry that allows placement of the label at any location within the probe. See, e.g., U.S. Pat. Nos. 5,585,481 and 5,185,439.

Hybridization (e.g., formation of a nucleic acid duplex) refers to the ability of a strand of nucleic acid to join with a complementary strand via base pairing. Hybridization occurs when complementary nucleic acid sequences in the two nucleic acid strands contact one another under appropriate conditions.

Nucleic acid hybridization is affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringency conditions depend on the length and base composition of the nucleic acid, which can be determined by techniques well known in the art. Generally, stringency can be altered or controlled by, for example, manipulating temperature and salt concentration during hybridization and washing. For example, a combination of high temperature and low salt concentration increases stringency. Such conditions are known to those skilled in the art and can be found in, for example, Strauss, W. M. "Hybridization With Radioactive Probes," in Current Protocols in Molecular Biology 6.3.1-6.3.6, (John Wiley & Sons, N.Y. 2000). Both aqueous and nonaqueous conditions as described in the art can be used.

An example of stringent hybridization conditions is hybridization in 0.1 x SSC (15 mM sodium chloride/1.5 mM sodium citrate) at 50 °C. or higher. Another example of stringent hybridization conditions is hybridization overnight at 42 °C. in 50% formamide, 1 x SSC (150 mM NaCl, 15 mM sodium citrate), 50 mM sodium phosphate (pH 7.6), 5 x Denhardt's solution, 10% (w/v) dextran sulfate, and 20 µg/ml denatured, sheared salmon sperm DNA, followed by washing in 0.1 x SSC at about 65 °C. Highly stringent conditions can include, for example, aqueous hybridization (e.g., free of formamide) in 6 x SSC (where 20 x SSC contains 3.0 M NaCl and 0.3 M sodium citrate), 1% (w/v) sodium dodecyl sulfate (SDS) at 65 °C. for about 8 hours (or more), followed by one or more washes in 0.2 x SSC, 0.1 % SDS at 65 °C.

Moderately stringent hybridization conditions permit a nucleic acid to bind a complementary nucleic acid that has at least about 60%, at least about 75%, at least about 85%, or greater than about 90% identity to the complementary nucleic acid. Stringency of hybridization is generally reduced by decreasing hybridization and washing temperatures, adding formamide to the hybridization buffer, or increasing salt concentration of the washing buffer, either individually or in combination. Moderately stringent conditions can include, for example, aqueous hybridization (e.g., free of formamide) in 6 x SSC, 1% (w/v) SDS at 65 °C. for about 8 hours (or more), followed by one or more washes in 2 x SSC, 0.1% SDS at room temperature. Another exemplary hybridization under moderate stringency comprises hybridization in 6 x SSC, 5 x Denhardt's reagent, 0.5% (w/v) SDS, and optionally 100 µg/ml sonicated salmon or herring sperm DNA, at about 42 °C., followed by washing in 2 x SSC, 0.1 % (w/v) SDS at 65 °C. Other permutations and possibilities will be readily apparent to those of ordinary skill in the art.

As used herein, the terms "complementary" or "complementarity" are used in reference to "polynucleotides" and "oligonucleotides" (which are interchangeable terms that refer to a sequence of nucleotides) related by the base-pairing rules. For example, the sequence "5'-CAGT-3'," is complementary to the sequence "5'-ACTG-3'." Complementarity can be "partial" or "total." "Partial" complementarity is where one or more nucleic acid bases are not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. This is of particular importance in amplification reactions, as well as detection methods which depend upon binding between nucleic acids.

In one aspect, substantially complementary nucleic acids have at least 50%, at least 60%, at least 70%, at least 80%, at least 90%, at least 95%, at least 98%, or at least 99% identical nucleotides.

The term "percentage identity" is intended to denote a percentage of nucleotides which are identical between the two sequences to be compared, obtained after the best alignment, this percentage being purely statistical and the differences between the two sequences being distributed randomly and over their entire length. Sequence comparisons between two nucleotide sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl. Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

As used herein, a nucleic acid sequence "substantially identical" to a target sequence, e.g., a target sequence contained within the target microRNA, is a nucleic acid sequence which is identical to the target sequence, or which differs from the target sequence by one or more nucleotides.

As used herein, the term "about" for example with respect to a value relating to a particular parameter (e.g. concentration, such as "about 100 mM") relates to the variation, deviation or error (e.g. determined via statistical analysis) associated with a device or method used to measure the parameter. For example, in the case where the value of a parameter is based on a device or method which is capable of measuring the parameter with an error of ±10%, "about" would encompass the range from less than 10% of the value to more than 10% of the value.

As used herein, the term "MMSE" relates to the mini-mental state examination (MMSE) or Folstein test which is known to the person skilled in the art. This test is generally a brief 30-point questionnaire test that is used to screen for cognitive impairment. It is commonly used in medicine to screen for dementia. It is also used to estimate the severity of cognitive impairment at a given point in time, and to follow the course of cognitive changes in an individual over time, thus making it an effective way to document an individual's response to treatment.

In the time span of about 10 minutes it samples various functions including arithmetic, memory and orientation. It was introduced by Folstein et al. in 1975, [Journal of Psychiatric Research 12 (3): 189-98]. The standard MMSE form which is currently published by Psychological Assessment Resources is based on its original 1975 conceptualization, with minor subsequent modifications by the authors.

It must be noted that, as used herein and in the appended claims, the singular forms "a," "an," and "the" include plural referents and plural referents include singular forms unless the context clearly dictates otherwise. Thus, for example, reference to "a subject polypeptide" includes a plurality of such polypeptides, reference to "the agent" includes reference to one or more agents and equivalents thereof known to those skilled in the art, reference to "nucleic acid molecules" includes reference to one or more nucleic acid molecules, and reference to "antibodies" includes reference to one or more antibodies and so forth.

With respect to ranges of values, the invention encompasses the upper and lower limits and each intervening value between the upper and lower limits of the range to at least a tenth of the upper and lower limit's unit, unless the context clearly indicates otherwise. Further, the invention encompasses any other stated intervening values.

### EXAMPLES

### MATERIALS AND METHODS

### I - Experimental individuals

AD and NEC populations are recruited and processed at a university hospital subcontract site. The Clinic is a member of the National Institute on Aging (NIA) Alzheimer's Disease Neuroimaging Initiative (ADNI) consortium. Figures 1 and 2 generally illustrate the recruitment and processing of AD and NEC individuals.

### II - AD and MCI Patient Assessment

The AD subjects all have AD proven by standard clinical criteria [McKhann et al., (1984) Neurology 34(7): 939-944]. The patients/research subjects are all well characterized, with geriatric and neurological exams, blood work, CT scans, and MRIs. The clinical assessments (of significant impairment in 2 or more cognitive domains, as well as in function) are supported by documentation of abnormal performance on memory and other cognitive domains (with age and education norms) of at least 1.5 standard deviations below the mean for the subject's age, on the neuropsychological battery described below. All subjects are being rated according to the Clinical Dementia Rating Scale [Hughes et al., (1982) British Journal of Psychiatry 140: 566-572]; physical function is being assessed using the OARS (Older Americans Resource Survey), as well as ADL and IADL scales [Fillenbaum, G. G. (1988) Multidimensional functional assessment of older adults: The Duke Older Americans Resources and Services Procedures. Hillsdale, NJ: Lawrence Erlbaum Associates, Inc.]. Clinical evaluation allows exclusion of individuals with other neurological and systemic illnesses liable to produce dementia or cognitive impairment. Significant depression is being excluded via the Yesavage Geriatric Depression Scale; we are excluding those (relatively few) individuals who demonstrate major depression (Geriatric Depression scale > 15) [Yesavage, J. A. (1988) Psychopharmacol. Bull. 24(4):709-711; Yesavage et al., (1983) In Assessment in geriatric psychopharmacology, T. Crook, S. Ferris, and R. Bartus, Eds. New Canaan, Ct: Mark Powley & Assoc., 153-165].

MCI victims are characterized as losing episodic memory but retaining activities of daily living (ADL) functional capacity, and many eventually convert to Alzheimer's disease type dementia, thus termed 'MCI due to AD' (Albert, M.S., et al., Alzheimers Dement. 7(3): 270-279), with further division MCI into early and late stages, EMCI and LMCI, for staging progression leading to AD conversion. MCI include subjective complaints of memory loss by the individual or family, gradual onset of cognitive decline, and objective abnormalities on a clinician's mental status assessment. All MCI subjects will meet Clinical Dementia Rating of about 0.5, with no functional impairment or general cognitive loss sufficient to qualify as dementia. Abnormal memory function documented by scoring within the education-adjusted ranges on the Logical memory II subscale (Delayed Paragraph Recall, Paragraph A only) from the Wechsler Memory Scale - Revised (the maximum score is 25): 8 or less for 16 or more years of education, and 4 or less for 8-15 years of education. Early MCI means that on the same test, the scores are: 9-11 for 16 or more years of education, and 5-9 for 8-15 years of education (Chertkow, H. Current Opinion in Neurology 15: 401-407).

### III - Normal Elderly Controls

In order to define a heterogeneous etiology not associated with late-onset autosomal dominant forms of AD, first were selected aged-matched septuagenarians with no first-order AD family members, to avoid the possibility of late-onset autosomal dominant forms of the disease. A second criterion was selecting individuals in good health, based on the least amount of medication taken, and by excluding subjects with a chronic disease, diabetes or cancer. Subjects with other confounding factors, such as tobacco or alcohol consumption, depression, head injury, or low level of education were excluded.

These community-based selected normal subjects were screened with the Montréal Cognitive Assessment (MoCA) [Nasreddine et al. (2005) J. Am. Geriatr. Soc. 53(4): 695-699.], on which they must score 26 or higher out of 30. Likewise, any subjects who complain of significant memory loss (score of 6 or more on the Subjective Memory Impairment scale) were excluded. Those who volunteer had a neuropsychological assessment similar to the AD subjects. Any individuals with scores below 1.5 standard deviations of the mean for his/her age on any of the tests were excluded.

The AD research subjects and normal controls were all followed annually for the duration of the analysis in the Memory Clinic at the University Hospital, with annual MMSE, Clinical Dementia Rating scale [Hughes et al., (1982) British Journal of Psychiatry 140: 566-572; Galluzzi et al, (2008) Dement. Geriatr. Cogn. Disord. 26: 314-322.], an extensive mental status assessment, and Yesavage Geriatric Depression scale. A plurality of clinical neuropsychological tests were performed. Subjects received an initial assessment, and annual reassessments containing the following elements: Modified WAIS subtests (digit symbol) [Wechsler, D. (1981) Wechsler Adult Intelligence Scale - Revised. New York: The Psychological Corporation.], Wechsler Memory Scale-R subtests (Logical Memory, Digit Span) [Wechsler, D. (1987) Wechsler Memory Scale - Revised. San Antonio: The Psychological Corporation.], Boston Naming Test [Kaplan et al., (1983) The Boston Naming Test. Philadelphia: Lea & Febiger], Raven's Progressive Matrix [Raven (1947) Colored Progressive Matrices Sets A, Ab, B. London: H.K. Lewis], tests of semantic memory [Chertkow, H. and Bub, D. (1990) Brain 113(Pt 2): 397-417.], Verbal Fluency, Benton Temporal Orientation Test [Benton et al., (1983) Contributions to Neuropsychological Assessment. A Clinical Manual, New York: Oxford University Press.], visual perceptual tests, RAVLT [Rey, A. (1941) L'Examen psychologique dans les cas d'encephalopathie traumatique. Archives de Psychologie 28: 286-340; Bondareff et al., (1987) Archives of General Psychiatry 44(5): 412-417.], and the Folstein Mini-Mental State test [Folstein et al., (1975) J. of Psychiatric Research 12(3): 189-198.]. Clock drawings were scored according to Freedman [Freedman et al., (1994) New York: Oxford University Press.], and an 18 point scale developed in Dr. Chertkow's laboratory [Chertkow et al., (1998) J. of Canadian Congress of Neurological Sciences, Suppl. 1: S27]. The Montréal Cognitive Assessment (Mo-CA) is a one-page paper and pencil test that requires 12 minutes to administer. Two highly trained neuropsychologists in the Memory Evaluation Unit carried out this evaluation, including extensive standardized tests of memory and other cognitive abilities.

Subjects did not show evidence of cerebral infarct on CT, had no evidence of significant heart disease, alcoholism, or drug use, and took no psychoactive medications.

### IV - determination of level of circulating microRNA in human

Total RNA was isolated from plasma samples according to the following protocol. Plasma is isolated from 30 ml of whole human blood. 1) Prepare 5 x 4ml of Ficoll per patient and gently lay 6 ml of EDTA blood onto the Ficoll layer without mixing, centrifuge at 1500 rpm for 30 min at 4 °C, do not use breaks. 2) Transfer the plasma to one or two 15 ml centrifuge tubes, each with ½ tablet of protease inhibitor. 3) Add equal volume of Trizol to plasma (1:1), mix by repetitive pipetting for 5 min. 4) Mix by repetitive pipetting for 5 min. After homogenization, samples can be stored at -80 °C for at least a year. Note: Incomplete homogenization will results in a lower yield. Homogenizing for too long and too continuously in a small volume (e.g, 1ml) may cause heating of the sample; this may result in degradation of the RNA in the tissue. Samples should be cooled on ice during homogenization. 5) Add 0.2 ml of chloroform per 1 ml of Trizol reagent and shake tubes vigorously by hand for 15 seconds and incubate samples at room temperature. 6) Centrifuge samples at 12000 x g for 15 minutes at 4 °C. Note: The 4 °C spin is preferable for phase separation. Room temperature centrifugation may result in variable phase separation, hence resulting in variable RNA yields. 7) Carefully remove the aqueous phase without disturbing the interphase, and transfer it to a fresh tube, save organic phase for DNA and protein isolation. Note: During the transfer of the aqueous phase, avoid pipetting the interphase which contains DNA. 8) Add an equal volume of Phenol:Chloroform:Isoamyl alcohol (25:24:1) and mix well. Note: This step completely removes protein contamination. 9) Centrifuge the mixture at 12000 x g for 5 min and transfert the aqueous phase to a fresh tube. 10) Repeat steps 8 and 9 twice in order to completely remove protein contamination. 11) Precipitate the RNA from the aqueous phase by mixing with isopropyl alcohol. Add 0.5 ml of isopropyl alcohol per 1 ml Trizol used to homogenize the samples. 12) Incubate at room temperature for 10 min and centrifuge at 12,000 x g for 10 min at 4 °C. 13) Discard the supernatant and wash the RNA pellet with 1 ml of 75% ethanol per 1 ml of Trizol reagent. Note: This washing step will remove organic residues from the RNA pellet. Mix the samples by inverting the tube or vortexing, and centrifuge at 7,500 x g for 5 min. at 4 °C. 14) Discard the ethanol, and briefly air dry the RNA pellet for 5-10 min, then dissolve it in RNase free water, incubation for 5 min at 50-55 °C is recommended to completely dissolve the RNA. Note: It is preferable not to let the RNA dry completely as it will decrease its solubility. Partially dissolved RNA samples have an A_{260/280} < 1.6

The purity (A260/280 ratio) and quantity of total RNA was estimated using the Nanodrop machine. Approximately 2µL of RNA sample was loaded on the machine.

The analysis of RNA Integrity was performed using the Agilent's 2100 Bioanalyzer (Agilent Technologies). [Also see, e.g., Fleige et al., Mol. Aspects Med.27, 126-139 (2006); Quantitation comparison of total RNA using the Agilent 2100 bioanalyzer, ribogreen analysis, and UV spectrometry. Agilent Application Note, Publication Number 5988-7650EN, 2002; Fleige et al. Biotechnol.Lett. 19, 1601-1613, 2006; Schroeder et al., BMC Mol. Biol, 2006.]

Besides the ratio of the 18S to 28S rRNAs, the Agilent 2100 bioanalyzer also takes the entire electrophoretic trace of the RNA sample, including the presence or absence of degradation products Agilent's 2100 bioanalyzer uses a lab on a chip approach to perform capillary electrophoresis and uses a fluorescent dye that binds to RNA to determine both the RNA concentration and integrity.

The Bioanalyzer uses two chips for the analysis of RNA. The RNA 6000 Nano kit for the quantitation and analysis of total and mRNA concentrations ranging from 25 ng/µl - 500 ng/µL and the 6000 Pico kit for the analysis of low concentrated RNA samples down to 50 pg/µL of total RNA or 250 pg/µL of mRNA. Although the lower quantitative limit of the 6000 Nano assay is 25 ng/µL, it is recommended to use at least 50 ng/µL. Moreover, the levels of sensitivities bellow 50 pg/µL will only be reached using extremely pure RNA.

The bioanalyzer electropherogram of total RNA will show a marker peak and two distinct ribosomal peaks corresponding to 18S and 28S eukaryotic RNA, with the 28S peak being the highest. The baseline between the marker and the 18S peak is relatively flat and free of small rounded peaks corresponding to smaller RNA molecules that are degradation products of the RNA transcripts. Both an elevated threshold baseline and a decreased 28S:18S ratio are indicative of degradation. The machine automatically assigns and integrity number to the total RNA sample analyzed. For good quality RNA samples, the RIN should be higher than 7.5. In the particular case of biological fluids that have little or no 28S nor 18S RNA, such as plasma and serum, the microRNA is usually visualized as part of the small RNA in the small peak in between 0 and 150 nucleotide (nt) basepair position. The height of the peak is the indicator of the abundance of the microRNA in this fraction.

Example of DNA, RNA, protein and plasma quantities isolated from representing AD and NEC individuals is shown in the following Table:

**TABLE 1**

| NORMAL Elderly Controls | | | | | | | | | | | | | | | |
|---|---|---|---|---|---|---|---|---|---|---|---|---|---|---|---|
| Patient No. | Diag. | WB (mL) | Plasma % (mL) | plasma WB | Total RNA (ug/uL) | Ratio 260/280 | Vol. (uL) | Yield ug | Total DNA (ug/uL) | Ratio 260/280 | Vol. (uL) | Yield ug | Total Protein (ug/uL) | Vol. (uL) | Yeld ug |
| R 454 | NEC | 28 | 15.5 | 55 | 1.06 | 1.73 | 67 | 71.0 | 0.47 | 1.62 | 500 | 235 | 1.88 | 2800 | 5264 |
| 3R 460 | NEC | 23 | 11.5 | 50 | 2.29 | 1.59 | 38 | 87.0 | 0.6 | 1.59 | 500 | 300 | 5.6 | 1600 | 8960 |
| 2R 464 | NEC | 30 | 15 | 50 | 1.03 | 1.67 | 67 | 69.0 | 0.49 | 1.66 | 500 | 245 | 1.53 | 1900 | 2907 |
| R 787 | NEC | 30 | 17 | 57 | 0.69 | 1.74 | 67 | 46.2 | 0.37 | 1.7 | 500 | 185 | 1.2 | 2400 | 2880 |
| R 887 | NEC | 29 | 13 | 45 | 1.03 | 1.57 | 67 | 69.0 | 1.11 | 1.62 | 500 | 555 | 2.46 | 2200 | 5412 |
| | | | | | | | | | | | | | | | |

| Alzheimer Disease | | | | | | | | | | | | | | | |
|---|---|---|---|---|---|---|---|---|---|---|---|---|---|---|---|
| Patient No. | Diag. | WB (mL) | Plasma (mL) | % plasma WB | Total RNA (ug/uL) | Ratio 260/280 | Vol. (uL) | Yeld ug | Total DNA (ug/uL) | Ratio 260/280 | Vol. (uL) | Yield ug | Total Protein (ug/uL) | Vol. (uL) | Yield ug |
| 2R 369 | DAT | 32 | 17 | 53 | 1.15 | 1.65 | 67 | 77.05 | 0.31 | 1.63 | 500 | 155 | 2.15 | 2200 | 4730 |
| 2R 377 | DAT | 32.5 | 16.5 | 51 | 0.98 | 1.74 | 67 | 65.66 | 0.48 | 1.62 | 500 | 240 | 2.34 | 1800 | 4212 |
| 3R 401 | DAT | 32.5 | 14.5 | 45 | 1.17 | 1.77 | 67 | 78.39 | 0.95 | 1.59 | 500 | 475 | 2.44 | 2000 | 4880 |
| 2R 411 | DAT | 24 | 14 | 58 | 0.66 | 1.57 | 67 | 44.22 | 0.13 | 1.58 | 500 | 65 | 2.55 | 1700 | 4335 |
| R 430 | DAT | 30 | 15 | 50 | 0.94 | 1.73 | 67 | 62.98 | 0.41 | 1.62 | 500 | 205 | 1.28 | 2000 | 2560 |

Alternatively, total RNA was isolated with the following protocol as described in Li et al., Mechanisms of Ageing and Dev. 132 (2011) 75-85. Frozen tissue samples were ground to fine powder in a mortar and pestle under liquid nitrogen, and dissolved in Trizol reagent (Invitrogen, Carlsbad, CA). Total RNA was isolated with the RNeasy Mini Kit (Qiagen, Valencia, CA), according to the manufacturer's protocol. Briefly, 700 ml of Trizol reagent containing samples was mixed vigorously with 140 ml of chloroform (Sigma-Aldrich, St. Louis, MO), and incubated at room temperature for 3 min, followed by centrifuging at 12,000 x g for 15 min at 4 8C. The upper aqueous phase was transferred to another tube containing 525 ml of 100% ethanol (Sigma-Aldrich). The mixture was loaded on RNeasy Mini columns, followed by serial washing with solutions provided in the kit. RNA was finally collected into RNase-free water for further experiments.

Total RNA was isolated from PBMC samples according to the following protocol as described in Lacelle et al., Journal Gerontol A Biol Sci Med Sci. 2002 07; 57(7):285-7. Peripheral blood mononuclear cells (PBMC), are isolated from 30 ml of whole human blood. 1) Prepare 5 x 4ml of Ficoll per patient and gently lay 6 ml of EDTA blood onto the Ficoll layer without mixing and centrifuge at 1500 rpm for 30 min at 4 °C, do not use breaks. 2) Transfer the plasma to one or two 15 ml centrifuge tubes, each with ½ tablet of protease inhibitor. 3) Transfer the white fluffy interphase layer (PBMC) into a 15 ml centrifuge tube. 4) Add 1X sterile PBS to the PBMC and centrifuge at 1500 rpm for 20-30 min at 4° C. 5) Remove the PBS supernatant by inversion and add 4 ml of Trizol per 30 ml whole blood. 6) Mix by repetitive pipetting for 5 min. After homogenization, samples can be stored at -80 °C for at least a year. Note: Incomplete homogenization will results in a lower yield. Homogenizing for too long and too continuously in a small volume (e.g, 1ml) may cause heating of the sample; this may result in degradation of the RNA. Samples should be cooled on ice during homogenization. 7) Add 0.2 ml of chloroform per 1 ml of Trizol reagent and shake tubes vigorously by hand for 15 seconds and incubate samples at room temperature. 8) Centrifuge samples at 12000 x g for 15 minutes at 4°C. Note: The 4° C spin is preferable for phase separation. Room temperature centrifugation may result in variable phase separation, hence resulting in variable RNA yields. 9) Carefully remove the aqueous phase without disturbing the interphase, and transfer it to a fresh tube, save organic phase for DNA and protein isolation. Note: During the transfer of the aqueous phase, avoid pipetting the interphase which contains DNA. 10) Precipitate the RNA from the aqueous phase by mixing with isopropyl alcohol. Add 0.5 ml of isopropyl alcohol per 1ml Trizol used to homogenize the samples. 11) Incubate at room temperature for 10 min and centrifuge at 12,000 x g for 10 min at 4° C. 12) Discard the supernatant and wash the RNA pellet with 1 ml of 75% ethanol per 1 ml of Trizol reagent. Note: This washing step will remove organic residues from the RNA pellet. 13) Mix the samples by inverting the tube or vortexing, and centrifuge at 7,500 x g for 5 min. at 4 °C. 14) Discard the ethanol, and briefly air dry the RNA pellet for 5-10 min, then dissolve it in RNase free water, incubation for 5 min at 50-55C is recommended to completely dissolve the RNA. Note: It is preferable not to let the RNA dry completely as it will decrease its solubility. Partially dissolved RNA samples have an A_{260/280} < 1.6.

The purity (A260/280 ratio) and quantity of total RNA was estimated using the Nanodrop machine. Approximately 2µL of RNA sample was loaded on the machine.

### V - Relative Quantitation of microRNAs by RT - qPCR: Comparative C_{T} Method

Quantification of microRNAs is done using a two-step RT - qPCR. In the reverse transcription step, specific microRNAs looped-primers are used to convert microRNAs to cDNA. The Real time PCR (qPCR) step amplifies the cDNAs using sequence-specific primers (Taqman MicroRNA Assays protocol, 2011, Applied Biosystems).

The following protocol was used for Reverse Transcription. Note: it is preferable to use high quality RNA free of inhibitors (proteins, phenol) for reverse transcription (RT) and PCR reactions. 1) Thaw and thoroughly mix all reagents. Note: Keep all TaqMan microRNAs assays protected from light source, excessive exposure to light may affect the fluorescence of the probes. Use 100 to 200 ng of total RNA per 15µL RT reaction. 2) Each 15µL RT reaction consists of 7µL of master mix (4.16µL of RNase free water, 0.19RNase inhibitor 20U/µL, 0.15 µL of 100mM dNTP, 1.5 µL 10X reverse transcription buffer, and 1.00µL of 50U/µL reverse transcriptase), 3µL of primer, and 5µL RNA sample (adjust the required amount of total RNA to RNase free water). For a 10µL RT reaction, adjust the volumes of master mix and primers accordingly. Always add and excess volume of 10% or more to compensate for pipetting losses. 3) Dispense the appropriate volume of RNase free water (5µL-RNA concentration) into a 0.2 ml polypropylene tube and cap the tube. 4) Prepare the master mix by scaling the volume listed above to the desired number of RT reactions. 5) Mix the master mix and centrifuge for 2-4 seconds at low speed (1500 rpm). 6) If the 5X primers are thawed, centrifuge them at low speed. 7) Add 3µL per reaction of the appropriate 5XmicroRNA primer to the master mix. 8) Centrifuge at 1500 rpm for 2-4 seconds and incubate the master mix tube on ice. 9) Dispense 10 µL of master mix in each tube and keep the tubes on ice. 10) Add the appropriate volume of total RNA in each tube. 11) Centrifuge the tubes at low speed for 2-4 seconds, and keep the tubes on ice until the thermal cycler is ready. Set the thermal cycler using the following parameters in Table 2:

**TABLE 2**

| **Step Type** | **Time (minutes)** | **Temperature (°C)** |
|---|---|---|
| HOLD | 30 | 16 |
| HOLD | 30 | 42 |
| HOLD | 5 | 85 |
| HOLD | ∞ | 4 |

12) Set the reaction volume to 15 µL or 10 µL. 13) Load the tubes/plates into the thermal cycler and press start. Note: The cDNA can be stored at -20 °C for later use.

The following protocol was used for Real time PCR. 1) Prior to use, thaw the Universal PCR Master Mix and mix it thoroughly by swirling the bottle. 2) Thaw the 20X microRNA primers and centrifuge them at low speed for 2-4 seconds. Note: For each experiment, perform at least three biological and two technical replicates per biological sample. 3) Prepare the PCR reaction mix: For a 20 µL reaction volume, add 7.67µL of RNase free water to a 2.0 ml polypropylene microcentrifuge tube, then 10 µL of 2x Universal PCR Master Mix and 1.00 µL of 20X microRNA primer. Note: Add and excess volume of 10% or more to account for pipetting losses. 4) For a 10µL qPCR reaction, adjust the volumes of water, Master Mix and primers accordingly. 5) Transfert 18.67µL of reaction mix into the corresponding plate well. Note: It is recommended to perform at least three replicates for each qPCR reaction. 6) Transfer 1.33µL of cDNA from the RT reaction into each well of the plate. Note: Do not use more than 10% of undiluted cDNA in the qPCR reaction. 7) Seal the plate with an adhesive cover, and centrifuge at 1000 rpm for 2-4 seconds to bring solution to the bottom of the plate and eliminate air bubbles. Note: Incomplete mixing drops on the sides of the wells, and air bubbles will contribute to precision errors. Replicate samples with a C_{T} standard deviation > 0.2 indicate poor precision. 8) Create a plate documents using the following parameters (refer to the appropriate instrument user guide for instructions on plate setup) in Table 3:

**TABLE 3**

| **Step** | Enzyme Activation | **PCR** | |
|---|---|---|---|
| | HOLD | Cycle (40 cycles) | |
| | | Denature | Anneal/Extend |
| Time | 10 min | 15 sec | 60 sec |
| Temp (°C) | 95 | 95 | 60 |

To insert a melting curve, refer to the Bio-Rad CFX Manager Software protocol. 9) Load the reaction plate into the instrument and start the run.

The reference gene most be chosen carefully. Ideally the reference gene should have its own sets of primers and probes. Failure to use a good endogenous control can severely compromise accurate target quantitation. Endogenous control gene expression should vary only slightly in all samples. An illustrative example of an internal control is snoRNA (AB Assay ID 001718, Applied Biosystems). Herein, the inventor used miR-16 as internal control since it is constantly expressed.

If SYBR Green I is used as a fluorescent dye, a melting curve analysis should be performed in order to identify primer dimers and analyze the specificity of the qPCR reaction.

The Data Analysis of RT qPCR Results was performed as follows. There are two sources of variability in qPCR assays that may affect the results: biological variabilities which are due to inherent differences in gene expression levels between organisms, tissues or cells and technical variabilities which are associated with RNA extraction, pipetting, calibration, and standard operating procedure. 1) Manually set the threshold after the qPCR run, and export analyzed results from the instrument software. Threshold level must be in the linear phase of the exponential amplification. Note: For help on how to export data from the instrument's software, refer to the instrument user guide. 2) Calculate the mean and standard deviation values of the replicate samples. Note: Standard deviation of the triplicate is calculated by the ABI PRISM 7500 SDS software. 3) Calculate the ΔC_{T} values. The ΔC_{T} value is calculated by: ΔC_{T} = C_{T} target - C_{T} reference.

The target gene is the microRNA, RNA or DNA sequence or gene of interest, and the reference gene is the gene or microRNA used to normalize experimental results. 4) Calculate the standard deviation of the ΔC_{T} values. The standard deviation of the ΔC_{T} is calculated from the standard deviations of the target and reference values using the formula: SD = (SD target² + SD reference²)^{1/2}.
5) Calculate the ΔΔC_{T} values. Normalize each ΔC_{T} value to a particular ΔC_{T} value of an assay calibrator. ΔΔC_{T} = ΔC_{T} test sample - ΔC_{T} calibrator sample. Calibrator samples are samples used as a basis for comparative expression results. The standard deviation of ΔΔC_{T} is the same as the standard deviation of ΔCT. Note: For human samples, or samples with significant biological variability, calculate 1/ΔC_{T} for both the test samples and the calibrator samples. 6) Calculate the fold change assuming the efficiency of target and reference gene is close to 100%. The fold difference in expression of test samples relative to the calibrator samples is expressed as: 2-^{ΔΔCT} with ΔΔC_{T} - SD and ΔΔC_{T} + SD.

The following validation experiment for the ΔΔC_{T} Method was performed. For the ΔΔC_{T} calculation to be valid, the amplification efficiencies of the target gene and the reference gene must be approximately equal. To determine if the two amplification reactions have the same PCR efficiency, one must look at how the ΔC_{T} varies with template dilutions. 1) Prepare at least 6-log dilutions range of the sample. The sample must express both the target and the reference genes. 2) Plot the log input amount of RNA vs ΔC_{T} values to create a semi-log regression line. 3) If the absolute value of the slope is < 0.1, the efficiencies of the target gene and reference genes are similar, and the ΔΔC_{T} calculation can be used for relative quantization of the target gene.

### VI - Protein extraction from tissue

Protein was extracted using known techniques. In brief, the tissues were chopped and dissolved in 1 ml RIPA buffer (150 mM NaCl, 10 mM Tris, pH 7.2, 0.1% SDS, 1.0% Triton X-100, 1% deoxycholate, 5 mM EDTA, pH 8.0) containing 1 x protease inhibitor cocktail set 3 (Calbiochem, San Diego, CA), vortexed for 30 min at 4 °C, and centrifuged at 10,000 x g for 10 min.

Supernatants containing the proteins were transferred to new tubes, and protein concentrations were determined by Bradford protein assay (Bio-Rad Laboratories, Hercules, CA).

### VII - Protein extraction from serum or plasma

Since serum/plasma contains 57-71% of serum albumin and 8-26% of gamma immunoglobulin (IgG), before protein extraction, plasma samples were processed by standard commercially available kit to remove both of these protein species. Combining an anti-human serum albumin cartridge and a protein G cartridge, both albumin and IgG can be removed. This allows close to up to 90% efficiency. Kit containing both anti-HSA antibody and Protein G affinity chromatography cartridges are available from Applied BioSystems. The plasma protein specimens after processing were then processed for protein concentration measurements by standard Bradford assays.

### VIII - Protein quantification

Fifty micrograms of proteins from each group was subjected to SDS-PAGE and transferred to nitrocellulose membranes (Schleicher & Schuell BioScience, Keene, NH) by electroblotting. Rabbit anti-Sirt1 and rabbit anti-alpha tubulin (Abcam Inc., Cambridge, MA), rabbit anti-protein of interest as primary antibodies, as well as sheep anti-rabbit and rabbit anti-goat HRP-conjugated secondary antibodies (Cappel Laboratories, West Chester, PA), were used to identify the target proteins.

Alpha tubulin from Abcam Inc. was used as the loading control for protein quantification where feasible. For plasma / serum western blotting assays, the standard for loading could not use either β-action or α-tubulin since neither of these two proteins is in constant amount in the plasma protein specimens. Therefore, the loading control in general relied on the image of protein bands across the gel transferred to the nitrocellulose paper, and stained by Ponceau red dye. A prominent band of visible intensity was selected as the standard instead of the above two protein species.

Protein expression was detected by SuperSignal West Pico Chemiluminescent Substrate, according to the manufacturer's instructions (Pierce Biotechnology Inc., Rockford, IL), and quantified by densitometry using ImageQuant software (ImageQuant version 5.2, available on the Internet website of Molecular Dynamics at: http://www5.GElifesciences.com/aptrix/upp01077.nsf/content/homepage_country _select). Intensity results were normalized by reference to the loading control.

### IX Animal Models for Alzheimer's Disease Study

Two types of animal models have been used to investigate pathological risks for Alzheimer's disease. The first group is composed of transgenic mice carrying a variety of mutant forms of the amyloid precursor protein (APP) for Aβ accumulation, or hyperphosphorylation of Tau for neurofibrillary tangles, or APP + Presenilin 1 [Journal of Alzheimer's Disease Volume 15, Number 4, December, 2008; Woodruff-Pak, D. 2008. J. of Alzheimer Disease 15: 507-521]. The second group is a mouse model showing accelerated aging, the senescence-accelerated prone mouse strain 8 (SAMP8) [Akiguchi, et al., (1994) pp 67-72 in The SAM Model of Senescence. (Takeda, T., ed). Amsterdam: Excerpta Medica; Ikegami et al., (1992) Behav. Brain Res. 51: 15-22; Kaisho et al., (1994) Brain Res. 647: 139-144; Katoh-Semba et al.,(1991) Mech. Ageing Dev. 59: 163-175; Katoh-Semba, R., et al. (1993) J. Mol. Neurosci. 4: 107-115; Kawamata et al., (1997) Exp. Gerontol. 32: 161-170; Kitamura et al., (1989) Neuroscience Letter 106: 334-338; Kitamura et al., (1992) Neuroscience Letter 137: 169-162 ; Kurokawa et al., (1996) Neuroscience Letter 214: 45-48; Lyon et al., (1996) Genetic variants and strains of the laboratory mouse. 2 Volumes. Oxford, New York, Tokyo: Oxford University Press; Miyamoto (1997) Exp. Gerontol. 32: 139-148; Miyamoto et al., (1992) Physiol. Behav. 51: 979-985; Miyamoto et al., (1986) Physiol. Behav. 45: 399-406; Nakamura et al., (1995) Acta Neuropathol. 90: 626-632; Nomuraet al., (1997) Behav. Brain Res. 83: Ohsawa et al., (1991) Mech. Ageing Dev. 59: 263-274).

One advantage of using the first (transgenic) group of mice is the knowledge of specific mutations that have been linked to the two hallmarks of AD pathology, amyloid plaques and neurofibrillary tangles. However, most of these mice were created to carry mutant forms of genes present in early-onset familial AD, a form which constitutes less than 5% of human AD victims. Even the best AD transgenic models, showing amyloid production, tau hyperphosphorylation, neuronal cell loss, and impaired learning and memory, are those carrying deletions of NOS2 and AβPP, early-onset AD mutations [Wilcock et al., (2008) J. Alzheimer's Disease 15: 555-569.]. Since so far age is the best predictor for AD, there is almost no mouse model approximating the late-onset/accelerated aging AD which accounts for the majority of human AD victims. This leaves the second mouse model, SAMP8 mice, as the more appropriate model for AD from the aspect that it is based on age rather than gene mutants.

SAMP8 mice have been bred since 1975, and were recently characterized as a model for accelerated aging. These mice have shorter median life spans of ∼12 months, approximate 40 to 50% of that of other laboratory mouse strains, such as C57/B6, etc. This accelerated aging has been linked to dysfunction of oxidative defense with hyper-oxidative stress. These mice show accelerated deterioration of learning and memory, with increased Aβ production, tau hyperphosphorylation, and other additional features such as neuronal cell loss, characteristic of AD pathogenesis. Recently, SAMP8 mice have been used to evaluate drug efficacy in terms of anti-Aging and/or anti-AD effect [Li et al., (2009) Neuroscience 163(3): 741-9; Tajes et al., (2008) J Neuropathol Exp Neurol. 67(6): 612-23]. Therefore, the SAMP8 mouse is the model which is used herein as an animal model for human AD, with age as the major risk factor, accumulating many age-related stresses.

### X - RNA extraction from mice tissues and plasma

Total RNA samples from brains and livers of SAMP8 and control SAMR1 mice were obtained and processed for RNA isolation according to the following protocol. In brief, brain and liver tissues from four male animals were flash frozen in liquid nitrogen. Total RNA was isolated from these tissues by using the Qiagen miRNeasy Mini Kit (Qiagen, Valencia, California) by homogenizing the tissues with QIAzol Lysis Reagent (Qiagen), addition of chloroform (Sigma-Aldrich, St. Louis, MO), and centrifugation. The aqueous portion was then mixed with 100 % ethanol (Sigma-Aldrich), followed by application to RNeasy Mini columns (Qiagen) and further centrifugation, discarding the flow through, and further centrifugation. Finally, 50 µl RNase-free water (Acros Organics, c/o Thermo-Fisher Scientific, Ghent, Belgium) was added to the column and centrifuged for 1 min at 8000 x g at 25 °C. The flow-through now contained the total RNA. Total RNA was then quantified and checked for purity using a NanoDrop 2000c Machine (Thermo Scientific). This procedure was repeated for both brain and liver tissues from all four animals of both SAMP8 and SAMR strains. The yields of RNA isolated from the two tissues of each of the four animals were calculated according to wet weight, as presented in the following:

**TABLE 4**

| | Brain (mg) | *RNA yield (ng/µl) | Volume (µl) | Liver (mg) | *RNA yield (ng/µl) | Volume (µl) |
|---|---|---|---|---|---|---|
| SAMP8-1 | 78.9 | 236 | 100 | 71.0 | 209.6 | 200 |
| SAMP8-2 | 54.6 | 161.6 | 100 | 62.5 | 284.5 | 200 |
| SAMP8-3 | 82.7 | 270.5 | 100 | 69.8 | 152.4 | 200 |
| SAMP8-4 | 99.7 | 297.8 | 100 | 72.7 | 193.7 | 200 |

**TABLE 5**

| | Brain (mg) | *RNA yield (ng/µl) | Volume (µl) | Liver (mg) | *RNA yield (ng/µl) | Volume (µl) |
|---|---|---|---|---|---|---|
| SAMR1-1 | 10.7 | 121.6 | 50 | 97.7 | 792.3 | 200 |
| SAMR1-2 | 49.8 | 265.9 | 50 | 86.9 | 818.4 | 200 |
| SAMR1-3 | 79.5 | 372.4 | 50 | 95.5 | 636.5 | 200 |
| SAMR1-4 | 93.4 | 544.9 | 50 | 97.0 | 934.4 | 200 |

*RNA yield is for half of the tissue weight, because once the tissue was homogenized in Trizol, half was used for RNA isolation while the other half was stored for future processing.

Alternatively, total RNA was isolated with the following protocol as described in Li et al., Mechanisms of Ageing and Dev. 132 (2011) 75-85. Frozen tissue samples were ground to fine powder in a mortar and pestle under liquid nitrogen, and dissolved in Trizol reagent (Invitrogen, Carlsbad, CA). Total RNA was isolated with the RNeasy Mini Kit (Qiagen, Valencia, CA), according to the manufacturer's protocol. Briefly, 700 ml of Trizol reagent containing samples was mixed vigorously with 140 ml of chloroform (Sigma-Aldrich, St. Louis, MO), and incubated at room temperature for 3 min, followed by centrifuging at 12,000 x g for 15 min at 4 8C. The upper aqueous phase was transferred to another tube containing 525 ml of 100% ethanol (Sigma-Aldrich). The mixture was loaded on RNeasy Mini columns, followed by serial washing with solutions provided in the kit. RNA was finally collected into RNase-free water for further experiments.

Total RNA was isolated from PBMC samples according to the following protocol as described in Lacelle et al., Journal Gerontol A Biol Sci Med Sci. 2002 07; 57(7):285-7. Plasma blood mononuclear cells (PBMC), are isolated from 30 ml of whole human blood. 1) Prepare 5 x 4ml of Ficoll per patient and gently lay 6 ml of EDTA blood onto the Ficoll layer without mixing and centrifuge at 1500 rpm for 30 min at 4 °C, do not use breaks. 2) Transfer the plasma to one or two 15 ml centrifuge tubes, each with ½ tablet of protease inhibitor. 3) Transfer the white fluffy interphase layer (PBMC) into a 15 ml centrifuge tube. 4) Add 1X sterile PBS to the PBMC and centrifuge at 1500 rpm for 20-30 min at 4 °C. 5) Remove the PBS supernatant by inversion and add 4 ml of Trizol per 30 ml whole blood. 6) Mix by repetitive pipetting for 5 min. After homogenization, samples can be stored at -80 °C for at least a year. Note: Incomplete homogenization will results in a lower yield. Homogenizing for too long and too continuously in a small volume (e.g, 1ml) may cause heating of the sample; this may result in degradation of the RNA. Samples should be cooled on ice during homogenization. 7) Add 0.2 ml of chloroform per 1 ml of Trizol reagent and shake tubes vigorously by hand for 15 seconds and incubate samples at room temperature. 8) Centrifuge samples at 12000 x g for 15 minutes at 4°C. Note: The 4° C spin is preferable for phase separation. Room temperature centrifugation may result in variable phase separation, hence resulting in variable RNA yields. 9) Carefully remove the aqueous phase without disturbing the interphase, and transfer it to a fresh tube, save organic phase for DNA and protein isolation. Note: During the transfer of the aqueous phase, avoid pipetting the interphase which contains DNA. 10) Precipitate the RNA from the aqueous phase by mixing with isopropyl alcohol. Add 0.5 ml of isopropyl alcohol per 1ml Trizol used to homogenize the samples. 11) Incubate at room temperature for 10 min and centrifuge at 12,000 x g for 10 min at 4 °C. 12) Discard the supernatant and wash the RNA pellet with 1 ml of 75% ethanol per 1 ml of Trizol reagent. Note: This washing step will remove organic residues from the RNA pellet. 13) Mix the samples by inverting the tube or vortexing, and centrifuge at 7,500 x g for 5 min. at 4 °C. 14) Discard the ethanol, and briefly air dry the RNA pellet for 5-10 min, then dissolve it in RNase free water, incubation for 5 min at 50-55C is recommended to completely dissolve the RNA. Note: It is preferable not to let the RNA dry completely as it will decrease its solubility. Partially dissolved RNA samples have an A_{260/280} < 1.6.

### RESULTS

### Altered microRNA expression in AD plasma

This study was performed with small cohorts of AD patients and normal elderly controls (NEC), matched for ethnicity, age, gender and educational level. The inventor has surprisingly and unexpectedly found that biomarkers in the brain of individuals having Alzheimer's Disease (AD) and/or Mild Cognitively Impairment (MCI) can be detected in biological fluids, such as blood, plasma or serum.

In Figure 3, it is shown that the level of microRNAs miR-29a, miR-9, and miR-146a as quantified by quantitative PCR (qPCR) in plasma samples of AD individuals relative to normal elderly control (NEC) individuals. For example, in the specific case of miR-9, the qPCR results show an increase in plasma level of AD relative to NEC.

In Figure 4, it is shown that the level of microRNAs Let-7f, miR-34a, miR-181b, miR-200b, miR-517* and miR-579 is increased in AD plasma relative to control NEC plasma samples. In Figure 4, the boxes extend from the 25th to the 75th percentile, with a line at the median (50th percentile). The whiskers extend above and below the box to show the highest and lowest values.

Similar experiments have been performed with SAMP8 and SAMR1 mice, and show similar results: the level of microRNA associated with AD and/or target protein in plasma reflect the level detected in the brain.

In Figure 5(A), it is shown that in a random AD population of 114 individuals there are at least four (4) subgroups based on the individuals MMSE scores, namely 26 individuals (23%) are outliers and have an MMSE score of 25-30, 40 individuals (36%) have an MMSE score of 21-24, 36 individuals (33%) have an MMSE score of 10-20, and 8 individuals (7%) have an MMSE score of 4-9 (one individual has no MMSE score). The AD outliers with normal range of MMSE scores failed daily function assessment and were thus classified as AD.

In Figure 5(B), it is shown that in a random NEC population of 124 individuals there are at least two (2) subgroups based on the individuals MMSE scores, namely 121 (97%) have an MMSE score of 25-30, and 1 individual (1%) is an outlier and has an MMSE score of 1-24 (2 individuals had no MMSE score). The NEC outlier with low MMSE score passed daily function assessment and was thus classified as NEC.

In Figure 6, it is shown that in the populations of Figures 5A and 5B, the MMSE score does not appear to be age-related. It is also shown that the age distributions for the two groups are similar, from 65 to the low 90s, validating the selection of NEC as truly age-matched.

In Figure 7, it is shown that in the AD population of Figure 5A, there are more individuals with low MMSE scores and education years < 10 than in the NEC control population, validating literature reports (Song et al., Neurology. 77(3): 227-34. Epub July 13; 2011; and Sharp et al., Alzheimer Dis. Assoc. Disord. July 12, 2011).

In Figures 8(A) and 8(B), it is shown box plot distributions of plasma level of miR-34c from an AD population of 78 individuals and an NEC population of 85 individuals determined using qPCR assays and quantified by inverted delta C_{T} value (1/delta C_{T} value). The error bars above and below each box represent the standard deviations (SD) of the inverse C_{T} values, with the center lines as the median values (see, e.g., Gilad et al., (2008) PLoS One 3(9): e3148, for box plot application). The graph discloses a significant difference in plasma level of miR-34c between AD and NEC (** represents p < 0.01). Further, the graph suggests that values of plasma level of miR-34c between 0.15 and 0.2 may be indicative of a likelihood of AD, while values of plasma level of miR-34c higher than 0.2 may be indicative of an increased likelihood of AD.

In Figure 8(C), it is shown that there is a significant increase of approximately 80 fold between delta C_{T} values of AD and NEC individuals (p < 0.01).

In Figures 9(A) and 9(B), it is shown the inverted delta C_{T} values from the above 78 AD and 85 NEC individuals, which values have been correlated with the individuals' MMSE score. The graph discloses the MMSE score on the x axis and the inverted delta C_{T} value on the y axis.

In Figure 9(C), it is shown that there is a significant increase of approximately 50, 120 and 130 fold between delta C_{T} values for circulating miR-34c in respective mild AD (MMSE score of 21-24), moderate AD (MMSE score of 10-20) and severe AD (MMSE score of 4-9) relative to NEC (MMSE score of 25-30) individuals (p < 0.01).

In Figure 10, it is shown the p values of each MMSE subgroup relative to each other or to NEC. ** represents p < 0.01 and * represents p < 0.05.

In Figure 11, the line represents a linear regression that models the relationship between a scalar variable y = 1/delta C_{T} value for miR-34c and explanatory variables denoted x = MMSE score. The linear regression analysis provides a curve with the following equation: y = -0.011x + 0.441 with an R² = 0.379 and suggests that there is an inverse relationship between MMSE scores and the 1/delta C_{T} value of plasma circulating level of miR-34c. In other words, when one considers all the population as a whole, i.e. both the AD and NEC, one obtains a significant correlation (p < 0.05) between the decrease of MMSE score and the increase of plasma circulating level of miR-34c: the lower the MMSE score, the higher the plasma level of miR-34c.

In other words, these results suggest that the deterioration of cognitive abilities in AD individuals, as reflected by decreasing MMSE scores, is accompanied by a general increase of plasma level of miR-34c.

In Figures 12(A) and 12(B), it is shown box plot distributions of plasma level of miR-34a from an AD population of 82 individuals and an NEC population of 101 individuals determined using qPCR assays and quantified by inverted delta C_{T} value (1/delta C_{T} value). The error bars above and below each box represent the standard deviations (SD) of the inverse C_{T} values, with the center lines as the median values. The graph discloses a significant difference in plasma level of miR-34a between AD and NEC (** represents p < 0.01). Further, the graph suggests that values of plasma level of miR-34a between 0.12 and 0.15 may be indicative of a likelihood of AD, while values of plasma level of miR-34a higher than 0.15 may be indicative of an increased likelihood of AD.

In Figure 12(C), it is shown that there is a significant increase of approximately 6 fold between delta C_{T} values of AD and NEC individuals (** represents p < 0.01). In Figures 13(A) and 13(B), it is shown the inverted delta C_{T} values of miR-34a levels from the above 82 AD and 101 NEC individuals, which values have been correlated with the individuals' MMSE score. The graph discloses the MMSE score on the x axis and the inverted delta C_{T} value on the y axis.

In Figure 13(C), it is shown that there is a significant increase of approximately 4, 4 and 11 fold between delta C_{T} values for circulating miR-34a in respective mild AD (MMSE score of 21-24), moderate AD (MMSE score of 10-20) and severe AD (MMSE score of 4-9) relative to NEC (MMSE score of 25-30) individuals (** represents p < 0.01).

In Figure 14, it is shown the p values of each MMSE subgroup relative to NEC. ** represents p < 0.01.

In Figure 15, the line represents a linear regression that models the relationship between a scalar variable y = 1/delta C_{T} value for plasma level of miR-34a and explanatory variables denoted x = MMSE score. The linear regression analysis provides a curve with the following equation: y = -0.002x + 0.198 with an R² = 0.165 and suggests that there is an inverse relationship between MMSE scores and the 1/delta C_{T} value of plasma level of circulating miR-34a. In other words, when one considers all the population as a whole, i.e. both the AD and NEC, one obtains a significant but weak correlation (p < 0.05) between the decrease of MMSE score and the increase of plasma level of circulating miR-34a: the lower the MMSE score, the higher the plasma level of miR-34a.

In other words, these results suggest that the deterioration of cognitive abilities in AD individuals, as reflected by decreasing MMSE scores, is accompanied by a general increase of plasma level of miR-34a.

In Figures 16(A) and 16(B), it is shown box plot distributions of PBMC level of miR-34c from an AD population of 85 individuals and an NEC population of 94 individuals determined using qPCR assays and quantified by inverted delta C_{T} value (1/delta C_{T} value). The error bars above and below each box represent the standard deviations (SD) of the inverse C_{T} values, with the center lines as the median values. The graph discloses that there is no significant difference in PBMC level of miR-34c between AD and NEC.

In Figure 16(C), it is shown that there is no significant variation between delta C_{T} values of AD and NEC individuals.

In Figures 17(A) and 17(B), it is shown the inverted delta C_{T} values from 66 AD and 92 NEC individuals, which values have been correlated with the individuals' MMSE score. The graph discloses the MMSE score on the x axis and the inverted delta C_{T} value on the y axis.

In Figure 17(C), it is shown that there is no significant variation between delta C_{T} values for PBMC level of miR-34c in mild AD (MMSE score of 21-24), moderate AD (MMSE score of 10-20) and severe AD (MMSE score of 4-9) relative to NEC (MMSE score of 25-30) individuals.

In Figure 18, the line represents a linear regression that models the relationship between a scalar variable y = 1/delta C_{T} value and explanatory variables denoted x = MMSE score. The linear regression analysis provides a curve with the following equation: y = -0.00006x + 0.064 with an R² = 0.006 and suggests that there is no relation / association between MMSE scores and the 1/delta C_{T} value of PBMC level of miR-34c (p = 0.3209).

In Figures 19(A) and 19(B), it is shown box plot distributions of PBMC level of miR-34a from an AD population of 92 individuals and an NEC population of 99 individuals determined using qPCR assays and quantified by inverted delta C_{T} value (1/delta C_{T} value). The error bars above and below each box represent the standard deviations (SD) of the inverse C_{T} values, with the center lines as the median values. The graph discloses that there is no significant difference in PBMC level of miR-34a between AD and NEC.

In Figure 19(C), it is shown that there is no significant variation between delta C_{T} values of AD and NEC individuals.

In Figures 20(A) and 20(B), it is shown the inverted delta C_{T} values for PBMC level of miR-34a from 70 AD and 97 NEC individuals, which values have been correlated with the individuals' MMSE score. The graph discloses the MMSE score on the x axis and the inverted delta C_{T} value on the y axis.

In Figure 20(C), it is shown that there is no significant variation between delta C_{T} values for PBMC level of miR-34a in mild AD (MMSE score of 21-24), moderate AD (MMSE score of 10-20) and severe AD (MMSE score of 4-9) relative to NEC (MMSE score of 25-30) individuals.

In Figure 21, the line represents a linear regression that models the relationship between a scalar variable y = 1/delta C_{T} value of PBMC level of miR-34a and explanatory variables denoted x = MMSE score. The linear regression analysis provides a curve with the following equation: y = 0.000x + 0.087 with an R² = 0.010 and suggests that there is no relation / association between MMSE scores and the 1/delta C_{T} value of PBMC level of miR-34a (p = 0.1900).

In Figure 22, the line represents a linear regression that models the relationship between a scalar variable y = 1/delta C_{T} value of miR-34a and explanatory variables denoted x = 1/delta C_{T} value of miR-34c. The linear regression analysis provides a curve with the following equation: y = 0.080x + 0.134 with an R² = 0.025 and suggests that there is a weak significant positive relationship which means that when there is an increase in miR-34c there is also an increase in miR-34a (p = 0.0253).

In Figures 23(A) and 23(B), it is shown box plot distributions of plasma level of miR-181b from an AD population of 87 individuals and an NEC population of 85 individuals determined using qPCR assays and quantified by inverted delta C_{T} value (1/delta C_{T} value). The error bars above and below each box represent the standard deviations (SD) of the inverse C_{T} values, with the center lines as the median values. The graph discloses a significant difference in plasma level of miR-181b between AD and NEC (** represents p < 0.01). Further, the graph suggests that values of plasma level of miR-181b between 0.15 and 0.18 may be indicative of a likelihood of AD, while values of plasma level of miR-181b higher than 0.18 may be indicative of an increased likelihood of AD.

In Figure 23(C), it is shown that there is a significant increase of approximately 5 fold between delta C_{T} values of AD and NEC individuals (** represents p < 0.01).

In Figures 24(A) and 24(B), it is shown the inverted delta C_{T} values for plasma level of miR-181b in 68 AD and 84 NEC individuals, which values have been correlated with the individuals' MMSE score. The graph discloses the MMSE score on the x axis and the inverted delta C_{T} value on the y axis.

In Figure 24(C), it is shown that there is a significant increase of approximately 7, 5 and 5 fold between delta C_{T} values for plasma level of circulating miR-181b in respective mild AD (MMSE score of 21-24), moderate AD (MMSE score of 10-20) and severe AD (MMSE score of 4-9) relative to NEC (MMSE score of 25-30) individuals (respective p < 0.01, p < 0.01 and p < 0.05).

In Figure 25, it is shown the p values of MMSE subgroups relative to NEC. ** represents p < 0.01.

In Figure 26, the line represents a linear regression that models the relationship between a scalar variable y = 1/delta C_{T} value of plasma level of miR-181b and explanatory variables denoted x = MMSE score. The linear regression analysis provides a curve with the following equation: y = -0.007x + 0.382 with an R² = 0.15 and suggests that there is an inverse relationship between MMSE scores and the 1/delta C_{T} value of plasma level of circulating miR-181b. In other words, when one considers all the population as a whole, i.e. both the AD and NEC, one obtains a significant but weak correlation (p < 0.05) between the decrease of MMSE score and the increase of plasma level of circulating miR-181b: the lower the MMSE score, the higher the plasma level of miR-181b.

In other words, these results suggest that the deterioration of cognitive abilities in AD individuals, as reflected by decreasing MMSE scores, is accompanied by a general increase of miR-181b plasma levels at least until the individual reaches the MMSE scores of severe AD.

These results suggest that plasma level of miR-34a and miR-181b may be used as a biomarker of AD status, and that plasma level of miR-34c may be used as a biomarker of AD status as well as a further biomarker of AD progression, e.g. as reflected by the MMSE scores. For example, the plasma level of miR-34c may be used to differentiate between the likelihood that a subject has AD and that the AD disease progression status is likely at the mild stage or moderate stage. The person skilled in the art will be able to understand how to implement the herein described relationship between plasma level of microRNA and AD status and/or AD progression stage.

As of today, there are three known microRNAs of the miR-34 family: miR-34a, b, and c. The miR-34a microRNA is transcribed as a single microRNA, while the miR-34b and miR-34c microRNAs are transcribed as a single RNA molecule which is later cleaved into two separate mature microRNAs (Liang et al., (2009) Current Genomics 10: 184-193). Without being bound by any theory, and based on bioinformatic analysis, it is believed that these three microRNAs have shared target genes as listed in Figures 27(A) and (B). These shared target genes include Sirt1, Bcl2, Onecut2, and Presenilins 1 and 2, in both mouse (panel A) and human sequence comparison analysis (panel B).

In Figures 28(A) and 28(B), is shown the numbers of 'hits' per target gene in their respective mouse (panel A) and human (panel B) sequence comparisons.

In Figure 29(A) is shown the putative binding sequences in mouse and in human for SIRT1 3' UTR. In Figure 29(B) is shown the schematic diagrams of the locations of these binding sequences in mouse (top) and human (bottom).

In Figures 30(A) and 30(B) are shown the putative binding sequences in mouse and in human for Bcl2 3' UTR. In Figure 30(C) is shown the schematic diagrams of the locations of these binding sequences in mouse (top) and human (bottom). In Figures 31 (A), 31(B), 31(C) and 31(D) are shown the putative binding sequences in mouse and in human for Onecut2 3' UTR. In Figure 31 (E) is shown the schematic diagrams of the locations of these binding sequences in mouse (top) and human (bottom).

In Figures 32(A) and 32(B) is shown the putative binding sequences in mouse and in human for Psen1 3' UTR. In Figure 32(C) is shown the schematic diagrams of the locations of these binding sequences in mouse (top) and human (bottom).

In Figure 33(A) is shown the putative binding sequences in mouse and in human for Psen2 3' UTR. In Figure 33(B) is shown the schematic diagrams of the locations of these binding sequences in mouse (top) and human (bottom).

In Figure 34, is shown the nucleotide sequences of mouse (panel A) and human (panel B) mature miR-34a, miR-34b and miR-34c.

In Figure 35, is shown intra-species comparison of the three microRNAs in mouse (panel A) and human (panel B).

In Figure 36, is shown cross-species comparison of the three microRNAs.

Since precursor microRNAs are usually composed of the sense and antisense strands connected by a hairpin loop, sequences of both strands are shown in Figures 34-36.

In Figure 37, it is shown a histogram of circulating putative target proteins of miR-34c in a randomly selected first group of AD and NEC individuals. The putative target proteins are Bcl2, Psen1, Onecut2, Cdk4 and A-beta 42. All histograms represent the mean +/- standard deviation (SD) and * represents p < 0.05. All the protein samples are normalized with the ponceau S stained band. The sample size is shown in the box on the right while the AD relative to NEC p values are shown in the bottom box. The results suggest that AD individuals have an increase in plasma level of Bcl2, Psen1 and Onecut2 relative to NEC, while there is a decrease in plasma level of Cdk4 and A-beta 42 relative to NEC.

In Figure 38(A), it is shown a Western blot result for the plasma level of target protein in 5 AD individuals having the highest plasma level of circulating miR-34c and 5 NEC individuals having the lowest level of circulating miR-34c from the populations tested in Figure 37.

In Figure 38(B), it is shown a histogram showing the normalized circulating level of protein of Figure 38(A), representing the mean +/- SD (n=5) and where ** represents p < 0.01 and * represents p < 0.05. In Figure 38(C), it is shown the raw data for the histograms in Figure 38(B). In Figure 38(D), it is shown the p value for AD vs. NEC.

In Figures 39(A), 39(B), 39(C), 39(D), 39(E), and 39(F) the line represents a linear regression that models the relationship between a scalar variable y = 1/delta C_{T} value of plasma level of miR-34c and explanatory variables denoted x = putative target protein thereof. The linear regression analysis provides a curve with an equation shown in each panel. There is an inverse relationship between plasma level of putative target proteins Bcl2, Onecut2, Psen1 and SIRT1 and the 1/delta C_{T} value of plasma level of circulating miR-34c. In other words, when one considers the population as a whole, i.e. both the AD and NEC, one obtains a significant correlation (p < 0.05) between the decrease of plasma level of putative target proteins Bcl2, Onecut2, Psen1 and SIRT1 and the increase of plasma level of circulating miR-34c: the lower the plasma level of putative target proteins Bcl2, Onecut2, Psen1 and SIRT1, the higher the plasma level of miR-34c. There is a relationship between plasma level of putative target proteins Cdk4 and A-beta 42 and the 1/delta C_{T} value of plasma level of circulating miR-34c. In other words, when one considers the population as a whole, i.e. both the AD and NEC, one obtains a significant correlation (p < 0.05) between the increase in plasma level of putative target proteins Cdk4 and A-beta 42 and the increase of plasma level of circulating miR-34c: the higher the plasma level of putative target proteins Cdk4 and A-beta 42, the higher the plasma level of miR-34c.

In Figure 40, on the top left panel, it is shown a Western blot result for the plasma level of six putative target protein from AD and NEC samples. In the top right panel, it is shown a histogram showing the normalized circulating level of protein form the top left panel, representing the mean +/- SD (n=3) and where ** represents p < 0.01 and * represents p < 0.05. In the bottom left panel, it is shown the p value for AD vs. NEC; in the bottom right panel, it is shown the raw data for the histograms in the top right panel.

In Figure 41, is shown an illustration of a working model which shows AD disease progression from normal (NEC: MMSE 25-30) to mild (MMSE 20-24), moderate (MMSE 10-19), and severe (MMSE 4-9) cognitive decline, as marked by the decrease of MMSE scores with correlated increase in plasma level of circulating miR-34a and/or miR-34c.

In Figure 42, is shown an illustration of a possible mechanism underlying AD patho-physiological signs, such as Tau tangles, Aβ₄₂ and neuronal death induced by elevated level of miR-34a. The person skilled in the art will understand that a similar illustration of possible mechanism can be deducted from the herein described aspects for miR-34c, miR-181b and any other microRNA associated with AD.

In Figure 43, is shown an illustration of a possible triple disadvantage for an increase in miR-34a and/or miR-34c for Alzheimer's disease. The illustrated miR-34x includes miR-34a and/or miR-34c. The person skilled in the art will understand that a similar illustration of possible mechanism can be deducted from the herein described aspects for miR-181b and any other microRNA associated with AD.

In Figure 44, is shown a sequence description of a human miR-34c-GFP construct. The precursor has-miR34c sequence is amplified from genomic DNA from HEK293 cells using the illustrated forward and reverse primers. The precursor miR-34c sequences are flanked by scaffold sequences at 5'- and 3'-sides to ensure that the post-transcriptional product of this microRNA is properly folded. The two scaffold sequences are each flanked by additional oligo sequences, with the Xba1 site restriction oligos at the 5'-end, and the BamHI site restriction oligos for the 3'-end fragment. The oligo-sequences of these fragments containing the two restriction site sequences were then used to generate the forward and reverse primers, which serve as primary oligos for a PCR reaction with genomic DNA from human embryonic kidney 293 (HEK 293) cells, to generate the initial 316 base pair product for cloning into the GFP vector.

In Figure 45(A), is shown an agarose gel image of PCR amplification results for cloning of has-miR-34c using the primers from Figure 44. In Figure 45(B) is shown an agarose gel image of the PCR amplified band from genomic DNA after cloning into vector pCDH-CMV-MCS-EF1-copGFP (CD511B-1) from Systems Biosciences using restriction sites XbaI and BamHI. The cloned sequence was validated by complete sequencing and sequence search alignment on UCSC Genome Sequence database.

The miR-34c-containing plasmid of the GFP vector was then ready to be used for functional analysis, along with the original GFP vector alone, and the GFP vector containing a computer-generated scrambled control sequence, as two negative controls for verification that the putative repression is indeed due to the overexpressed microRNA action, not the vector or vector with an unrelated, scrambled control insert. The GFP-bearing scrambled control vector was purchased from SBI (Systems Biosciences, Mountain View, CA).

In Figure 46, is shown a transfection experiment with the indicated GFP vectors of HEK293 cultures by the lipofectamine procedure, as recommended by the commercial vendor (invitrogen). The cultures transfected with any of these three plasmids, miR-34c-bearing GFP vector, GFP vector alone, or the scrambled control vector, all show close to 95% GFP positivity, while untransfected or mock-transfected cultures (by lipofectamine reagent itself) exhibit no detectable fluorescence signals.

In Figure 47, is shown in the left panel a Western Blot of putative target proteins of miR-34c from cell cultures. On top of the panel, is indicated whether the cell culture was transfected or not (un-infected), and if so, with what construct. β-actin was used as the loading control for all experimental assays, since it is not a target of miR-34x. In the right panel is shown a histogram of band level quantification from the left panel. These results suggest that expression of four target genes, Sirt1, Onecut2, Bcl2 and Psen1 is repressed by overexpression of transfected miR-34c, compared with cultures transfected with vector alone or the scrambled control vector. In contrast, the Cdk4 level is increased in miR-34c transfected, but not in negative control cultures. No difference was observed between scrambled control, vector alone, untransfected or mock transfected cultures. Therefore, scrambled control vector-transfected cultures were used as controls for further analysis of individual gene levels repressed by exogenous miR-34c.

In Figure 48, it is shown on the left panel the ratios of Onecut2, Sirt1, Bcl2 and Psen1 expression compared to β-actin, in miR-34c-transfected cultures compared to the scrambled control. β-action is used here as control since it exhibits no observed difference between scrambled control and miR-34c-transfected cultures. The right panel shows the repression levels for the four proteins, with Onecut2 showing the highest repression of 44.8%, 29% for Sirt1, 26% for Bcl2, 23% for Psen1, and almost no repression at all for β-actin. These results not only suggest that miR-34c indeed represses expression of multiple genes, but also validate the bioinformatic identification of true targets of this microRNA. Moreover, the differential repression levels among the four genes reflect the numbers of 'hits' of these four genes for miR-34c as predicted by the microRNA.org algorithm, which shows the same hierarchic pattern for the four genes, with 4 'hits' for Onecut2, 3 for Sirt1, 2 for Bcl2, and only one for Psen1.

In Figure 49, it is shown on the left panel the primers used to generate the precursor has-miR-34a by PCR on genomic DNA of HEK293 cells. In the right panel, it is shown an agarose gel image of PCR amplification results for cloning of has-miR-34c using the primers from the left panel. The PCR amplified band is indicated by the arrow.

In Figure 50, it is shown on the top left panel, a western blot image of HEK293 cells extracts, which cells were transfected with GFP constructs expressing miR-34a or scrambled control. On top of the panel, is indicated whether the cell culture was transfected or not (un-infected), and if so, with what construct. On the right top panel, it is shown a histogram obtained from the protein band level quantification from the top left panel, where the levels have been normalized with β-Actin as an internal control. (* represents p < 0.05; all histograms represent average ± SD; n = 3, three samples from each of three experiments). Bottom panel: numerical value of the band level intensities in the top left panel. These results suggest that intracellular level of putative target proteins Sirt1, Onecut2, Bcl2 and Psen1 is decreased by increased level of miR-34a, compared with cultures transfected with vector alone or the scrambled control vector. In contrast, the Cdk4 level is increased in miR-34a transfected, but not in negative control cultures. No difference was observed between scrambled control, vector alone, untransfected or mock transfected cultures.

In Figure 51, it is shown a histograms illustrating the statistical analysis of the results obtained in Figure 50. Top panel, left: percentage of fold decrease for each putative protein target; right: percentage of repression. Bottom panel, numerical values from the histograms. In the top left panel, it is shown on the left panel the ratios of Onecut2, Sirt1, Bcl2 and Psen1 intracellular level compared to β-actin intracellular level, in miR-34a-transfected cell cultures extracts compared to the scrambled control cell cultures extract. The protein β-action is used here as control since it exhibits no observed difference between scrambled control and miR-34a-transfected cultures. The right panel shows the repression levels for the four proteins, with Onecut2 showing the highest repression of about 29.95%, 20.58 for Sirt1, 18.37% for Bcl2, 19.18% for Psen1, and almost no repression at all for β-actin. These results not only suggest that miR-34a indeed represses expression of multiple genes, but also validate the bioinformatic identification of true targets of this microRNA.

The herein described results also suggest that one skilled in the art may use, for example but without being limited thereto, the herein described relationship between microRNA associated with AD and/or MCI, or with AD and/or MCI status for implementing a screening system for selecting a candidate therapeutic compound.

In one aspect, this screening system for selecting a candidate therapeutic compound comprises a cell expressing a microRNA associated with AD, where the cell is adapted for being contacted with a compound. One may determine the intracellular level of the microRNA and/or of a target protein thereof, derive information from this intracellular level, and select the candidate therapeutic compound at least partly based on this information. In the specific case of miR-34c, one may use a cell overexpressing miR-34c and determine the intracellular level of miR-34c and/or of its target protein, for example of Bcl2, SIRT1, Onecut2, Psen1, or any combinations thereof, following cell contact with the compound, The person skilled in the art may use the herein described relationship of microRNA and/or protein target thereof with AD / MCI in order to implement a screening system and any other system.

In Figures 52, is shown putative shared target proteins of miR-34a, miR-34c and miR-181b.

In Figure 53, is shown an illustrative hypothetical working model for miR-34a/miR-34c and miR-181b and putative target proteins.

Non-limiting process and apparatus implementing an aspect of the present disclosure.

With reference to Figure 54, there is shown a block diagram of a system 150 comprising a microRNA detecting agent sensor 110, a target protein detecting agent sensor 120, a user input device 118, an apparatus 100 implementing a user interface for displaying information that is indicative of the AD and/or MCI status of a subject, or the likelihood of AD and/or MCI, or the increased likelihood of AD and/or MCI, and the like, as described in the present specification, and a display unit 114. The person skilled in the art will understand that in the context of the disclosure described herein, the system 150 may include either or both sensors 110 and 120.

In accordance with a specific non-limiting implementation, the sensor 110 detects at least one detecting reagent of a microRNA in a sample to generate a signal indicative of the microRNA level in the sample. The resulting signal conveys information related to the level of microRNA in the sample. Such signal, hereinafter referred to as secondary signal, may be a signal generated by qPCR, radiolabeled-oligo northern hybridization, etc. Such sensor may thus be based on spectrophotometer devices (e.g., to detect fluorescence, etc.), scintillation counters (e.g., ionizing radiation, etc.), and the like. Sensors for detecting and measuring secondary signal are well known in the art to which this invention pertains and any suitable sensor may be used.

In accordance with a specific non-limiting implementation, the sensor 120 detects at least one detecting reagent of a target protein in a sample to generate a signal indicative of the target protein level in the sample. The resulting signal conveys information related to the level of target protein in the sample. Such signal, hereinafter referred to as secondary signal, may be a signal generated by chemiluminescence, fluorescence, bioluminescence, ionizing radiation, etc., for example from a molecule linked to an antibody having binding specificity to the target protein of interest. Such sensor may thus be based on spectrophotometer devices (e.g., to detect chemiluminescence, fluorescence, bioluminescence signal), scintillation counters (e.g., ionizing radiation), and the like. Sensors for detecting and measuring secondary signal are well known in the art to which this invention pertains and any suitable sensor may be used.

Alternatively, certain aspects of the system 150 may omit the sensors 110 and/or 120 and instead make use of a user-controlled input 118 for generating the information conveyed by the secondary signal. The user-controlled input allows a user to provide information relating to the secondary signal previously obtained, for example in a previous Western Blot assay, ELISA assay, and the like. The user input device 118 will be described later in the specification.

The apparatus 100 is for implementing a graphical user interface module for displaying information that is indicative in a subject of AD and/or MCI status, or the likelihood of AD and/or MCI, or the increased likelihood of AD and/or MCI, and the like as described in the present specification. The information may be displayed in various forms as will become apparent later on in the specification. Optionally, the graphical user interface module implemented by the apparatus 100 selectively causes an alarm event based at least in part on the information that is indicative in a subject of the AD and/or MCI status, or the likelihood of AD and/or MCI, or the increased likelihood of AD and/or MCI, and the like as described in the present specification. The apparatus 100 also releases a signal for causing the display unit 114 to display the graphical user interface module. Optionally, the apparatus is further adapted for releasing signals to a data output module 130 for causing the latter to convey information related to AD and/or MCI status, or the likelihood of AD and/or MCI, or the increased likelihood of AD and/or MCI, and the like as described in the present specification, to a user of the system 150. Specific examples of implementation of the apparatus 100 and of the graphical user interface module will be described later on in the specification.

The user input device 118 is for receiving data from a user of the system 150. The user input device 118 may be used, for example, to enter information associated with the patient and/or to manipulate the information displayed by the user interface implemented by the apparatus 100. Optionally, the user may also create via the input device 118 an alarm event based at least in part on the information that is indicative in a subject of the AD and/or MCI status, or the likelihood of AD and/or MCI, or the increased likelihood of AD and/or MCI, and the like as described in the present specification. Optionally still, the user input device 118 may be used to enter medical information conveying information associated to administration of a compound to the subject. The medical information may indicate whether (i) an anti-AD and/or MCI compound, and/or (ii) an anti-AD and/or MCI candidate compound, was administered and, optionally, the dosage of the compound that was administered. The user input device 118 may include any one or a combination of the following: keyboard, pointing device, touch sensitive surface, keypad or speech recognition unit. Certain aspects of the system 150 may omit the user input device 118.

The display unit 114 is in communication with the apparatus 100 and receives a signal causing the display unit 114 to display a graphical user interface module implemented by apparatus 100. The display unit 114 may be in the form of a display screen, a printer or any other suitable device for conveying to the physician or other health care professional information associated to the patient. In aspects where the display unit 114 is in the form of a display screen, it may be part of any suitable type of apparatus including, without being limited to, a desktop/laptop computing apparatus, a personal digital assistant (PDA), a telephone equipped to video display capability, a TV monitor or any other suitable device equipped with a display screen for visually conveying information to a user.

Optionally, the system 150 may further include a data output module 130. The data output module 130 is in communication with the apparatus 100 and is suitable for receiving signals generated by the apparatus 100. In a first specific example of implementation, the data output module 130 includes an audio module for releasing audio signals on the basis of signals received from the apparatus 100. In a second specific example of implementation, the data output module 130 includes a data communication entity suitable for transmitting messages to remote devices causing the latter to convey to a user of the system 150 information related to the subject AD and/or MCI status, or likelihood of AD and/or MCI, or increased likelihood of AD and/or MCI, and the like as described in the present specification. Examples of remote devices include, without being limited to, PDAs, telephones, pagers and computing terminals.

### Apparatus 100

A specific example of implementation of apparatus 100 will now be described with reference to Figure 55. The apparatus 100 includes an input 202 (labelled as first input in Figure 55), a processing unit 206 and an output 208. The first input 202 is for receiving a signal originating from a first sensor (e.g., either sensor 110 or sensor 120) and conveying information related to microRNA / protein detecting agent level in a sample. The processing unit 206 is in communication with the first input 202 and implements a graphical user interface for displaying information related to the subject AD and/or MCI status, or likelihood of AD and/or MCI, or increased likelihood of AD and/or MCI, and the like as described in the present specification. The output 208 is for releasing a signal for causing the display unit 114 to display the graphical user interface implemented by processing unit 206. Optionally, as shown in Figure 55, the apparatus further includes a second input 204 for receiving data from a user through input device 118. Optionally still, the apparatus 100 further includes a data interface 210 for exchanging signals with a data output module 130 for causing the latter to convey information related to the subject AD and/or MCI status, or likelihood of AD and/or MCI, or increased likelihood of AD and/or MCI and the like as described in the present specification, to a user of the system 150.

Optionally, the user may input mini-mental state examination (MMSE) or Folstein test information or information relating to the medical background of the patient (for example, but without being limited thereto, familial antecedence, age, genetic markers, e.g., APP and/or Tau mutations, etc.) through input device 118. The apparatus 100 is adapted for processing this information together with the calculated or user entered level of microRNA and/or target protein to determine the AD and/or MCI status, the likelihood of AD and/or MCI, or a level of risk of AD and/or MCI, and the like as described in the present specification. For example, where the information that the user inputs through input device 118 includes MMSE information, the processor is adapted for processing this information together with the level of microRNA and/or target protein to determine AD and/or MCI, or a level of risk of AD and/or MCI through a method as described in the present specification.

The graphical user interface (GUI) module implemented by apparatus 100 will now be described in greater detail.

The graphical user interface module receives the signal conveying information related to the level of detection agent (e.g. microRNA detecting agent and/or target protein detecting agent) and displays first and second information. The first information conveys a level of microRNA and/or target protein which is derived at least in part on the basis of at least a portion of the signal received at input 202. The second information conveys a threshold level of microRNA and/or target protein, where the nature of the second information (microRNA vs. protein) is consistent with the nature of the first information.

In one aspect, the threshold level defines boundaries of safe care and may be set in accordance best practices or in accordance with hospital/care-giver facility policy.

In another aspect, the threshold level defines boundaries of MMSE defined subgroups.

The specific manner in which the information can be displayed to a user of the system 150 by the graphical user interface module may vary from one implementation to the other. Specific non-limiting examples of implementation of a graphical user interface module are shown in Figures 59A, 59B and 61.

A first specific example of implementation of the graphical user interface module is shown in Figures 59A and 59B of the drawings. In the specific implementation shown in Figure 59A, the first information includes a first alphanumeric element 602A conveying a level of microRNA associated to an individual and the second information includes a second alphanumeric element 604 conveying a threshold level of microRNA. The first alphanumeric element 602A and the second alphanumeric element 604 are displayed concurrently in viewing window 600a. In this specific implementation, the first alphanumeric element 602A reflects the current level of microRNA derived on the basis of a signal received at input 202. In this non-limiting example, the first alphanumeric element 602A conveys an absolute number which may represent an actual value, e.g. a concentration of microRNA, or may represent a factory-defined relative value where a specific concentration value of microRNA corresponds to a value on a scale, for example in a scale from 1 to 10 where 10 is the highest concentration and 1 is the lowest concentration. The person skilled in the art will understand that any other way of representing the level of biomarker can be used.

Advantageously, the implementation depicted in Figures 59A and 59B allows the user to also readily appreciate whether the current level is within the boundaries of safe care as conveyed by the second alphanumeric element 604.

Optionally, as depicted in the specific examples shown in Figures 59A and 59B, the graphical user interface module also displays an alphanumeric indicator 610a in the form of a rating for conveying to the user of the graphical user interface module an indication of whether the current level is within the boundaries of safe care. In the example depicted in Figure 59A, the first alphanumeric element 602a conveys a level of 4, which is below the 5 threshold level conveyed by the second alphanumeric element 604. In this case the alphanumeric indicator 610a indicates the message "OK or SAFE" conveying that the current level is within an acceptable range. In Figure 59B, the same example of implementation of the graphical user interface module as that shown in Figure 59A is shown but with a different value of the current level. In this example, in the viewing window 600b, the first alphanumeric element 602B conveys a level of 7, which is above the 5 threshold level conveyed by the second alphanumeric element 604. In this case the alphanumeric indicator 610b indicates the message "ELEVATED RISK" conveying that the current level is not within an acceptable range. In alternative examples of implementation, the alphanumeric indicator 610b may be adapted for displaying graded risk levels such as for example "mildly elevated", "moderately elevated" and "critically elevated" for example or alternatively for displaying cognitive graded classification such as for example "Robust NEC", "Normal NEC", "ADAR", "Early MCI", "Late MCI", "Mild AD", "Moderate AD", "Severe AD", and the like.

### Alarm Events

The graphical user interface module is adapted for selectively causing an alarm event based at least in part on a level of microRNA and/or target protein and the threshold level of microRNA and/or target protein. In a specific example of implementation, the alarm event is for alerting the user of the system (e.g. medical staff, patient, insurance company, lab technician, etc.) of the status of AD / MCI and/or of the likelihood of AD / MCI and/or modulation thereof, and the like as described in the present specification. The alarm event may be triggered in a number of situations and may be optionally at least partly based on user defined parameters and/or factory-based parameters. Non-limiting examples of the manners in which an alarm event may be selectively caused will be described later on in the specification.

An alarm event, in accordance with a specific example of implementation, may include one or more components for communicating information to a user of the graphical user interface module.

In a first specific illustrative implementation, the alarm event includes displaying a visual indicator to convey to a user of the graphical user interface module the likelihood of AD / MCI and/or modulation thereof. The visual indicator may be displayed as part of the graphical user interface module or in a separate display at a remote location. Any suitable type of visual indicator may be used. Examples of visual indicators that may be used include, without being limited to:
Variations in color. For example, a color scheme may be established whereby certain colors are associated with varying levels of risk. Portions of the graphical user interface may turn a certain color associated with a high level of risk when, for example, the level rate of microRNA and/or of target protein falls outside a limit set by the threshold level rate of microRNA and/or of target protein. Alternatively, the entire display window or a portion of the window may be displayed may turn a certain color associated with a high level of risk based at least in part on a level of microRNA and/or of target protein conveyed by the first information and the threshold level of microRNA and/or of target protein. A non-limiting example of a color scheme is green = normal; yellow: intermediate risk level; red: high level of risk, however any suitable color scheme may be used.

Variation in display intensity of the viewing window. For example, flashing or blinking of the viewing window may be used as a visual indicator to draw the attention of the user;
Variation in the size or position of the viewing window. For example, the viewing window may be made to appear more prominently on the display unit or at a location that is more likely to draw the attention of the user;
Displaying a message prompting/alerting the user. For example, in Figure 59B, an alphanumeric message 610b is displayed as "ELEVATED RISK" to convey that the current level of biomarker conveyed by the first alphanumeric element 602B has exceeded the threshold level conveyed by the second alphanumeric element 604. In this example, when the current level rate falls below the threshold rate of 5, either no message may be displayed or a message conveying that the current rate is within the limit set by the threshold as shown in Figure 59A.

In a second specific illustrative implementation, the alarm event includes causing an audio signal to be issued, alone or in combination with a visual indicator, to draw attention of a user of the graphical user interface module. In this second specific implementation, the processing unit 206 releases a signal at the data interface 210 for causing an audio unit (not shown in the figures) to issue an audio signal. The audio unit may be connected directly to the data interface 210 through either a wire-line link or a wireless link. Alternatively, the audio unit may be in communication with the data interface 210 over a network. Alternatively still, the audio unit may be an integral part of apparatus 100.

In a third specific illustrative implementation, the alarm event includes causing a message signal to be transmitted to a remote device. The remote device may be, for example, a PDA, telephone, pager or a remote computing terminal. Other suitable types of remote devices may also be envisaged in other specific implementations. In this third specific implementation, the processing unit 206 releases a signal at the data interface 210 for causing a message signal to be transmitted to the remote device. The remote device may be connected directly to the data interface 210 though either a wire-line link or a wireless link. Alternatively, the remote device may be in communication with the data interface 210 over a network.

In a first practical example of interaction, the remote device is a PDA assigned to a lab technician responsible for overseeing a screening process for identifying and selecting candidate compounds for treatment and/or prevention of AD, where a compound is administered to AD transgenic mice models and a resulting modulation of the level of the biomarker is indicative that the compound may be a candidate compound for treatment and/or prevention of AD. At least based in part on a level rate of biomarker (e.g., microRNA and/or protein) conveyed by the first information and the threshold level of this biomarker, the graphical user interface module selectively sends a message through the data interface 210 and over a network to the PDA of the lab technician to alert that lab technician. The message may include any suitable useful information including, but not limited to, the identification of the candidate compound, the transgenic mouse, the level of microRNA, and the like. Optionally, the message may also enable the PDA of the lab technician to display all or part of the user interface module described in the present specification. For example, the message may enable the PDA of the lab technician to display a user interface of the type depicted in Figures 59A, 59B and/or 61. Alternatively, the message may only indicate that a certain mouse requires closer monitoring of the microRNA. The specific format of the message is not critical.

In second practical example of interaction, the remote device is a remote computing terminal located at a centralised nursing station in a hospital centre. At least based in part on level rate of microRNA and/or target protein conveyed by the first information and the threshold level rate, the graphical user interface module selectively causes a message to be sent to the remote computing terminal. Advantageously, by allowing a message to be transmitted to a remote device, the clinical staff need not be located near the patient or in proximity to the patient to be alerted to potentially problematic situations. In addition, the clinical staff need not be expressly monitoring the progression of the biomarker to be alerted to an unsafe or deteriorating condition for the patient.

### A Process

An exemplary aspect of the process implemented by the graphical user interface will now be described with reference to *FIGURE* 21.

With reference to Figure 58, at step 300, the microRNA detecting agent signal is received by the graphical user interface module. At step 302, the graphical user interface module computes a microRNA level on the basis of the microRNA detecting agent signal received at step 300.

The specific manner in which the microRNA level is computed will depend on the format of the microRNA detecting agent signal. In a first specific example, the microRNA detecting agent signal is obtained through a qPCR resulting signal conveying the amount of microRNA in the sample.

In a specific implementation, the graphical user interface module computes a microRNA level in the sample. The microRNA level in the sample may be computed in a number of suitable manners as described elsewhere in the present specification. It will be readily apparent to the person skilled in the art, in light of the present description, that other well-known techniques for computing a microRNA level on the basis of a microRNA detecting agent signal may be used.

At step 304, the graphical user interface module, implemented by the processing unit 206, displays the first information conveying the level derived at step 302. At step 306, the graphical user interface module, implemented by the processing unit 206, displays concurrently with the first information, the second information conveying a threshold level of microRNA. Specific non-limiting examples of formats for the first information and second information were described with reference to Figures 59A and 59B of the drawings.

At step 308, the graphical user interface module determines, at least in part on the basis of the computed level and the threshold level, whether an alarm event should be caused.

As will become apparent to the person skilled in the art in light of the present specification, different conditions may bring the graphical user interface module to cause an alarm event.

In a first specific example of implementation, an alarm event is triggered depending on the specific circumstances conveyed by the computed level and the threshold level alone.

In a second specific example of implementation, an alarm event is triggered depending on the specific circumstances conveyed by the computed level and the threshold level in combination with other factors. Such other factors may include, without being limited to, MMSE information and the like.

In either one of the above described specific examples of implementation, the conditions for causing an alarm event may be determined on the basis of a hospital policy or in accordance with best recognised practices in health care.

In a specific example of implementation, step 308 shown in Figure 60 includes multiple sub-steps for determining whether an alarm event should be caused. A non-limiting example of implementation of process step 308 may proceed as follows: the graphical user interface module determines whether the computed level exceeds the limit set by threshold level,
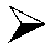 if the answer is in the negative and the computed level does not exceed the limit set by threshold level, step 308 determines that no alarm should be caused and the graphical user interface proceeds to step 300;
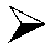 if step 350 is answered in the affirmative and the computed level exceeds the limit set by threshold level, the graphical user interface may proceed to test an additional condition, or may proceed to step 310.

The additional condition may include, for example, in the context of a screening process for identifying candidate compounds for treatment and/or prevention of AD, evaluating the level (or dosage) of administered compound if any was changed and use that information in effecting the decision step.

In the specific example of implementation shown in Figure 60, the multiple sub-steps are optional steps which may be included or omitted from specific implementations. In addition, it will be appreciated in light of the present specification that other suitable manners of determining whether an alarm event should be caused on the basis of the computed level and the threshold level may be used. As such, it should be understood that the example depicted in Figure 60 was presented for the purpose of illustration only.

Returning now to Figure 60, if step 308 determines that an alarm event should be caused, the graphical user interface module proceeds to step 310 where an alarm event is triggered. Examples of alarm events were described previously in the specification. The graphical user interface module then returns to step 300 where another signal may be received and subsequently processed.

If step 308 determines that no alarm event should be caused, the graphical user interface module returns to step 300 where another signal may be received and subsequently processed.

As can be observed, the process illustrated in Figure 60 is an iterative process whereby steps 300 to 308 (and selectively step 310 when an alarm event is caused to occur) are repeated as time progresses and as new segments of the signal are received by the apparatus. Over time, the graphical user interface module processes the signal to derive a set of biomarker level data elements, where each level data element in the set of level data elements is associated to a segment of the signal. In a non-limiting example, the graphical user interface module may compute a running average of level in the signal to derive the set of level data elements. Such may be useful for example to derive a signal over time progression, which may be indicative, for example in the context of a specific implementation relating to the screening for a candidate compound for treatment of prevention of AD and/or MCI, of the relative mid- to long-term therapeutic efficacy of a candidate compound.

Although the exemplary aspect of the process implemented by the graphical user interface described with reference to Figure 60 made reference to a single alarm event presented in box 310, it will be appreciated that different types of alarm events may be caused by the graphical user interface. More specifically, different circumstances conveyed by the computed level, medication information, and optionally other conditions may be associated to respective types of alarm events. Therefore, although the specification described causing a given alarm event, it should be understood that different types of alarm events may be caused and that the type of alarm event caused may be conditioned at least in part on the basis of the circumstances conveyed by the computed level, (optionally) medication information, and optionally other conditions.

### Variant

As a variant, the graphical user interface module is adapted for displaying, concurrently with the first information conveying level of biomarker and the second information conveying a threshold level of the biomarker, additional information elements related to disease progression, e.g. MMSE score.

Figure 61 of the drawings depicts a non-limiting example of implementation of a display generated by the graphical user interface module in accordance with this variant.

As shown, the graphical user interface module displays a first tracing (top triangle) conveying levels of plasma biomarker, a second (optional) tracing (middle triangle) conveying an MMSE score, a third (optional) Disease progression tracing (bottom triangle), a fourth element which sub-divides at least a part of the window and/or at least a part of any of these tracing according to disease status, such as but without being limited thereto, "normal", "MCI", "Mild AD", "Moderate AD", "Severe AD", and the like. These tracings and element may be displayed in a single window or in a plurality of separate windows.

### Specific Physical Implementation

Those skilled in the art should appreciate that in some aspects, all or part of the functionality previously described herein with respect to the apparatus for implementing a user interface for displaying information related to a subject AD and/or MCI status, or likelihood of AD and/or MCI, or increased likelihood of AD and/or MCI, and the like as described throughout this specification, may be implemented as pre-programmed hardware or firmware elements (e.g., application specific integrated circuits (ASICs), electrically erasable programmable read-only memories (EEPROMs), etc.), or other related components.

In other aspects, all or part of the functionality previously described herein with respect to the apparatus for implementing a user interface for information related to a subject AD and/or MCI status, or likelihood of AD and/or MCI, or increased likelihood of AD and/or MCI, and the like as described throughout this specification, may be implemented as software consisting of a series of instructions for execution by a computing unit. The series of instructions could be stored on a medium which is fixed, tangible and readable directly by the computing unit, (e.g., removable diskette, CD-ROM, ROM, PROM, EPROM or fixed disk), or the instructions could be stored remotely but transmittable to the computing unit via a modem or other interface device (e.g., a communications adapter) connected to a network over a transmission medium. The transmission medium may be either a tangible medium (e.g., optical or analog communications lines) or a medium implemented using wireless techniques (e.g., microwave, infrared or other transmission schemes).

The apparatus implementing a user interface for displaying information related to a subject AD and/or MCI status, or likelihood of AD and/or MCI, or increased likelihood of AD and/or MCI, and the like as described throughout this specification, may be configured as a computing unit of the type depicted in Figure 56, including a processing unit 702 and a memory 704 connected by a communication bus 708. The memory 704 includes data 710 and program instructions 706. In a specific example of implementation, the data 710 stored in memory 704 includes: one or more threshold level, alternatively or additionally one or more MMSE score value, alternatively or additionally one or more correlation between biological / psychological parameters and MCI / AD status, and the like. The processing unit 702 is adapted to process the data 710 and the program instructions 706 in order to implement the functional blocks described in the specification and depicted in the drawings. In a non-limiting implementation, the program instructions 706 implement the functionality of processing unit 206 described above. The processing unit 702 may also comprise a number of interfaces 712, 714, 716 for receiving or sending data elements to external devices. For example, interface 712 is used for receiving data streams indicative of biomarker detecting agent signal and interface 714 is used for receiving control signals and/or information from the user. Interface 716 is for releasing a signal causing a display unit to display the user interface generated by the program instructions 706.

It will be appreciated that the system for implementing a user interface for displaying information related to a subject AD and/or MCI status, or likelihood of AD and/or MCI, or increased likelihood of AD and/or MCI, and the like as described throughout this specification, may also be of a distributed nature where the biomarker detecting agent signal is collected at one location by a sensor and transmitted over a network to a server unit implementing the graphical user interface. The server unit may then transmit a signal for causing a display unit to display the graphical user interface. The display unit may be located in the same location as the sensor, in the same location as the server unit or in yet another location. Figure 57 illustrates a network-based client-server system 900 for displaying information related to a subject AD and/or MCI status, or likelihood of AD and/or MCI, or increased likelihood of AD and/or MCI, and the like as described throughout this specification. The client-server system 900 includes a plurality of client systems 912, 914, 916, 918 connected to a server system 910 through network 920. The communication links 950 between the client systems 912, 914, 916, 918 and the server system 910 can be metallic conductors, optical fibers or wireless. The network 920 may be any suitable network including but not limited to a global public network such as the Intranet, a private network and a wireless network. The server 910 may be adapted to process and issue signals to display multiple biomarker levels derived from multiple sensors 926, 928 concurrently using suitable methods known in the computer related arts.

The server system 910 includes a program element 960 for execution by a CPU. Program element 960 implements similar functionality as program instructions 706 and includes the necessary networking functionality to allow the server system 910 to communicate with the client systems 912, 914, 916, 918 over network 920. In a non-limiting implementation, program element 960 includes a number of program element components, each program element components implementing a respective portion of the functionality of the user interface for displaying information related to a subject AD and/or MCI status, or likelihood of AD and/or MCI, or increased likelihood of AD and/or MCI, and the like as described throughout this specification.

Figure 58 shows a non-limiting example of the architecture of program element 960 at the server system. As shown, the program element 960 includes three program element components:
1. the first program element component 800 is executed on server system 910 and is for receiving a biomarker detecting agent signal (e.g., a signal from a qPCR result) conveying information related to the level of biomarker in a subject;
2. the second program element component 802 is executed on server system 910 and is for sending messages to a client system, say client system 914, for causing client system 914 to:
   - display first information conveying level of biomarker, the first information being derived at least in part on the basis of at least a portion of the biomarker detecting agent signal; and
   - display, concurrently with the first information, second information conveying a threshold level of this biomarker;
3. the third program element component 804 is executed on server system 910 and is for selectively sending messages to client system 914 for causing an alarm event based at least in part on the level of biomarker conveyed by said first information and the threshold level of this biomarker. Alternatively, the third program element component 804 is executed on server system 910 and is for selectively sending messages to a client system distinct from the client system 914 for causing an alarm event at the distinct client system. The messages for causing an alarm event may include alarm program elements for execution at the client system, the alarm program elements implementing the alarm events when executed at the client system. Alternatively, alarm program elements for implementing the alarm events are stored at the client system and the messages for causing an alarm event transmitted from the server system 910 include instructions for causing the alarm program elements at the client system to be executed.

Those skilled in the art should further appreciate that the program instructions 706 and 960 may be written in a number of programming languages for use with many computer architectures or operating systems. For example, some aspects may be implemented in a procedural programming language (e.g., "C") or an object oriented programming language (e.g., "C++" or "JAVA").

These programs can be further applied with finer separation of MCI from mild AD and normal elderly controls (NEC). Figure 62 shows miR-34c levels in MCI plasma specimens between the ranges of AD and NEC. Further separation of AD into three groups, *i.e.* mild, moderate, and severe cases, defined by MMSE scores in the ranges of 21-24, 10-20, and 4-9 respectively, shows that the miR-34c level in plasma from MCI patients is intermediate between mild AD and normal elderly controls (NEC) (Figure 63). Similar differentiation of MCI from Alzheimer's disease patients can also be seen by assaying miR-34a levels in plasma specimens of these two clinical cohorts (Figure 64). However, qPCR values show no significant difference in this microRNA's levels in the plasma of MCI and NEC groups, suggesting that this microRNA cannot be used to differentiate mildly cognitively impaired individuals from normal elderly controls. Thus, while miR-34c in plasma can be used to differentiate all three groups of AD, *i.e.* mild, moderate, and severe, from both MCI and normal elderly control groups, miR-34a levels in plasma differentiate only MCI from the AD group.

### ADDITIONAL REFERENCES

1. Wang E. (2007) MicroRNA, the putative molecular control for mid-life decline. Aging Research Review 6(1): 1-11.
2. Finch CE. (2009) The neurobiology of middle-age has arrived. Neurobiology of Aging 30: 515-520.
3. Nemetz, P.N., Leibson, C., Naessens, J. M., Beard, M., Kokmen, E., Annegers, J. F., and Kurland, L. T. (1999) Traumatic brain injury and time to onset of Alzheimer's disease: a population-based study. Am. J. Epidemiol. 149: 32-30.
4. Van Den Heuvel, C., Thornton, E., and Vink, R. (2007) Traumatic brain injury and Alzheimer's disease: a review. Progress in Brain Research. 161: 303-316.
5. Emmerling, M. R., Morganti-Kossmann, M. C., Kossmann, T., Stahel, P. F., Watson, M. D., Evans, L. M., Mehta, P. D., Spiegel, K., Kuo, Y-M., Roher, A.E., and Raby, C. A. Traumatic brain injury elevates the Alzheimer's amyloid peptide Aβ42 in human CSF: a possible role for nerve cell injury. Annals of the New York Academy of Sciences
6. Takeda T, Hosokawa M, Higuchi, K. (1991) Senescence-accelerated mouse (SAM): A novel murine model of accelerated senescence. J. Amer. Geriatr. Soc. 39: 911-919.
7. Ali AK, Banks WA, Kumar VB, et al. (2009) Nitric oxide activity and isoenzyme expression in the senescence-accelerated mouse p8 model of Alzheimer's disease: effects of anti-amyloid antibody and antisense treatments. J Gerontol A Biol Sci Med Sci. 64(10):1025-1030.
8. Woodruff-Pak D. (2008) Animal models of Alzheimer's disease: Therapeutic Implications. Jour. of Alzheimer Disease 15: 507-521.
9. Li, N., Bates, D. J., An, J., Terry, D. A., and Wang, E. (2009) Up-regulation of key microRNAs, and inverse down-regulation of their predicted oxidative phosphorylation target genes, during aging in mouse brain. Neurobiology of Aging. in press. PMID: 19487051
10. Khanna, A., Muthusamy, S., Liang, R., Sarojini, H., and Wang, E. (2011) Gain of survival signaling by down-regulation of three key miRNAs in brain of calorie-restricted mice. Aging 3(3): 1-14.
11. Li, X., Khanna, A., Li, N., and Wang, E. Circulatory miR-34a as an RNA-based, noninvasive biomarker for brain aging. Aging 3(10): 1-14 (2011). PMID:22064828
12. Wilcock DM, Colton CA. (2008) Anti-amyloid-β immunotherapy in Alzheimer's disease: relevance of transgenic mouse studies to clinical trials. Journal of Alzheimer's Disease 15: 555-569.
13. Hsiao K, Chapman P, Nilsen S, et al. (1996) Correlative memory deficits, elevation and amyloid plaques in transgenic mice. Science 274:99-102.
14. Delay, C., and Hebert, S. S. (2011) MicroRNAs and Alzheimer's disease mouse models: current insights and future research avenues. International J. of Alzheimer's disease. Doi: 10: 4061/2011/894938.
15. Wang X, Liu P, Zhu H, Xu Y, Ma C, Dai X, Huang L, Liu Y, Zhang L, and Qin C. (2009) miR-34a, a microRNA up-regulated in a double transgenic mouse model of Alzheimer's disease, inhibits bcl2 translation Brain Res Bull. 80:268-73.
16. Golde, W.T., Gollobin, P., and Rodriguez, L.L. (2005) A rapid, simple, and humane method for submandibular bleeding of mice using a lancet. Lab Animal Technique 34(2): 39-43
17. http://www.ars.usda.gov/is/pr/2005/050921.htm, or page 20 of the USDA Annual Report of Agency
   Technology Transfer, available at the following website: http://www.ars.usda.govSP2UserFiles/Place/00000000/OTTGeneral/commercere port05.pdf.
18. Li Q, Zhao HF, Zhang ZF, et al. (2009) Long-term green tea catechin administration prevents spatial learning and memory impairment in senescence-accelerated mouse prone-8 mice by decreasing Aβ1-42 oligomers and upregulating synaptic plasticity-related proteins in the hippocampus. Neuroscience 163(3): 741-749.
19. Tajes M, Gutierrez-Cuesta J, Folch J, et al. (2008) Lithium treatment decreases activities of tau kinases in a murine model of senescence. J Neuropathol Exp Neurol. 67(6): 612-623.
20. Ali AK, Banks WA, Kumar VB, et al. (2009) Nitric oxide activity and isoenzyme expression in the senescence-accelerated mouse p8 model of Alzheimer's disease: effects of anti-amyloid antibody and antisense treatments. J Gerontol A Biol Sci Med Sci. 64(10):1025-1030.
21. Pinheiro, J. C. and Bates, D. M. (2000) Mixed-Effects Models in S and S-PLUS. New York: Springer-Verlag. ISBN: 978-0-387-98957-0.
22. Demidenko, E. (2004) Mixed models: Theory and Applications. Hoboken, NJ: J. Wiley & Sons, Inc.
23. Rosner, B. (2008) Fundamentals of Biostatistics. Thomson Brooks/Cole, Belmont, CA. ISBN 0131569872
24. Schroeder, A., Mueller, O., Stocker, S., Salowsky, R., Leiber, M., Gassmann, M., Lightfoot, S., Menzel, W., Granzow, M., and Raqq, T. (2006) The RIN: an RNA integrity number for assigning integrity values to RNA measurements. BMC Mol. Biol. doi: 10.1186/1471-2199-7-3.
25. Simone Fleige, Michael W. Pfaffl. RNA integrity and the effect on the real-time qRT-PCR performance. Molecular Aspects of Medicine 27 (2006) 126-139
26. McShea A., Harris, P. L., Webster, K. R., Wahl, A.F., and Smith, M. A. (1997) Abnormal expression of the cell cycle regulators P16 and CDK4 in Alzheimer's disease. Am J Pathol. 150(6):1933-9.
27. Stone, J.G., Siedlak, S.L., Tabaton, M., Hirano, A., Castellani, R.J., Santocanale, C., Perry, G., Smith, M.A., Zhu, X., and Lee, H. G. (2011) The Cell Cycle Regulator Phosphorylated Retinoblastoma Protein Is Associated With Tau Pathology in Several Tauopathies. J Neuropathol Exp Neurol. 70(7):578-587.
28. Liang, R, Bates, D. J., and Wang, E. (2009) Epigenetic control of microRNA expression and aging. Current Genomics 10: 184-193.
29. Lanceta, J., Liang, R., Prough, R. A. and Wang, E. (2010). MicroRNA Group Disorganization in Aging. Experimental Gerontology. 45, 269-278.
30. Liang, R., Khanna, A., Muthusamy, S., Li, N., Sarojini, H., Kopchick, J., Masternak, M.M., Bartke, A., and Wang, E. Post-transcriptional regulation of IGF1R by key microRNAs in long-lived mutant mice. Aging Cell. 10(6): 1080-1088 (2011). PMID:21967153
31. Duran, E. M., Shapshak, P., Worley, J., Minager, A., Ziegler, F., Haliko, S., Moleon-Borodowski, I., and Haslett, P. A. J. (2005) Presenilin-1 detection in brain neurons and Fox03 in peripheral blood mononuclear cells: normalize gene selection for real time reverse transcriptase PCR using ΔΔCT method. Frontiers in Bioscience 10: 2955-2965.
32. Citron M, Westaway D, Xia W, Carlson G, Diehl T,Levesque G, Johnson-Wood K, Lee M, Seubert P, Davis A, Kholodenko D, Motter R, Sherrington R, Perry B, Yao H, Strome R, Lieberburg I, Rommens J, Kim S, Schenk D, Fraser P, St George Hyslop P, Selkoe DJ (January 1997). "Mutant presenilins of Alzheimer's disease increase production of 42-residue amyloid beta-protein in both transfected cells and transgenic mice". Nat. Med. 3 (1): 67-72.
33. Zhang C, Wu B, Beglopoulos V, Wines-Samuelson M, Zhang D, Dragatsis I, Südhof TC,Shen J (July 2009). "Presenilins are Essential for Regulating Neurotransmitter Release".Nature 460 (7255): 632-6.
34. Brouwers N, Sleegers K, Van Broeckhoven C. (2008). "Molecular genetics of Alzheimer's disease: an update". Ann Med 40 (8): 562-83.
35. Ho, A., and Shen, J. (2011) Presenilins in synaptic function and disease. Trends Mol. Med. 17: 617-24.
36. Clotman, F., Jacquemin, P., et al., (2005) Control of liver cell fate decision by a gradient of TGFβ signaling modeulated by Onecut transcriptional factors. Genes Dev. 19: 1849-1845.
37. Shi, G.P., Chapman, H.A., Bhairi, S.M., DeLeeuw, C., Reddy, V.Y., Weiss, S.J. (1995) Molecular cloning of human cathepsin O, a novel endoproteinase and homologue of rabbit OC2. FEBS Lett.
38. Hodge, L. K., Klassen, M. P., Han, B. X., Yiu, G., Hurrell, J., Howell, A., Rousseau, G., Lemaigre, F., Tessier-Laavingne, M., and Wang, F. (2007) Retrograde BMP signaling regulates trigeminal sensory neuron identities and the formation of precise face maps. Neuron 55: 572-586.
39. Francius, C., and Clotman, F. (2010). Dynamic expression of the onecut transcription factors HNF-6, OC-2, and OC-3 during spinal motor neuron development. Neuroscience 165: 116-129.
40. Simion, A., Laudadio, I., Prevot, P.P., Raynaud, P., Lemaigre, F. P., and Jacquemin, P. (2010). MiR-495 and miR-218 regulate the expression of the Onecut transcription factors HNF-6 and OC-2. Biochem. Biophys. Res. Communication. 391: 293-298.
41. Plaisance, V., Abderrahmani, A., Perret-Menoud, V., Jacquemin, P., Lemaigre, F., Regazzi, R. J. Biol. Chem. (2006) MicroRNA-9 Controls the Expression of Granuphilin/Slp4 and the Secretory Response of Insulin-producing Cells.
42. Rabinowits G, Gerçel-Taylor C, Day JM, Taylor DD, Kloecker GH Exosomal microRNA: a diagnostic marker for lung cancer Clin Lung Cancer. 2009 Jan;10(1):42-6.
43. Suarez-Gomez M, Alejandre-Duran E, Ruiz-Rubio M. [MicroRNAs in bipolar disorder: diagnostic and therapeutic applications.] Rev Neurol. 2011 Jul 16;53(2):91-98.
44. Turchinovich A, Weiz L, Langheinz A, Burwinkel B. Characterization of extracellular circulating microRNA Nucleic Acids Res. 2011 May 24. [Epub ahead of print]PMID: 21609964
45. Weng H, Shen C, Hirokawa G, Ji X, Takahashi R, Shimada K, Kishimoto C, Iwai N. Plasma miR-124 as a biomarker for cerebral infarction Biomed Res. 2011;32(2):135-41.
46. Kotlabova K, Doucha J, Hromadnikova I. Placental-specific microRNA in maternal circulation-identification of appropriate pregnancy-associated microRNAs with diagnostic potential. J Reprod Immunol. 2011 May;89(2):185-91.
47. Gaughwin PM, Ciesla M, Lahiri N, Tabrizi SJ, Brundin P, Björkqvist M. Hsa-miR-34b is a plasma-stable microRNA that is elevated in pre-manifest Huntington's disease Hum Mol Genet. 2011 Jun 1;20(11):2225-37.
48. Ray, S., Britschgi, M., Herbert, C., Takeda-Uchimura, Y., Boxer, A., Blennow, K., Friedman, L. F., Galasko, D. R., Jutel, M., Karydas, A., Kaye, J. A., Leszek, J., Miller, B. L., Minthon, L., Quinn, J. F., Rabinovici, G. D., Robinson, W. H., Sabbagh, M. N., So, Y. T., Sparks, D. L., Tabaton, M., Tinklenberg, J., Yesavage, J. A., Tibshirani, R., and Wyss-Coray, T. (2007) Classification and prediction of clinical Alzheimer's diagnosis based on plasma signaling proteins. Nat. Med. 13: 1359-62.
49. Salehi, Z., Mashayekhi, F., and Naji, M. (2008) Insulin like growth factor-1 and insulin like growth factor binding proteins in the cerebrospinal fluid and serum from patients with Alzheimer's disease. BioFactors 33: 99-106.
50. Hye, A., Lynham, S., Thambisetty, M., Causevic, M., Campbell, J., Byers, H. L., Hopper, C., Rijsdijk, F., Tabrizi, S. J., Banner, S., Shaw, C. E., Foy, C., Poppe, M., Archer, N., Hamilton, G., Powell, J., Brown, R. G., Sham, P., Ward, M., and Lovestone, S. (2006) Proteome-based plasma biomarkers for Alzheimer's disease. Brain 129: 3042-50.
51. Maes, O. C., Yu, B, Xu, S., Chertkow, H.M., Wang, E., and Schipper, H. M. (2007) Transcriptional profiling of Alzheimer blood mononuclear cells by microarray. Neurobiology of Aging 28: 1795-1809. (2007).
52. Schipper, H. M., Maes, O. C., Chertkow, H. M., and Wang, E. (2007) MicroRNA expression in Alzheimer blood mononuclear cells. Gene Regulation and Systems Biology 1: 263-274.
53. Maes, O. C., Chertkow, H. M., Wang, E., and Schipper, H. M. (2009) MicroRNA: Implications for Alzheimer disease and other human CNS disorders. Current Genomics 10: 154-168.
54. Schipper, H. M., Maes, O. C., Chertkow, H. M., and Wang, E. (2007) MicroRNA expression in Alzheimer blood mononuclear cells. Gene Regulation and Systems Biology 1: 263-274.
55. Li, X., Khanna, A., Li, N., and Wang, E. Circulatory miR-34a as an RNA-based, noninvasive biomarker for brain aging. Aging 3(10): 1-14 (2011). PMID:22064828
56. Haberman AB. (2004) Therapeutic strategies in animal models, The case of Alzheimer's disease. Genetic Engineering News 24(16): 28-31.
57. Walsh, D. M. and Selkoe, D. J. (2007) A beta oligomers - a decade of discovery. J. Neurochem. 101: 1172-1184.
58. Hardy, J. and Selkoe, D. J. (2002) The amyloid hypothesis of Alzheimer's disease: progress and problems on the road to therapeutics. Science 297: 353-356.
59. McGowan E, Eriksen J, Hutton M. A decade of modeling Alzheimer's disease in transgenic mice. Trends Genet. 2006 May;22(5):281-9.
60. Zhang Q, Zhang X, Sun A. Truncated tau at D421 is associated with neurodegeneration and tangle formation in the brain of Alzheimer transgenic models Acta Neuropathol. 2009 Jun;117(6):687-97
61. Benveniste H, Ma Y, Dhawan J, Gifford A, Smith SD, Feinstein I, Du C, Grant SC, Hof PR Anatomical and functional phenotyping of mice models of Alzheimer's disease by MR microscopy Ann N Y Acad Sci. 2007 Feb;1097:12-29. Review
62. Y.Y. Li a, J.G. Cuia, J.M. Hill, S. Bhattacharjeea, Y. Zhaoe, W.J. Lukiw Increased expression of miRNA-146a in Alzheimer's disease transgenic mouse models. Neuroscience Letters 487 (2011) 94-98
63. Wang H, Liu J, Zong Y, Xu Y, Deng W, Zhu H, Liu Y, Ma C, Huang L, Zhang L, Qin C. miR-106b aberrantly expressed in a double transgenic mouse model for Alzheimer's disease targets TGF-β type II receptorBrain Res. 2010 Oct 21;1357:166-74. Epub 2010 Aug 13
64. P. T. Nelson, W. X. Wang, and B. W. Rajeev, "MicroRNAs (miRNAs) in neurodegenerative diseases," Brain Pathology, vol. 18, no. 1, pp. 130-138, 2008.
65. Petersen RC Clinical practice. Mild cognitive impairment. N Engl J Med. 2011 Jun 9;364(23):2227-34. Review
66. Borenstein AR, Copenhaver CI, Mortimer JA. Early-life risk factors for Alzheimer disease Alzheimer Dis Assoc Disord. 2006 Jan-Mar;20(1):63-72.
67. Albert, M., S., DeKosky, S. T., Dickson, D., Dubois, B., Feldman, H. H., Fox, N. C., Gamsst, A., Holtzman, Jagust, W. J., Petersen, Snyder, P. J., Carrillo, M. C., Thies, B., and Phelps, C. H. (2011). The diagnosis of mild cognitive impairment due to Alzheimer's disease: Recommendations from the National Institute on Aging-Alzheimer's association workgroups on diagnostic guidelines for Alzheimer's disease. Alzheimer's & Dementia 7: 270-279.

## Claims

1. A method for determining a level of likelihood of moderate Alzheimer's disease (AD), mild AD, or severe AD or for determining a level of likelihood for distinguishing between Alzheimer's disease (AD) and mild cognitive impairment (MCI) in a subject, said method comprising
- determining a level of a circulating microRNA from a plasma or serum sample from the subject, said microRNA being selected from the group consisting of miR-34a and miR-34c,
- comparing the level of the respective circulating microRNA to a reference level of the circulating microRNA to derive information conveying a level of likelihood of moderate AD, or mild AD, or severe AD or a level of likelihood for distinguishing between MCI and AD, wherein said reference control level is a circulating level in a serum or plasma sample from a normal elderly control subject; and
- conveying the information to a recipient for determining a level of likelihood of moderate AD, or mild AD, or severe AD or a level of likelihood for distinguishing between MCI and AD.

2. The method as defined in claim 1, wherein said microRNA is miR-34c and said information conveys a level of likelihood of moderate AD, or mild AD, or severe AD.

3. The method as defined in claim 1, wherein said microRNA is miR-34c and said information conveys a level of likelihood for distinguishing between MCI and any one of mild AD, moderate AD, and severe AD.

4. The method as defined in claim 1, wherein said microRNA is miR-34a and said information conveys a level of likelihood for distinguishing between MCI and AD.

## Patentansprüche

1. Ein Verfahren zur Bestimmung einer Wahrscheinlichkeit für eine moderate Alzheimer Erkrankung (AD), milde AD oder schwere AD oder zur Bestimmung einer Wahrscheinlichkeit zur Unterscheidung zwischen Alzheimer Erkrankung (AD) und einer milden kognitiven Beeinträchtigung (MCI) in einem Subjekt, das Verfahren umfasst
- Bestimmen einer Menge an zirkulierender mikroRNA in einer Plasma- oder Serumprobe des Subjekts, die mikroRNA ist ausgewählt aus der Gruppe bestehend aus miR-34a und miR-34c,
- Vergleichen der Menge der entsprechend zirkulierenden mikroRNA mit einer Referenzmenge an der zirkulierenden mikroRNA, um Informationen zu erhalten, die eine Wahrscheinlichkeit einer moderaten AD oder milden AD oder schweren AD vermitteln oder eine Wahrscheinlichkeit, um zwischen MCI und AD zu unterscheiden, wobei die Referenzkontrollmenge eine zirkulierenden Menge in einer Serum- oder Plasmaprobe eines normalen älteren Kontrollsubjekts ist; und
- Übermitteln der Information an einen Empfänger zur Bestimmung einer Wahrscheinlichkeit für moderate AD oder milde AD oder schwere AD oder einer Wahrscheinlichkeit, um zwischen MCI und AD zu unterscheiden.

2. Das Verfahren wie in Anspruch 1 definiert, wobei die mikroRNA miR-34c ist und die Information eine Wahrscheinlichkeit für moderate AD oder milde AD oder schwere AD vermittelt.

3. Das Verfahren wie in Anspruch 1 definiert, wobei die mikroRNA miR-34c ist und die Information eine Wahrscheinlichkeit zur Unterscheidung zwischen MCI und irgendeiner von milder AD, moderater AD und schwerer AD ist.

4. Das Verfahren wie in Anspruch 1 definiert, wobei die mikroRNA miR-34a ist und die Information eine Wahrscheinlichkeit zur Unterscheidung zwischen MCI und AD vermittelt.

## Revendications

1. Méthode pour déterminer un taux de probabilité de maladie d'Alzheimer (MA) modérée, de MA légère, ou de MA sévère ou pour déterminer un taux de probabilité pour faire la distinction entre une maladie d'Alzheimer (MA) et un trouble cognitif léger (TCL) chez un sujet, ladite méthode comprenant
- la détermination d'un taux d'un microARN circulant à partir d'un échantillon de plasma ou de sérum provenant du sujet, ledit microARN étant choisi dans le groupe constitué de miR-34a et miR-34c,
- la comparaison du taux du microARN circulant respectif à un taux de référence du microARN circulant pour en déduire des informations transmettant un taux de probabilité de MA modérée, ou de MA légère, ou de MA sévère ou un taux de probabilité pour faire la distinction entre un TCL et une MA, dans laquelle ledit taux témoin de référence est un taux circulant dans un échantillon de sérum ou de plasma provenant d'un sujet témoin âgé normal ; et
- la transmission des informations à un receveur pour déterminer un taux de probabilité de MA modérée, ou de MA légère, ou de MA sévère ou un taux de probabilité pour faire la distinction entre un TCL et une MA.

2. Méthode telle que définie dans la revendication 1, dans laquelle ledit microARN est le miR-34c et lesdites informations transmettent un taux de probabilité de MA modérée, ou de MA légère, ou de MA sévère.

3. Méthode telle que définie dans la revendication 1, dans laquelle ledit microARN est le miR-34c et lesdites informations transmettent un taux de probabilité pour faire la distinction entre un TCL et l'une quelconque de la MA légère, la MA modérée, et la MA sévère.

4. Méthode telle que définie dans la revendication 1, dans laquelle ledit microARN est le miR-34a et lesdites informations transmettent un taux de probabilité pour faire la distinction entre un TCL et une MA.


### SEQUENCE LISTING

<110> Wang, Eugenia
<120> biomarker of Azheimer's Disease and/or Mild cognitively impairment, and use thereof
<130> 87582-12
<150> U.S. 61/521,241
   <151> 2011-08-08
<150> U.S. 61/586,291
   <151> 2012-01-13
<150> U.S. 61/647,214
   <151> 2012-05-15
<160> 6
<170> PatentIn version 3.5
<210> 1
   <211> 21
   <212> DNA
   <213> artificial
<220>
   <223> forward primer for amplifying human miR-34a
<400> 1
   gagtcccctc cggatgccgt g 21
<210> 2
   <211> 18
   <212> DNA
   <213> artificial
<220>
   <223> reverse primer for amplifying human miR-34a
<400> 2
   ccacccaccg tggcgcag 18
<210> 3
   <211> 110
   <212> DNA
   <213> homo sapiens
<220>
   <221> misc_feature
   <222> (1)..(110)
   <223> precursor human microRNA miR-34a
<400> 3
   ggccagctgt gagtgtttct ttggcagtgt cttagctggt tgttgtgagc aatagtaagg 60
   aagcaatcag caagtatact gccctagaag tgctgcacgt tgtggggccc 110
<210> 4
   <211> 29
   <212> DNA
   <213> Artificial Sequence
<220>
   <223> forward primer for amplifying human miR-34c
<400> 4
   tctagaagcc cctccatcca tgtaacggt 29
<210> 5
   <211> 27
   <212> DNA
   <213> artificial
<220>
   <223> reverse primer for amplifying human miR-34c
<400> 5
   ggatccaaca cccctcttcc ccacgca 27
<210> 6
   <211> 77
   <212> DNA
   <213> homo sapiens
<220>
   <221> misc_feature
   <222> (1)..(77)
   <223> precursor human microRNA miR-34c
<400> 6
   agtctagtta ctaggcagtg tagttagctg attgctaata gtaccaatca ctaaccacac 60
   ggccaggtaa aaagatt 77